# FASCICULE DE BREVET EUROPEEN

(11) **EP 2 859 349 B1**
(45) Date de publication et mention de la délivrance du brevet: **23.10.2019**
(21) Numéro de dépôt: 13737806.3
(22) Date de dépôt: 05.06.2013
(51) Int. Cl.: G01N 33/574, G01N 33/68, C07K 16/18

(54) **PROCÉDÉ DE DÉTECTION ET/OU DE DOSAGE DE L'ANNEXINE A3 D'UN MAMMIFÈRE DANS LE SANG OU UN DE SES DÉRIVÉS**
VERFAHREN ZUM NACHWEIS UND/ODER ZUR DOSIERUNG VON ANNEXIN A3 IM SÄUGETIERBLUT ODER IN DESSEN DERIVATEN
METHOD FOR DETECTING AND/OR ASSAYING ANNEXIN A3 IN MAMMALIAN BLOOD OR IN A DERIVATIVE THEREOF

(30) Priorité: 07.06.2012 FR 1255340
(43) Date de publication de la demande: 15.04.2015
(73) Titulaire: bioMérieux, 69280 Marcy l'Etoile (FR)
(72) Inventeur: ATAMAN-ÖNAL, Yasemin, F-01600 Reyrieux (FR); CHOQUET-KASTYLEVSKY, Geneviève, F-69340 Francheville (FR); HAIDOUS, Hader, 69280 Marcy l'Etoile (FR); MICHEL-BUSSERET, Sandrine, F-69001 Lyon (FR); OTT, Catherine, 69800 Saint Priest (FR)
(74) Mandataire: Murgitroyd & Company
(86) Numéro de dépôt international: PCT/EP2013/061593
(87) Numéro de publication internationale: WO 2013/182602

(56) Documents cités:
- WO-A1-2006/125580
- WO-A1-2012/076812
- WO-A2-2007/141043
- WO-A2-2010/034825

### SEQUENCE LISTING

<110> bioMérieux et ProteoSys
<120> Procédé de détection et/ou de dosage de l'Annexine A3 d'un mammifère dans le sang ou au moins un de ses dérivés
<130> P124405.FR.01
<160> 50
<170> PatentIn version 3.5
<210> 1
   <211> 61
   <212> PRT
   <213> Homo sapiens
<400> 1
   Ile Gln Lys Ala Ile Arg Gly Ile Gly Thr Asp Glu Lys Met Leu Ile
   1               5                   10                  15
   Ser Ile Leu Thr Glu Arg Ser Asn Ala Gln Arg Gln Leu Ile Val Lys
               20                  25                  30
   Glu Tyr Gln Ala Ala Tyr Gly Lys Glu Leu Lys Asp Asp Leu Lys Gly
           35                  40                  45
   Asp Leu Ser Gly His Phe Glu His Leu Met Val Ala Leu
       50                  55                  60
<210> 2
   <211> 61
   <212> PRT
   <213> Homo sapiens
<400> 2
   Leu His Arg Ala Leu Lys Gly Ile Gly Thr Asp Glu Phe Thr Leu Asn
   1               5                   10                  15
   Arg Ile Met Val Ser Arg Ser Glu Ile Asp Leu Leu Asp Ile Arg Thr
               20                  25                  30
   Glu Phe Lys Lys His Tyr Gly Tyr Ser Leu Tyr Ser Ala Ile Lys Ser
           35                  40                  45
   Asp Thr Ser Gly Asp Tyr Glu Ile Thr Leu Leu Lys Ile
       50                  55                  60
<210> 3
   <211> 61
   <212> PRT
   <213> Homo sapiens
<400> 3
   Leu Lys Lys Ser Met Lys Gly Ala Gly Thr Asn Glu Asp Ala Leu Ile
   1               5                   10                  15
   Glu Ile Leu Thr Thr Arg Thr Ser Arg Gln Met Lys Asp Ile Ser Gln
               20                  25                  30
   Ala Tyr Tyr Thr Val Tyr Lys Lys Ser Leu Gly Asp Asp Ile Ser Ser
           35                  40                  45
   Glu Thr Ser Gly Asp Phe Arg Lys Ala Leu Leu Thr Leu
       50                  55                  60
<210> 4
   <211> 61
   <212> PRT
   <213> Homo sapiens
<400> 4
   Tyr Lys Ala Gly Glu Asn Arg Trp Gly Thr Asp Glu Asp Lys Phe Thr
   1               5                   10                  15
   Glu Ile Leu Cys Leu Arg Ser Phe Pro Gln Leu Lys Leu Thr Phe Asp
               20                  25                  30
   Glu Tyr Arg Asn Ile Ser Gln Lys Asp Ile Val Asp Ser Ile Lys Gly
           35                  40                  45
   Glu Leu Ser Gly His Phe Glu Asp Leu Leu Leu Ala Ile
       50                  55                  60
<210> 5
   <211> 323
   <212> PRT
   <213> Homo sapiens
<400> 5
   Met Ala Ser Ile Trp Val Gly His Arg Gly Thr Val Arg Asp Tyr Pro
   1               5                   10                  15
   Asp Phe Ser Pro Ser Val Asp Ala Glu Ala Ile Gln Lys Ala Ile Arg
               20                  25                  30
   Gly Ile Gly Thr Asp Glu Lys Met Leu Ile Ser Ile Leu Thr Glu Arg
           35                  40                  45
   Ser Asn Ala Gln Arg Gln Leu Ile Val Lys Glu Tyr Gln Ala Ala Tyr
   50 55 60
   Gly Lys Glu Leu Lys Asp Asp Leu Lys Gly Asp Leu Ser Gly His Phe
   65 70 75 80
   Glu His Leu Met Val Ala Leu Val Thr Pro Pro Ala Val Phe Asp Ala
   85 90 95
   Lys Gln Leu Lys Lys Ser Met Lys Gly Ala Gly Thr Asn Glu Asp Ala
   100 105 110
   Leu Ile Glu Ile Leu Thr Thr Arg Thr Ser Arg Gln Met Lys Asp Ile
   115 120 125
   Ser Gln Ala Tyr Tyr Thr Val Tyr Lys Lys Ser Leu Gly Asp Asp Ile
   130 135 140
   Ser Ser Glu Thr Ser Gly Asp Phe Arg Lys Ala Leu Leu Thr Leu Ala
   145 150 155 160
   Asp Gly Arg Arg Asp Glu Ser Leu Lys Val Asp Glu His Leu Ala Lys
   165 170 175
   Gln Asp Ala Gln Ile Leu Tyr Lys Ala Gly Glu Asn Arg Trp Gly Thr
   180 185 190
   Asp Glu Asp Lys Phe Thr Glu Ile Leu Cys Leu Arg Ser Phe Pro Gln
   195 200 205
   Leu Lys Leu Thr Phe Asp Glu Tyr Arg Asn Ile Ser Gln Lys Asp Ile
   210 215 220
   Val Asp Ser Ile Lys Gly Glu Leu Ser Gly His Phe Glu Asp Leu Leu
   225 230 235 240
   Leu Ala Ile Val Asn Cys Val Arg Asn Thr Pro Ala Phe Leu Ala Glu
   245 250 255
   Arg Leu His Arg Ala Leu Lys Gly Ile Gly Thr Asp Glu Phe Thr Leu
   260 265 270
   Asn Arg Ile Met Val Ser Arg Ser Glu Ile Asp Leu Leu Asp Ile Arg
   275 280 285
   Thr Glu Phe Lys Lys His Tyr Gly Tyr Ser Leu Tyr Ser Ala Ile Lys
   290 295 300
   Ser Asp Thr Ser Gly Asp Tyr Glu Ile Thr Leu Leu Lys Ile Cys Gly
   305 310 315 320
   Gly Asp Asp
<210> 6
   <211> 81
   <212> PRT
   <213> Artificial Sequence
<220>
   <223> Recombinant D1
<400> 6
   Met Gly His His His His His His His His Ser Pro Ser Val Asp Ala
   1 5 10 15
   Glu Ala Ile Gln Lys Ala Ile Arg Gly Ile Gly Thr Asp Glu Lys Met
   20 25 30
   Leu Ile Ser Ile Leu Thr Glu Arg Ser Asn Ala Gln Arg Gln Leu Ile
   35 40 45
   Val Lys Glu Tyr Gln Ala Ala Tyr Gly Lys Glu Leu Lys Asp Asp Leu
   50 55 60
   Lys Gly Asp Leu Ser Gly His Phe Glu His Leu Met Val Ala Leu Val
   65 70 75 80
   Thr
<210> 7
   <211> 79
   <212> PRT
   <213> Artificial Sequence
<220>
   <223> Recombinant D2
<400> 7
   Met Gly His His His His His His His His Ala Val Phe Asp Ala Lys
   1 5 10 15
   Gln Leu Lys Lys Ser Met Lys Gly Ala Gly Thr Asn Glu Asp Ala Leu
   20 25 30
   Ile Glu Ile Leu Thr Thr Arg Thr Ser Arg Gln Met Lys Asp Ile Ser
   35 40 45
   Gln Ala Tyr Tyr Thr Val Tyr Lys Lys Ser Leu Gly Asp Asp Ile Ser
   50 55 60
   Ser Glu Thr Ser Gly Asp Phe Arg Lys Ala Leu Leu Thr Leu Ala
   65 70 75
<210> 8
   <211> 85
   <212> PRT
   <213> Artificial Sequence
<220>
   <223> Recombinant D3
<400> 8
   Met Gly His His His His His His His His Asp Glu His Leu Ala Lys
   1 5 10 15
   Gln Asp Ala Gln Ile Leu Tyr Lys Ala Gly Glu Asn Arg Trp Gly Thr
   20 25 30
   Asp Glu Asp Lys Phe Thr Glu Ile Leu Cys Leu Arg Ser Phe Pro Gln
   35 40 45
   Leu Lys Leu Thr Phe Asp Glu Tyr Arg Asn Ile Ser Gln Lys Asp Ile
   50 55 60
   Val Asp Ser Ile Lys Gly Glu Leu Ser Gly His Phe Glu Asp Leu Leu
   65 70 75 80
   Leu Ala Ile Val Asn
   85
<210> 9
   <211> 82
   <212> PRT
   <213> Artificial Sequence
<220>
   <223> Recombinant D4
<400> 9
   Met Gly His His His His His His His His Ala Phe Leu Ala Glu Arg
   1               5                   10                  15
   Leu His Arg Ala Leu Lys Gly Ile Gly Thr Asp Glu Phe Thr Leu Asn
               20                  25                  30
   Arg Ile Met Val Ser Arg Ser Glu Ile Asp Leu Leu Asp Ile Arg Thr
           35                  40                  45
   Glu Phe Lys Lys His Tyr Gly Tyr Ser Leu Tyr Ser Ala Ile Lys Ser
       50                  55                  60
   Asp Thr Ser Gly Asp Tyr Glu Ile Thr Leu Leu Lys Ile Cys Gly Gly
   65                  70                  75                  80
   Asp Asp
<210> 10
   <211> 17
   <212> PRT
   <213> Homo sapiens
<400> 10
   Ser Asn Ala Gln Arg Gln Leu Ile Val Lys Glu Tyr Gln Ala Ala Tyr
   1               5                   10                  15
   Gly
<210> 11
   <211> 11
   <212> PRT
   <213> Homo sapiens
<400> 11
   Leu Ile Val Lys Glu Tyr Gln Ala Ala Tyr Gly
   1               5                   10
<210> 12
   <211> 12
   <212> **PRT**
   <213> Homo sapiens
<400> 12
   Ile Val Lys Glu Tyr Gln Ala Ala Tyr Gly Lys Glu
   1               5                   10
<210> 13
   <211> 8
   <212> PRT
   <213> Homo sapiens
<400> 13
   Lys Glu Tyr Gln Ala Ala Tyr Gly
   1 5
<210> 14
   <211> 9
   <212> PRT
   <213> Homo sapiens
<400> 14
   Asp Leu Ser Gly His Phe Glu His Leu
   1 5
<210> 15
   <211> 7
   <212> PRT
   <213> Homo sapiens
<400> 15
   Leu Ser Gly His Phe Glu His
   1 5
<210> 16
   <211> 20
   <212> PRT
   <213> Homo sapiens
<400> 16
   Ala Ser Ile Trp Val Gly His Arg Gly Thr Val Arg Asp Tyr Pro Asp
   1 5 10 15
   Phe Ser Pro Ser
   20
<210> 17
   <211> 18
   <212> PRT
   <213> Homo sapiens
<400> 17
   Ser Ile Trp Val Gly His Arg Gly Thr Val Arg Asp Tyr Pro Asp Phe
   1 5 10 15
   Ser Pro
<210> 18
   <211> 4
   <212> PRT
   <213> Homo sapiens
<400> 18
   Tyr Pro Asp Phe
   1
<210> 19
   <211> 38
   <212> PRT
   <213> Homo sapiens
<400> 19
   Lys Glu Tyr Gln Ala Ala Tyr Gly Lys Glu Leu Lys Asp Asp Leu Lys
   1               5                   10                  15
   Gly Asp Leu Ser Gly His Phe Glu His Leu Met Val Ala Leu Val Thr
               20                  25                  30
   Pro Pro Ala Val Phe Asp
           35
<210> 20
   <211> 56
   <212> PRT
   <213> Homo sapiens
<400> 20
   Gln Lys Ala Ile Arg Gly Ile Gly Thr Asp Glu Lys Met Leu Ile Ser
   1               5                   10                  15
   Ile Leu Thr Glu Arg Ser Asn Ala Gln Arg Gln Leu Ile Val Lys Glu
               20                  25                  30
   Tyr Gln Ala Ala Tyr Gly Lys Glu Leu Lys Asp Asp Leu Lys Gly Asp
           35                  40                  45
   Leu Ser Gly His Phe Glu His Leu
       50                  55
<210> 21
   <211> 26
   <212> PRT
   <213> Homo sapiens
<400> 21
   Lys Glu Tyr Gln Ala Ala Tyr Gly Lys Glu Leu Lys Asp Asp Leu Lys
   1               5                   10                  15
   Gly Asp Leu Ser Gly His Phe Glu His Leu
               20                  25
<210> 22
   <211> 17
   <212> PRT
   <213> Homo sapiens
<400> 22
   Lys Glu Tyr Gln Ala Ala Tyr Gly Lys Glu Leu Lys Asp Asp Leu Lys
   1 5 10 15
   Gly
<210> 23
   <211> 61
   <212> PRT
   <213> Mus musculus
<400> 23
   Ile Arg Lys Ala Ile Arg Gly Leu Gly Thr Asp Glu Lys Thr Leu Ile
   1 5 10 15
   Asn Ile Leu Thr Glu Arg Ser Asn Ala Gln Arg Gln Leu Ile Val Lys
   20 25 30
   Gln Tyr Gln Ala Ala Tyr Glu Gln Glu Leu Lys Asp Asp Leu Lys Gly
   35 40 45
   Asp Leu Ser Gly His Phe Glu His Val Met Val Ala Leu
   50 55 60
<210> 24
   <211> 61
   <212> PRT
   <213> Rattus norvegicus
<400> 24
   Ile Arg Lys Ala Ile Lys Gly Ile Gly Thr Asp Glu Lys Thr Leu Ile
   1 5 10 15
   Asn Ile Leu Thr Glu Arg Ser Asn Ala Gln Arg Gln Leu Ile Val Lys
   20 25 30
   Gln Tyr Gln Glu Ala Tyr Glu Gln Ala Leu Lys Ala Asp Leu Lys Gly
   35 40 45
   Asp Leu Ser Gly His Phe Glu His Val Met Val Ala Leu
   50 55 60
<210> 25
   <211> 61
   <212> PRT
   <213> Bos taurus
<400> 25
   Ile Arg Lys Ala Ile Arg Gly Ile Gly Thr Asp Glu Lys Thr Leu Ile
   1               5                   10                  15
   Ser Ile Leu Thr Glu Arg Thr Asn Ala Gln Arg Leu Leu Ile Ala Lys
               20                  25                  30
   Glu Tyr Gln Ala Leu Cys Gly Lys Glu Leu Lys Asp Asp Leu Lys Gly
           35                  40                  45
   Asp Leu Ser Gly His Phe Lys His Leu Met Val Ala Leu
       50                  55                  60
<210> 26
   <211> 61
   <212> PRT
   <213> Canis familiaris
<400> 26
   Ile Arg Lys Ala Ile Arg Gly Ile Gly Thr Glu Glu Lys Thr Leu Ile
   1               5                   10                  15
   Ser Ile Leu Thr Glu Arg Thr Asn Ala Gln His Gln Leu Ile Val Arg
               20                  25                  30
   Glu Tyr Gln Ala Ala Tyr Gly Lys Glu Leu Lys Asp Asp Leu Lys Gly
           35                  40                  45
   Asp Leu Ser Gly His Phe Lys Gln Leu Met Val Ala Leu
       50                  55                  60
<210> 27
   <211> 61
   <212> PRT
   <213> Oryctolagus cuniculus
<400> 27
   Ile Arg Lys Ala Ile Arg Gly Ile Gly Thr Asp Glu Lys Thr Leu Ile
   1               5                   10                  15
   Ser Ile Leu Ala Glu Arg Ser Ser Ala Gln Arg Gln Leu Ile Val Lys
               20                  25                  30
   Glu Tyr Gln Ala Ala Tyr Gly Lys Glu Leu Lys Asp Asp Leu Lys Gly
           35                  40                  45
   Asp Leu Ser Gly His Phe Glu His Leu Met Val Ala Leu
       50                  55                  60
<210> 28
   <211> 61
   <212> PRT
   <213> Macaca mulatta
<400> 28
   Ile Arg Lys Ala Ile Arg Gly Ile Gly Thr Asp Glu Lys Met Leu Ile
   1               5                   10                  15
   Ser Ile Leu Thr Glu Arg Ser Asn Ala Gln Arg Gln Leu Ile Val Lys
               20                  25                  30
   Glu Tyr Gln Ala Ala Tyr Gly Lys Glu Leu Lys Asp Asp Leu Lys Gly
           35                  40                  45
   Asp Leu Ser Gly His Phe Glu His Leu Met Val Ala Leu
       50                  55                  60
<210> 29
   <211> 61
   <212> PRT
   <213> Gorilla gorilla
<400> 29
   Ile Gln Lys Ala Ile Arg Gly Ile Gly Thr Asp Glu Lys Met Leu Ile
   1               5                   10                  15
   Ser Ile Leu Thr Glu Arg Ser Asn Ala Gln Arg Gln Leu Ile Val Lys
               20                  25                  30
   Glu Tyr Gln Ala Ala Tyr Gly Lys Glu Leu Lys Asp Asp Leu Lys Gly
           35                  40                  45
   Asp Leu Ser Gly His Phe Glu His Leu Met Val Ala Leu
       50                  55                  60
<210> 30
   <211> 61
   <212> PRT
   <213> Mus musculus
<400> 30
   Leu His Gln Ala Leu Lys Gly Ala Gly Thr Asp Glu Phe Thr Leu Asn
   1               5                   10                  15
   Arg Ile Met Val Ser Arg Ser Glu Ile Asp Leu Leu Asp Ile Arg His
               20                  25                  30
   Glu Phe Lys Lys His Tyr Gly Tyr Ser Leu Tyr Ser Ala Ile Gln Ser
           35                  40                  45
   Asp Thr Ser Gly Asp Tyr Arg Thr Val Leu Leu Lys Ile
              50                  55                  60
<210> 31
   <211> 61
   <212> PRT
   <213> Rattus norvegicus
<400> 31
   Leu His Gln Ala Leu Lys Gly Ala Gly Thr Asp Glu Phe Thr Leu Asn
   1               5                   10                  15
   Arg Ile Met Val Ser Arg Ser Glu Ile Asp Leu Leu Asp Ile Arg Arg
               20                  25                  30
   Glu Phe Lys Lys His Tyr Gly Cys Ser Leu Tyr Ser Ala Ile Gln Ser
           35                  40                  45
   Asp Thr Ser Gly Asp Tyr Arg Thr Val Leu Leu Lys Ile
       50                  55                  60
<210> 32
   <211> 61
   <212> PRT
   <213> Bos taurus
<400> 32
   Leu Tyr Arg Ala Leu Lys Gly Ala Gly Thr Asp Glu Phe Thr Leu Asn
   1               5                   10                  15
   Arg Ile Met Val Ser Arg Ser Glu Ile Asp Leu Leu Asp Ile Arg Ala
               20                  25                  30
   Glu Phe Lys Lys Leu Ser Gly Tyr Ser Leu Tyr Ser Ala Ile Lys Ser
           35                  40                  45
   Asp Thr Ser Gly Asp Tyr Glu Ile Thr Leu Leu Lys Ile
       50                  55                  60
<210> 33
   <211> 61
   <212> PRT
   <213> Canis familiaris
<400> 33
   Leu His Gln Ala Leu Lys Gly Ala Gly Thr Asp Glu Phe Thr Leu Asn
   1               5                   10                  15
   Arg Ile Met Val Ser Arg Ser Glu Ile Asp Leu Leu Asp Ile Arg Ala
               20                  25                  30
   Glu Phe Lys Lys His Tyr Gly Tyr Ser Leu Tyr Ser Ala Ile Lys Ser
           35                  40                  45
   Asp Thr Ser Gly Asp Tyr Glu Leu Thr Leu Leu Lys Ile
       50                  55                  60
<210> 34
   <211> 61
   <212> PRT
   <213> Oryctolagus cuniculus
<400> 34
   Leu Tyr Gln Ala Leu Lys Gly Ala Gly Thr Asp Glu Phe Thr Leu Asn
   1               5                   10                  15
   Arg Ile Met Val Ser Arg Ser Glu Ile Asp Leu Leu Asp Ile Arg Ala
               20                  25                  30
   Glu Phe Lys Lys Gln Tyr Gly Tyr Ser Leu Tyr Ser Ala Ile Lys Ser
           35                  40                  45
   Asp Thr Ser Gly Asp Tyr Glu Val Thr Leu Leu Lys Ile
       50                  55                  60
<210> 35
   <211> 61
   <212> PRT
   <213> Macaca mulatta
<400> 35
   Leu His Arg Ala Leu Lys Gly Ala Gly Thr Asp Glu Phe Thr Leu Asn
   1               5                   10                  15
   Arg Ile Met Val Ser Arg Ser Glu Ile Asp Leu Leu Asp Ile Arg Ile
               20                  25                  30
   Glu Phe Lys Lys His Tyr Gly Tyr Ser Leu Tyr Ser Ala Ile Lys Ser
           35                  40                  45
   Asp Thr Ser Gly Asp Tyr Glu Ile Thr Leu Leu Lys Ile
       50                  55                  60
<210> 36
   <211> 61
   <212> PRT
   <213> Gorilla gorilla
<400> 36
   Leu His Arg Ala Leu Lys Gly Ile Gly Thr Asp Glu Phe Thr Leu Asn
   1               5                   10                  15
   Arg Ile Met Val Ser Arg Ser Glu Ile Asp Leu Leu Asp Ile Arg Thr
               20                  25                  30
   Glu Phe Lys Lys His Tyr Gly Tyr Ser Leu Tyr Ser Ala Ile Lys Ser
           35                  40                  45
   Asp Thr Ser Gly Asp Tyr Glu Ile Thr Leu Leu Lys Ile
       50                  55                  60
<210> 37
   <211> 17
   <212> PRT
   <213> Artificial Sequence
<220>
   <223> épitope inclus au sein du premier domaine répété de l'Annexine A3 de mammifère
<220>
   <221> Xaa1
   <222> (1)..(1)
   <223> Xaa1 représente un résidu sérine, un résidu thréonine ou un acide aminé analogue, avantageusement un résidu sérine ou un résidu thréonine
<220>
   <221> Xaa2
   <222> (2)..(2)
   <223> Xaa2 représente un résidu asparagine, un résidu sérine ou un acide aminé analogue, avantageusement un résidu asparagine ou un résidu sérine
<220>
   <221> Xaa3
   <222> (5)..(5)
   <223> Xaa3 représente un résidu arginine, un résidu histidine ou un acide aminé analogue, avantageusement un résidu arginine ou un résidu histidine
<220>
   <221> Xaa4
   <222> (6)..(6)
   <223> Xaa4 représente un résidu glutamine, un résidu leucine ou un acide aminé analogue, avantageusement un résidu glutamine ou un résidu leucine
<220>
   <221> Xaa5
   <222> (9)..(9)
   <223> Xaa5 représente un résidu valine, un résidu alanine ou un acide aminé analogue, avantageusement un résidu valine ou un résidu alanine
<220>
   <221> Xaa6
   <222> (10)..(10)
   <223> Xaa6 représente un résidu lysine, un résidu arginine ou un acide aminé analogue, avantageusement un résidu lysine ou un résidu arginine
<220>
   <221> Xaa7
   <222> (11)..(11)
   <223> Xaa7 représente un résidu acide glutamique, un résidu glutamine ou un acide aminé analogue, avantageusement un résidu acide glutamique ou un résidu glutamine
<220>
   <221> Xaa8
   <222> (14)..(14)
   <223> Xaa8 représente un résidu alanine, un résidu acide glutamique ou un acide aminé analogue, avantageusement un résidu alanine ou un résidu acide glutamique
<220>
   <221> Xaa9
   <222> (15)..(15)
   <223> Xaa9 représente un résidu alanine, un résidu leucine ou un acide aminé analogue, avantageusement un résidu alanine ou un résidu leucine
<220>
   <221> Xaa10
   <222> (16)..(16)
   <223> Xaa10 représente un résidu tyrosine, un résidu cystéine ou un acide aminé analogue, avantageusement un résidu tyrosine ou un résidu cystéine
<220>
   <221> Xaa11
   <222> (17)..(17)
   <223> Xaa11 représente un résidu glycine, un résidu acide glutamique ou un acide aminé analogue, avantageusement un résidu glycine ou un résidu acide glutamique
<400> 37
   Xaa Xaa Ala Gln Xaa Xaa Leu Ile Xaa Xaa Xaa Tyr Gln Xaa Xaa Xaa
   1               5                   10                  15
   Xaa
<210> 38
   <211> 11
   <212> PRT
   <213> Artificial Sequence
<220>
   <223> épitope inclus au sein du premier domaine répété de l'Annexine A3 de mammifère
<220>
   <221> Xaa5
   <222> (3)..(3)
   <223> Xaa5 représente un résidu valine, un résidu alanine ou un acide aminé analogue, avantageusement un résidu valine ou un résidu alanine
<220>
   <221> Xaa6
   <222> (4)..(4)
   <223> Xaa6 représente un résidu lysine, un résidu arginine ou un acide aminé analogue, avantageusement un résidu lysine ou un résidu arginine
<220>
   <221> Xaa7
   <222> (5)..(5)
   <223> Xaa7 représente un résidu acide glutamique, un résidu glutamine ou un acide aminé analogue, avantageusement un résidu acide glutamique ou un résidu glutamine
<220>
   <221> Xaa8
   <222> (8)..(8)
   <223> Xaa8 représente un résidu alanine, un résidu acide glutamique ou un acide aminé analogue, avantageusement un résidu alanine ou un résidu acide glutamique
<220>
   <221> Xaa9
   <222> (9)..(9)
   <223> Xaa9 représente un résidu alanine, un résidu leucine ou un acide aminé analogue, avantageusement un résidu alanine ou un résidu leucine
<220>
   <221> Xaa10
   <222> (10)..(10)
   <223> Xaa10 représente un résidu tyrosine, un résidu cystéine ou un acide aminé analogue, avantageusement un résidu tyrosine ou un résidu cystéine
<220>
   <221> Xaa11
   <222> (11)..(11)
   <223> Xaa11 représente un résidu glycine, un résidu acide glutamique ou un acide aminé analogue, avantageusement un résidu glycine ou un résidu acide glutamique
<400> 38
   Leu Ile Xaa Xaa Xaa Tyr Gln Xaa Xaa Xaa Xaa
   1               5                   10
<210> 39
   <211> 12
   <212> PRT
   <213> Artificial Sequence
<220>
   <223> épitope inclus au sein du premier domaine répété de l'Annexine A3 de mammifère
<220>
   <221> Xaa5
   <222> (2)..(2)
   <223> Xaa5 représente un résidu valine, un résidu alanine ou un acide aminé analogue, avantageusement un résidu valine ou un résidu alanine
<220>
   <221> Xaa6
   <222> (3)..(3)
   <223> Xaa6 représente un résidu lysine, un résidu arginine ou un acide aminé analogue, avantageusement un résidu lysine ou un résidu arginine
<220>
   <221> Xaa7
   <222> (4)..(4)
   <223> Xaa7 représente un résidu acide glutamique, un résidu glutamine ou un acide aminé analogue, avantageusement un résidu acide glutamique ou un résidu glutamine
<220>
   <221> Xaa8
   <222> (7)..(7)
   <223> Xaa8 représente un résidu alanine, un résidu acide glutamique ou un acide aminé analogue, avantageusement un résidu alanine ou un résidu acide glutamique
<220>
   <221> Xaa9
   <222> (8)..(8)
   <223> Xaa9 représente un résidu alanine, un résidu leucine ou un acide aminé analogue, avantageusement un résidu alanine ou un résidu leucine
<220>
   <221> Xaa10
   <222> (9)..(9)
   <223> Xaa10 représente un résidu tyrosine, un résidu cystéine ou un acide aminé analogue, avantageusement un résidu tyrosine ou un résidu cystéine
<220>
   <221> Xaa11
   <222> (10)..(10)
   <223> Xaa11 représente un résidu glycine, un résidu acide glutamique ou un acide aminé analogue, avantageusement un résidu glycine ou un résidu acide glutamique
<220>
   <221> Xaa12
   <222> (11)..(11)
   <223> Xaa12 représente un résidu lysine, un résidu glutamine ou un acide aminé analogue, avantageusement un résidu lysine ou un résidu glutamine
<220>
   <221> Xaa13
   <222> (12)..(12)
   <223> Xaa13 représente un résidu acide glutamique, un résidu alanine ou un acide aminé analogue, avantageusement un résidu acide glutamique ou un résidu alanine
<400> 39
   Ile Xaa Xaa Xaa Tyr Gln Xaa Xaa Xaa Xaa Xaa Xaa
   1               5                   10
<210> 40
   <211> 8
   <212> PRT
   <213> Artificial Sequence
<220>
   <223> épitope inclus au sein du premier domaine répété de l'Annexine A3 de mammifère
<220>
   <221> Xaa6
   <222> (1)..(1)
   <223> Xaa6 représente un résidu lysine, un résidu arginine ou un acide aminé analogue, avantageusement un résidu lysine ou un résidu arginine
<220>
   <221> Xaa7
   <222> (2)..(2)
   <223> Xaa7 représente un résidu acide glutamique, un résidu glutamine ou un acide aminé analogue, avantageusement un résidu acide glutamique ou un résidu glutamine
<220>
   <221> Xaa8
   <222> (5)..(5)
   <223> Xaa8 représente un résidu alanine, un résidu acide glutamique ou un acide aminé analogue, avantageusement un résidu alanine ou un résidu acide glutamique
<220>
   <221> Xaa9
   <222> (6)..(6)
   <223> Xaa9 représente un résidu alanine, un résidu leucine ou un acide aminé analogue, avantageusement un résidu alanine ou un résidu leucine
<220>
   <221> Xaa10
   <222> (7)..(7)
   <223> Xaa10 représente un résidu tyrosine, un résidu cystéine ou un acide aminé analogue, avantageusement un résidu tyrosine ou un résidu cystéine
<220>
   <221> Xaa11
   <222> (8)..(8)
   <223> Xaa11 représente un résidu glycine, un résidu acide glutamique ou un acide aminé analogue, avantageusement un résidu glycine ou un résidu acide glutamique
<400> 40
   Xaa Xaa Tyr Gln Xaa Xaa Xaa Xaa
   1               5
<210> 41
   <211> 17
   <212> PRT
   <213> Artificial Sequence
<220>
   <223> épitope inclus au sein du premier domaine répété de l'Annexine A3 de mammifère
<220>
   <221> Xaa6
   <222> (1)..(1)
   <223> Xaa6 représente un résidu lysine, un résidu arginine ou un acide aminé analogue, avantageusement un résidu lysine ou un résidu arginine
<220>
   <221> Xaa7
   <222> (2)..(2)
   <223> Xaa7 représente un résidu acide glutamique, un résidu glutamine ou un acide aminé analogue, avantageusement un résidu acide glutamique ou un
résidu glutamine
<220>
   <221> Xaa8
   <222> (5)..(5)
   <223> Xaa8 représente un résidu alanine, un résidu acide glutamique ou un acide aminé analogue, avantageusement un résidu alanine ou un résidu acide glutamique
<220>
   <221> Xaa9
   <222> (6)..(6)
   <223> Xaa9 représente un résidu alanine, un résidu leucine ou un acide aminé analogue, avantageusement un résidu alanine ou un résidu leucine
<220>
   <221> Xaa10
   <222> (7)..(7)
   <223> Xaa10 représente un résidu tyrosine, un résidu cystéine ou un acide aminé analogue, avantageusement un résidu tyrosine ou un résidu cystéine
<220>
   <221> Xaa11
   <222> (8)..(8)
   <223> Xaa11 représente un résidu glycine, un résidu acide glutamique ou un acide aminé analogue, avantageusement un résidu glycine ou un résidu acide glutamique
<220>
   <221> Xaa12
   <222> (9)..(9)
   <223> Xaa12 représente un résidu lysine, un résidu glutamine ou un acide aminé analogue, avantageusement un résidu lysine ou un résidu glutamine
<220>
   <221> Xaa13
   <222> (10)..(10)
   <223> Xaa13 représente un résidu acide glutamique, un résidu alanine ou un acide aminé analogue, avantageusement un résidu acide glutamique ou un résidu alanine
<220>
   <221> Xaa14
   <222> (13)..(13)
   <223> Xaa14 représente un résidu acide aspartique, un résidu alanine ou un acide aminé analogue, avantageusement un résidu acide aspartique ou un résidu alanine
<400> 41
   Xaa Xaa Tyr Gln Xaa Xaa Xaa Xaa Xaa Xaa Leu Lys Xaa Asp Leu Lys
   1               5                   10                  15
   Gly
<210> 42
   <211> 9
   <212> PRT
   <213> Artificial Sequence
<220>
   <223> épitope inclus au sein du premier domaine répété de l'Annexine A3 de mammifère
<220>
   <221> Xaa20
   <222> (7)..(7)
   <223> Xaa20 représente un résidu acide glutamique, un résidu lysine ou un acide aminé analogue, avantageusement un résidu acide glutamique ou un résidu lysine
<220>
   <221> Xaa21
   <222> (8)..(8)
   <223> Xaa21 représente un résidu histidine, un résidu glutamine ou un acide aminé analogue, avantageusement un résidu histidine ou un résidu glutamine
<220>
   <221> Xaa22
   <222> (9)..(9)
   <223> Xaa22 représente un résidu leucine, un résidu valine ou un acide aminé analogue, avantageusement un résidu leucine ou un résidu valine
<400> 42
   Asp Leu Ser Gly His Phe Xaa Xaa Xaa
   1               5
<210> 43
   <211> 7
   <212> PRT
   <213> Artificial Sequence
<220>
   <223> épitope inclus au sein du premier domaine répété de l'Annexine A3 de mammifère
<220>
   <221> Xaa20
   <222> (6)..(6)
   <223> Xaa20 représente un résidu acide glutamique, un résidu lysine ou un acide aminé analogue, avantageusement un résidu acide glutamique ou un résidu lysine
<220>
   <221> Xaa21
   <222> (7)..(7)
   <223> Xaa21 représente un résidu histidine, un résidu glutamine ou un acide aminé analogue, avantageusement un résidu histidine ou un résidu glutamine
<400> 43
   Leu Ser Gly His Phe Xaa Xaa
   1               5
<210> 44
   <211> 17
   <212> PRT
   <213> Canis familiaris
<400> 44
   Thr Asn Ala Gln His Gln Leu Ile Val Arg Glu Tyr Gln Ala Ala Tyr
   1               5                   10                  15
   Gly
<210> 45
   <211> 11
   <212> PRT
   <213> Canis familiaris
<400> 45
   Leu Ile Val Arg Glu Tyr Gln Ala Ala Tyr Gly
   1               5                   10
<210> 46
   <211> 12
   <212> PRT
   <213> Canis familiaris
<400> 46
   Ile Val Arg Glu Tyr Gln Ala Ala Tyr Gly Lys Glu
   1               5                   10
<210> 47
   <211> 8
   <212> PRT
   <213> Canis familiaris
<400> 47
   Arg Glu Tyr Gln Ala Ala Tyr Gly
   1               5
<210> 48
   <211> 9
   <212> PRT
   <213> Canis familiaris
<400> 48
   Asp Leu Ser Gly His Phe Lys Gln Leu
   1               5
<210> 49
   <211> 7
   <212> PRT
   <213> Canis familiaris
<400> 49
   Leu Ser Gly His Phe Lys Gln
        1               5
<210> 50
   <211> 17
   <212> PRT
   <213> Canis familiaris
<400> 50
   Arg Glu Tyr Gln Ala Ala Tyr Gly Lys Glu Leu Lys Asp Asp Leu Lys
   1               5                   10                  15
   Gly

## Description

La présente invention a pour objet un procédé et un coffret pour la détection et/ou le dosage de l'Annexine A3 d'un mammifère (par exemple l'homme) dans le sang ou dans les fluides biologiques qui en dérivent comme le sérum ou le plasma. Une détection et/ou un dosage de cette nature est tout à fait souhaitable notamment afin de diagnostiquer, pronostiquer ou de déterminer la susceptibilité à des pathologies aigües ou chroniques.

L'Annexine A3 (ANXA3) appartient à la famille des Annexines. Les protéines de cette famille possèdent la caractéristique commune de pouvoir se fixer aux membranes phospholipidiques en présence de calcium. Ces protéines sont exprimées de façon relativement ubiquitaire dans différents tissus et types cellulaires, chez les animaux et les plantes. Il y a 12 Annexines qui ont été identifiées chez les vertébrés, elles forment la famille des Annexines A [1].

La propriété caractérisant les Annexines, à savoir la capacité de lier les phospholipides membranaires de manière calcium-dépendante, résulte de caractéristiques structurelles partagées par tous les membres de cette famille de protéines. Ainsi, chaque Annexine contient dans sa séquence protéique des domaines répétés appelés « annexin repeat » qui sont au nombre de 4 ou 8 (4 pour l'Annexine A3) et ont une longueur d'environ 70 acides aminés [2]. Chaque domaine répété, appelé aussi domaine endonexine, est replié en 5 hélices alpha et contient un motif caractéristique de type 2 (GxGT-[38 residus]-D/E) qui permet de fixer des ions Ca²⁺. Les Annexines du règne animal possèdent des domaines N-terminaux variables, qui portent souvent les spécificités fonctionnelles additionnelles des protéines. Les analyses cristallographiques montrent que les structures secondaires et tertiaires des Annexines sont conservées même si l'identité des séquences en acides aminés ne dépasse pas 45 à 55%. Les 4 domaines répétés forment une structure qui ressemble à un disque, avec une surface un peu convexe sur laquelle se situent les sites de fixation du Ca²⁺ et une surface concave où les extrémités N- et C-terminales de la protéine se trouvent à proximité [3].

Ainsi, de par leurs propriétés structurelles, les Annexines peuvent former des réseaux sur la surface membranaire et organiser des microdomaines membranaires et des foyers de recrutement pour les protéines interactives. De façon plus générale, elles sont impliquées dans la liaison membrane plasmique-cytosquelette, la régulation des flux de l'ion calcium à travers les membranes et le trafic exo- et endocytotique [4-6]. De ce fait, les Annexines jouent un rôle important dans la différenciation cellulaire, la migration cellulaire et l'immunomodulation. La plupart des Annexines sont également des inhibiteurs de la phospholipase A2 et possèdent des propriétés anticoagulantes. De plus, chaque membre de la famille peut être impliqué de manière individuelle dans d'autres processus physiologiques ou pathologiques.

L'Annexine A3 est exprimée au niveau de différents tissus tels que le poumon, la rate, le placenta, la prostate, le rein [7]. Elle est considérée comme une Annexine rare, en comparaison avec d'autres membres de la famille qui ont une expression plus ubiquitaire et/ou des niveaux d'expression plus élevés. L'Annexine A3 est très abondante dans les neutrophiles humains, où elle représente environ 1% des protéines cytosoliques [8, 9]. Initialement, deux isoformes de l'Annexine A3 ont été décrites, une de 33 kDa de masse moléculaire apparente, présente majoritairement dans les neutrophiles, l'autre de 36 kDa de masse moléculaire apparente, retrouvée dans les monocytes. La différence de masse moléculaire entre les deux isoformes ne résulte pas d'une modification post-traductionnelle telle que la phosphorylation ou la glycosylation. En 2010, il a été démontré qu'un phénomène d'épissage alternatif pouvait être un mécanisme expliquant la présence de ces deux isoformes. Ainsi, les auteurs ont identifié deux variants d'ADN complémentaire de l'Annexine A3 : le premier d'une longueur de 973 paires de bases (pb) code pour une protéine entière de 323 acides aminés correspondant à l'isoforme de 36 kDa ; le second, d'une longueur de 885 pb, ne contient pas l'exon III suite à un épissage alternatif. Lorsque cet exon n'est pas présent, le cadre de lecture ouvert est interrompu, et la traduction ne devient possible qu'à partir du codon ATG présent dans l'exon IV. Par conséquent, les 39 premiers acides aminés de la protéine ne sont pas exprimés, il en résulte une protéine tronquée à l'extrémité N-terminale de 284 acides aminés, correspondant à l'isoforme de 33 kDa [10]. De plus, l'analyse par Western blot après une électrophorèse à 2 dimensions (2DE-WB) montre que l'isoforme de 36 kDa de l'Annexine A3 migre sous la forme de 4 à 6 spots, ayant des points isoélectriques différents. L'isoforme de 36 kDa identifiée par un Western blot à 1 dimension correspond en fait à un groupe d'isoformes hétérogènes [10].

En ce qui concerne sa fonction, il est démontré que l'Annexine A3 est une lipocortine, c'est à dire un inhibiteur de la phospholipase A2 [11]. De plus, elle exerce une activité anticoagulante au niveau placentaire [11] et provoque l'agrégation des vésicules membranaires, comme par exemple les granules des neutrophiles [12]. *In vitro,* la présence de la protéine Annexine A3 est nécessaire à la croissance d'hépatocytes de rat [7].

De façon plus générale, la protéine Annexine A3 est impliquée de façon directe ou indirecte dans de nombreuses pathologies.

Dans l'art antérieur, l'Annexine A3 a été fréquemment associée à différents types de cancers. Par exemple, la demande de brevet EP-A-1724586 décrit la détermination de l'abondance de l'Annexine A3 intracellulaire et/ou extracellulaire dans un échantillon d'urine en vue de diagnostiquer un cancer uro-génital ou du tractus digestif. En particulier, l'Annexine A3 est significativement associée à la progression et aux divers stades de maladies prostatiques et de cancers de la prostate. Dans le tissu prostatique, ainsi que dans l'urine, le niveau d'expression de la protéine Annexine A3 diminue en cas de cancer de la prostate [10, 14, 15].

En ce qui concerne le cancer colorectal, il est connu que l'expression de l'ARNm codant pour l'Annexine A3 et l'expression de la protéine Annexine A3 sont augmentées dans les tumeurs colorectales par rapport au tissu sain adjacent [16]. De plus, l'Annexine A3 est utilisée dans une méthode permettant de déterminer la probabilité d'avoir un cancer colorectal chez un sujet (WO 2009/125303). Pour ce faire, du sang total est collecté dans les tubes PAXgene Blood RNA tubes (PreAnalytiX)™ permettant de stabiliser les ARN intracellulaires des cellules présentes dans le sang périphérique, l'ARN est extrait, puis le niveau d'expression de l'ARN codant pour l'Annexine A3 est mesuré par RT-PCR quantitative en temps réel. Toutefois, ce niveau d'expression de l'ARN codant pour l'Annexine A3 est le reflet de la réponse immunitaire de l'hôte à la pathologie. De ce fait, c'est un marqueur indirect, non spécifique des cellules cancéreuses qui nécessite la combinaison avec d'autres gènes pour améliorer la spécificité. C'est ainsi qu'il a été montré que la combinaison avec six autres gènes permet d'améliorer les performances cliniques [17]. De plus, cette technologie de biologie moléculaire est coûteuse.

En ce qui concerne le cancer du poumon, il est connu que l'expression de la protéine Annexine A3 est significativement élevée dans les adénocarcinomes primaires ayant des métastases locales au niveau des ganglions lymphatiques, comparée aux tumeurs primaires n'ayant pas métastasé. Ce résultat suggère que l'Annexine A3 joue un rôle important dans la progression du cancer du poumon et qu'elle est un facteur pronostique pour cette pathologie [18].

L'Annexine A3 présente également un intérêt diagnostique pour ce qui concerne le cancer du pancréas [19].

Par ailleurs, la diminution de l'expression de l'Annexine A3 est corrélée avec la progression tumorale dans le cancer de la thyroïde papillaire [20] ; cette protéine étant également un biomarqueur candidat dans le cadre d'une prédisposition au cancer du sein (cf. US 2011/0251082).

En outre, il existe également des liens entre l'expression d'Annexine A3 et le processus inflammatoire. La cyclooxygenase-2 (COX-2) est une peroxydase qui permet de convertir l'acide arachidonique en prostaglandine H2. Il s'agit d'une enzyme inductible qui est exprimée au site de l'inflammation et qui a une action pro-inflammatoire. L'inhibition de COX-2 par les médicaments anti-inflammatoires non stéroïdiens permet de diminuer l'expression de médiateurs inflammatoires et a des effets analgésiques. L'Annexine A3 fait partie des gènes dont l'expression est augmentée suite à un traitement par rofecoxib (un inhibiteur sélectif de COX-2) et qui sont associés à l'inhibition de la phospholipase A2 ainsi qu'à la suppression de cascades de cytokines [21]. L'Annexine A3 est impliquée dans les voies de signalisation permettant de contrôler l'inflammation, et par conséquent a une utilité en tant que marqueur ou cible thérapeutique dans toute pathologie impliquant une inflammation aigüe ou chronique.

Le syndrome de réponse inflammatoire systémique ou SIRS (en langue anglaise) désigne un état inflammatoire généralisé qui peut être induit par une infection ou une cause non infectieuse comme par exemple un traumatisme, une brûlure importante ou encore une pancréatite. Plus précisément, un SIRS résultant d'une infection documentée, c'est à dire confirmée au laboratoire, est dénommé sepsis. Les tests microbiologiques sont déterminants : ils permettent d'identifier l'agent infectieux à l'origine de l'infection et, si nécessaire, de tester son profil de résistance aux antibiotiques. L'hémoculture est un examen clé qui doit être pratiqué dans des conditions optimales afin d'obtenir toutes les chances de détecter le plus rapidement possible le micro-organisme responsable de l'infection. Cependant, l'agent infectieux responsable (bactérie, virus, champignon ou parasite) n'est statistiquement identifié que dans 2 cas sur 3 environ. Il est donc nécessaire de prendre en considération d'autres signes et symptômes ainsi que les résultats d'examens cliniques et biologiques pour pouvoir poser le diagnostic, déterminer le pronostic et suivre la réponse au traitement. Un biomarqueur, la procalcitonine (PCT) permet d'améliorer les performances cliniques pour le diagnostic précoce du sepsis et le choix d'un traitement adéquat. L'expression de PCT est très spécifique de l'origine bactérienne du syndrome d'inflammation systémique et elle est aussi très précoce : une augmentation du taux sérique est mesurable en 3 à 6h après le démarrage de l'infection. Lorsque l'agent infectieux est de nature non bactérienne, le diagnostic du sepsis est plus difficile à poser. De façon plus générale, il est très utile de pouvoir prédire le développement du sepsis chez les patients présentant un état de SIRS. Dans ce but, la demande de brevet US 2011/0105350 décrit des méthodes utilisant la combinaison de marqueurs ARNm mesurés dans des extraits d'ARN obtenus à partir de prélèvements sanguins. L'Annexine A3 fait partie de ces marqueurs de choix.

La demande de brevet WO 2010/011860, quant à elle, divulgue des méthodes pour la détection du pré-diabète et du diabète. Ces méthodes sont basées sur la comparaison de profils protéomiques qui contiennent au moins une signature d'expression unique, caractéristique du pré-diabète ou du diabète. Ces profils protéomiques contiennent de l'information sur l'expression de différentes protéines dont l'Annexine A3.

Bien que l'Annexine A3 soit reconnue comme un marqueur pour le cancer et autres pathologies, les procédés pour la détecter et la quantifier décrits jusqu'à présent soit manquent de spécificité dans la mesure où les anticorps utilisés présentent des réactions croisées avec d'autres Annexines (cf. WO 2006/125580), soit manquent de sensibilité pour la détecter dans un fluide biologique complexe comme l'urine (cf. WO 2007/141043). De manière générale, les procédés antérieurs manquent de robustesse et ne permettent pas la détection et la quantification fiable de très faibles concentrations en Annexine A3 de l'ordre du ng/mL dans un fluide biologique complexe et en particulier dans le sang ou au moins un de ses dérivés tels que le plasma et le sérum.

Jusqu'à ce jour, aucune technique d'immunodétection et/ou d'immunodosage ne permettait de détecter et/ou doser l'Annexine A3 dans le sang, le plasma ou le sérum (notamment à des quantités de l'ordre du ng/mL). Or un tel dosage sanguin, plasmatique ou sérique est extrêmement souhaitable dans la mesure où il est aisément réalisable (simple prise de sang), reproductible et le résultat obtenu ne repose pas en tout ou partie sur le succès d'une manipulation préalable du patient telle qu'un toucher prostatique visant à récupérer un échantillon d'urine exprimée (dans le cas d'un soupçon de cancer de la prostate).

De manière surprenante, les inventeurs ont découvert qu'afin de remédier à tout ou partie des inconvénients précités, il convenait d'utiliser un couple d'anticorps dont l'un est dirigé contre le premier domaine répété (D1) de l'Annexine A3 d'un mammifère et l'autre est dirigé contre le quatrième domaine répété (D4) de l'Annexine A3 dudit mammifère. Si l'on considère l'Annexine A3 humaine, il convient d'utiliser un couple d'anticorps dont l'un est dirigé contre le premier domaine répété de l'Annexine A3 humaine dont la séquence est identifiée en SEQ ID NO: 1 (acides aminés 27-87 de l'Annexine A3 humaine, selon la numérotation de la base de données UniProtKB de l'ANXA3 complète humaine n° d'accession P12429) et l'autre est dirigé contre le quatrième domaine répété de l'Annexine A3 humaine dont la séquence est identifiée en SEQ ID NO: 2 (acides aminés 258-318, selon la numérotation de la base de données UniProtKB de l'ANXA3 complète humaine n° d'accession P12429).

Dans la description qui suit, la séquence du premier domaine répété de l'Annexine A3 humaine est identifiée en SEQ ID NO: 1, la séquence du quatrième domaine répété de l'Annexine A3 humaine est identifiée en SEQ ID NO: 2, la séquence du deuxième domaine répété de l'Annexine A3 humaine est identifiée en SEQ ID NO: 3, la séquence du troisième domaine répété de l'Annexine A3 humaine est identifiée en SEQ ID NO: 4. La séquence identifiée en SEQ ID NO: 5 correspond à la séquence en acides aminés de l'Annexine A3 humaine complète (UniProtKB n° d'accession P12429).

Par « détecter la présence d'Annexine A3 dans le sang ou au moins un de ses dérivés » (fluides biologiques, tels que plasma, sérum...), l'on entend déceler la présence de cette protéine, en particulier dans les fluides biologiques précités.

Par « doser (quantifier) l'Annexine A3 dans le sang ou au moins un de ses dérivés » (fluides biologiques, tels que plasma, sérum...), l'on entend le fait de déterminer la quantité (par exemple la concentration) d'Annexine A3 en particulier au sein desdits fluides biologiques.

En conséquence, la présente invention a pour objet un procédé de détection et/ou de dosage de l'Annexine A3 d'un mammifère dans le sang ou au moins un de ses dérivés tels que le plasma et le sérum, dans lequel le sang ou au moins un de ses dérivés est mis en contact avec un premier anticorps et un deuxième anticorps, le premier anticorps étant dirigé contre le premier domaine répété de l'Annexine A3 dudit mammifère et le deuxième anticorps étant dirigé contre le quatrième domaine répété de l'Annexine A3 dudit mammifère. Si l'on considère l'Annexine A3 humaine, le premier anticorps est dirigé contre le premier domaine répété de l'Annexine A3 humaine dont la séquence est identifiée en SEQ ID NO: 1 et le deuxième anticorps est dirigé contre le quatrième domaine répété de l'Annexine A3 humaine dont la séquence est identifiée en SEQ ID NO: 2.

En d'autres termes, le premier anticorps reconnaît et se lie/se fixe à tout ou partie du premier domaine répété de l'Annexine A3 du mammifère d'intérêt et le deuxième anticorps reconnaît et se lie/se fixe à tout ou partie du quatrième domaine répété de l'Annexine A3 dudit mammifère.

Cette combinaison spécifique d'un premier anticorps dirigé contre le premier domaine répété de l'Annexine A3 d'un mammifère et d'un deuxième anticorps dirigé contre le quatrième domaine répété de l'Annexine A3 de ce mammifère permet de détecter et/ou doser l'Annexine A3 dans le sang, le plasma ou le sérum (notamment à des quantités de l'ordre du ng/mL) chez ledit mammifère. Ceci est d'autant plus inattendu que si l'on utilise, en lieu et place dudit deuxième anticorps, un anticorps dirigé contre un domaine répété de l'Annexine A3 autre que son quatrième domaine répété (par exemple dirigé contre le premier domaine répété), l'on ne parvient pas à doser voire dans certains cas à détecter l'Annexine A3 dans le sang, le plasma ou le sérum du mammifère en question.

Chez l'être humain, le procédé de détection et/ou de dosage selon l'invention permet de détecter et/ou de doser l'isoforme de 33 kDa [10] de l'Annexine A3 humaine et/ou celui de 36 kDa (correspondant en fait à un groupe d'isoformes hétérogènes [10] ; cf. supra). L'isoforme de 33 kDa est une molécule tronquée de 39 acides aminés du côté N-terminal par rapport à l'isoforme de 36 kDa. En d'autres termes, sa séquence polypeptidique correspond aux acides aminés 40-323 de SEQ ID NO: 5. Ainsi, si le premier anticorps choisi se lie/se fixe au fragment 27-39 du premier domaine répété de l'Annexine A3 humaine, le procédé de dosage détecte et/ou dose uniquement l'isoforme de 36 kDa. En revanche, si le premier anticorps choisi se lie/se fixe au fragment 40-87 du premier domaine répété de l'Annexine A3 humaine, le procédé de dosage détecte et/ou dose tous les isoformes.

Les domaines D1 et D4 de l'Annexine A3 présentent une forte identité chez les mammifères et notamment chez les mammifères couramment étudiés. En conséquence, le procédé et le coffret de la présente invention sont appliqués à la détection et/ou au dosage de l'Annexine A3 dans le sang ou un de ses dérivés, tels que le plasma et le sérum, chez un mammifère.

En particulier, le procédé et le coffret de la présente invention s'appliquent à la détection et/ou au dosage de l'Annexine A3 notamment parmi les espèces suivantes : Homo Sapiens (Homme), Sus scrofa domesticus (Porc), Bos taurus (Taureau), Canis familiaris (Chien), Oryctolagus cuniculus (Lapin), Macaca Mulatta (Macaque rhésus), Gorilla gorilla gorilla (Gorille). Avantageusement, le procédé et le coffret de la présente invention pourront être mis en oeuvre bien évidemment chez l'homme mais encore chez les mammifères dits domestiques (tels que le chat, le chien, etc.), ainsi que chez les primates.

De manière particulièrement intéressante, le procédé de détection et/ou de dosage selon l'invention est mis en oeuvre *in vitro* sur des échantillons biologiques d'origine humaine.

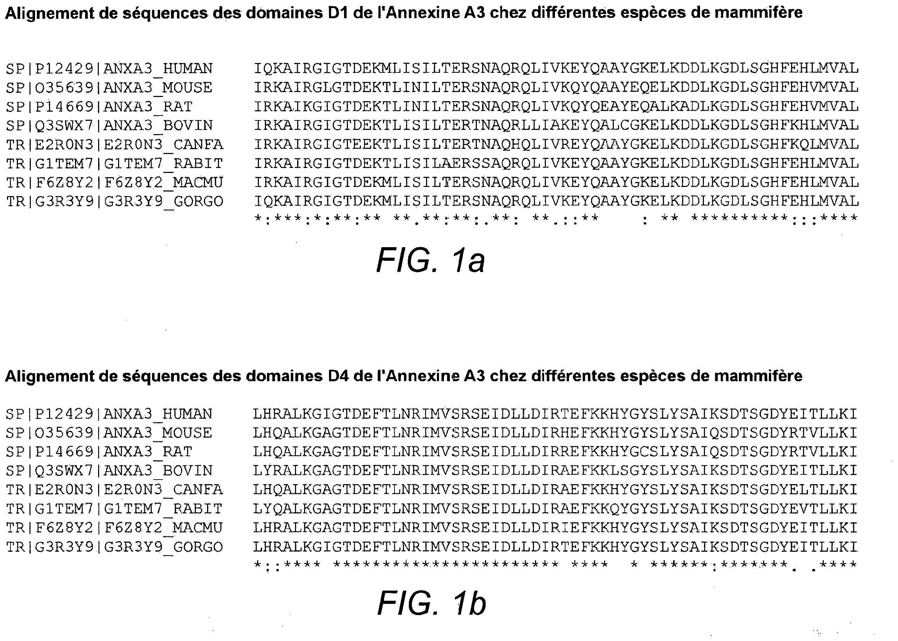

Plus précisément, et ce afin d'analyser l'identité des séquences protéiques D1 et D4 de l'Annexine A3 chez différents mammifères, les séquences en acides aminés des premier et quatrième domaines répétés (respectivement désignés « D1 » et « D4 ») de l'Annexine A3 humaine ont été comparées à celles des premier et quatrième domaines répétés de l'Annexine A3 chez d'autres mammifères. Dans ce but, une interrogation de la base de données UniprotKB (www.uniprot.org) a été réalisée. Le Tableau 1 ci-après indique, pour chaque espèce de mammifère incluse dans la présente analyse, le numéro d'accession de la séquence de la protéine Annexine A3 de l'espèce, ainsi que le nom de l'entrée au sein de la base de données. Pour chaque espèce mammifère, les séquences des domaines D1 et D4 ont été extraites manuellement puis alignées avec la séquence correspondante de l'Annexine A3 humaine en utilisant la fonction « Align » qui est disponible sur l'onglet « Align » sur le site internet d'Uniprot. Cette fonction fait appel au programme Clustal O (v1.0.3). Ledit Tableau 1 présente, pour chacun des domaines D1 et D4, le pourcentage d'identité par rapport à la séquence correspondante de l'Annexine A3 humaine. Les alignements de séquences des domaines D1 et D4 sont respectivement présentés en Figures 1a et 1b.

**Tableau 1**

| No d'accession Unipro | Nom de l'entrée | Organisme | % identité (D1) | % identité (D4) |
|---|---|---|---|---|
| P12429 | ANXA3_HUMAN | Homo sapiens (Homme) | 100% | 100% |
| 035639 | ANXA3_MOUSE | Mus musculus (Souris) | 87% | 89% |
| P14669 | ANXA3_RAT | Rattus norvegicus (Rat) | 82% | 87% |
| Q3SWX7 | ANXA3_BOVIN | Bos taurus (Taureau) | 87% | 92% |
| E2R0N3 | E2R0N3_CANFA | Canis familiaris (Chien) | 87% | 93% |
| G1TEM7 | G1TEM7_RABIT | Oryctolagus cuniculus (Lapin) | 93% | 90% |
| F6Z8Y2 | F6Z8Y2_MACMU | Macaca Mulatta (Macaque rhésus) | 98% | 97% |
| G3R3Y9 | G3R3Y9_GORGO | Gorilla gorilla gorilla (Gorille) | 100% | 100% |

Tel qu'indiqué précédemment, le procédé de dosage selon la présente invention peut être réalisé sur un échantillon de sang ou un de ses fluides dérivés (plasma, sérum etc.). Les définitions des termes « plasma » et « sérum » sont les définitions usuelles utilisées en biologie, à savoir :
- le plasma consiste en la partie liquide du sang, dans laquelle les éléments cellulaires (globules rouges, globules blancs, plaquettes) sont en suspension ; et
- le sérum désigne le liquide se séparant du caillot après coagulation du sang, correspondant au plasma dépourvu de fibrinogène.

L'invention a également pour objet un procédé de détection et/ou de dosage *in vitro* de l'Annexine A3 dans un échantillon biologique d'un mammifère, ledit échantillon biologique étant choisi parmi le sang ou au moins un de ses dérivés tels que le plasma et le sérum, dans lequel l'échantillon biologique est mis en contact avec un premier anticorps et un deuxième anticorps, le premier anticorps étant dirigé contre le premier domaine répété de l'Annexine A3 dudit mammifère et le deuxième anticorps étant dirigé contre le quatrième domaine répété de l'Annexine A3 dudit mammifère.

Avantageusement, le premier anticorps est un anticorps de capture et le deuxième anticorps est un anticorps de détection (révélation).

Selon un mode de réalisation préféré, le premier anticorps est sélectionné parmi les anticorps dirigés contre un épitope dont la séquence en acides aminés comprend au moins 7 acides aminés consécutifs et pas plus de 17 acides aminés consécutifs du premier domaine répété (D1) de l'Annexine A3 du mammifère d'intérêt. Dans le cas du dosage de l'Annexine A3 humaine, le premier anticorps est sélectionné parmi les anticorps dirigés contre un épitope dont la séquence en acides aminés comprend au moins 7 acides aminés consécutifs et pas plus de 17 aminés consécutifs de SEQ ID NO: 1.

Préférablement, le premier anticorps est sélectionné parmi les anticorps dirigés contre un épitope inclus dans le premier domaine répété de l'Annexine A3 dudit mammifère, ledit épitope ayant une séquence en acides aminés sélectionnée parmi les séquences suivantes :
Xaa1-Xaa2-A-Q-Xaa3-Xaa4-L-I-Xaa5-Xaa6-Xaa7-Y-Q-Xaa8-Xaa9-Xaa10-Xaa11 (SEQ ID NO: 37),
L-1-Xaa5-Xaa6-Xaa7-Y-Q-Xaa8-Xaa9-Xaa10-Xaa11 (SEQ ID NO: 38),
1-Xaa5-Xaa6-Xaa7-Y-Q-Xaa8-Xaa9-Xaa10-Xaa11-Xaa12-Xaa13 (SEQ ID NO: 39),
Xaa6-Xaa7-Y-Q-Xaa8-Xaa9-Xaa10-Xaa11 (SEQ ID NO: 40),
Xaa6-Xaa7-Y-Q-Xaa8-Xaa9-Xaa10-Xaa11-Xaa12-Xaa13-L-K-Xaa14-D-L-K-G (SEQ ID NO: 41), à la condition que la séquence en acides aminés SEQ ID NO: 41 soit fusionnée du côté N-terminal à une séquence d'au moins 30 acides aminés,
DLSGHF-Xaa20-Xaa21-Xaa22 (SEQ ID NO: 42),
LSGHF-Xaa20-Xaa21 (SEQ ID NO: 43),
dans lequel :
- Xaa1 représente un résidu sérine, un résidu thréonine, ou un acide aminé analogue,
- Xaa2 représente un résidu asparagine, un résidu sérine ou un acide aminé analogue,
- Xaa3 représente un résidu arginine, un résidu histidine ou un acide aminé analogue,
- Xaa4 représente un résidu glutamine, un résidu leucine ou un acide aminé analogue,
- Xaa5 représente un résidu valine, un résidu alanine ou un acide aminé analogue,
- Xaa6 représente un résidu lysine, un résidu arginine ou un acide aminé analogue,
- Xaa7 représente un résidu acide glutamique, un résidu glutamine ou un acide aminé analogue,
- Xaa8 représente un résidu alanine, un résidu acide glutamique ou un acide aminé analogue,
- Xaa9 représente un résidu alanine, un résidu leucine ou un acide aminé analogue,
- Xaa10 représente un résidu tyrosine, un résidu cystéine ou un acide aminé analogue,
- Xaa11 représente un résidu glycine, un résidu acide glutamique ou un acide aminé analogue,
- Xaa12 représente un résidu lysine, un résidu glutamine ou un acide aminé analogue,
- Xaa13 représente un résidu acide glutamique, un résidu alanine ou un acide aminé analogue,
- Xaa14 représente un résidu acide aspartique, un résidu alanine ou un acide aminé analogue,
- Xaa20 représente un résidu acide glutamique, un résidu lysine ou un acide aminé analogue,
- Xaa21 représente un résidu histidine, un résidu glutamine ou un acide aminé analogue,
- Xaa22 représente un résidu leucine, un résidu valine ou un acide aminé analogue.

Avantageusement :
- Xaa1 représente un résidu sérine ou un résidu thréonine,
- Xaa2 représente un résidu asparagine ou un résidu sérine,
- Xaa3 représente un résidu arginine ou un résidu histidine,
- Xaa4 représente un résidu glutamine ou un résidu leucine,
- Xaa5 représente un résidu valine ou un résidu alanine,
- Xaa6 représente un résidu lysine ou un résidu arginine,
- Xaa7 représente un résidu acide glutamique ou un résidu glutamine,
- Xaa8 représente un résidu alanine ou un résidu acide glutamique,
- Xaa9 représente un résidu alanine ou un résidu leucine,
- Xaa10 représente un résidu tyrosine ou un résidu cystéine,
- Xaa11 représente un résidu glycine ou un résidu acide glutamique,
- Xaa12 représente un résidu lysine ou un résidu glutamine,
- Xaa13 représente un résidu acide glutamique ou un résidu alanine,
- Xaa14 représente un résidu acide aspartique ou un résidu alanine,
- Xaa20 représente un résidu acide glutamique ou un résidu lysine,
- Xaa21 représente un résidu histidine ou un résidu glutamine,
- Xaa22 représente un résidu leucine ou un résidu valine.

L'expression « acide aminé analogue » se réfère à un acide aminé qui, lorsqu'il remplace l'acide aminé présent dans l'épitope d'intérêt, n'entraîne aucune modification substantielle de la réactivité antigénique dudit épitope vis à vis de l'anticorps d'intérêt.

Les analogues particulièrement préférés incluent ceux présentant des substitutions conservatrices en nature, c'est-à-dire les substitutions qui prennent place dans une famille d'acides aminés. Il existe plusieurs classifications d'acides aminés en famille, comme cela est connu de l'homme du métier. Ainsi, selon un exemple de classification, les acides aminés peuvent être divisés en quatre familles, à savoir (1) les acides aminés acides tels que l'acide aspartique ou l'acide glutamique, (2) les acides aminés basiques tels que la lysine, l'arginine et l'histidine, (3) les acides aminés non polaires tels que la leucine, l'isoleucine et la méthionine et (4) les acides aminés non chargés polaires tels que la glycine, l'asparagine, la glutamine, la sérine, la thréonine et la tyrosine. La phénylalanine, le tryptophane et la tyrosine sont parfois classés en acides aminés aromatiques. Par exemple, l'on peut prédire, de façon raisonnable, qu'un remplacement isolé d'un résidu leucine par un résidu isoleucine ou valine, d'un résidu aspartate par un résidu glutamate, d'un résidu thréonine par un résidu sérine, ou un remplacement conservateur similaire d'un acide aminé par un autre acide aminé ayant un rapport structurel, n'aura pas d'effet majeur sur l'activité biologique. Un autre exemple de méthode permettant de prédire l'effet sur l'activité biologique d'une substitution d'acide aminé a été décrite par Ng et Henikoff, 2001 [27].

En particulier, dans le cas de la détection et/ou du dosage de l'Annexine A3 humaine, le premier anticorps est sélectionné parmi les anticorps dirigés contre un épitope inclus dans SEQ ID NO: 1, ledit épitope ayant une séquence en acides aminés sélectionnée parmi les séquences suivantes :
SNAQRQLIVKEYQAAYG (SEQ ID NO: 10),
LIVKEYQAAYG (SEQ ID NO: 11),
IVKEYQAAYGKE (SEQ ID NO: 12),
KEYQAAYG (SEQ ID NO: 13),
DLSGHFEHL (SEQ ID NO: 14),
LSGHFEH (SEQ ID NO: 15),
   et
KEYQAAYGKELKDDLKG (SEQ ID NO: 22), à la condition que la séquence en acides aminés SEQ ID NO: 22 soit fusionnée du côté N-terminal à une séquence d'au moins 30 acides aminés.

Si l'on souhaite détecter et/ou doser l'Annexine A3 chez un mammifère autre que l'homme - par exemple un des mammifères listés dans le Tableau 1 supra - le premier anticorps peut être sélectionné parmi les anticorps dirigés contre un épitope dont la séquence en acides aminés correspond à l'une des séquences SEQ ID NO : 10, 11, 12, 13, 14, 15 et 22 chez le mammifère d'intérêt. A titre d'exemple, dans le cadre de la détection et/ou du dosage de l'Annexine A3 du chien (Canis Familiaris), ledit premier anticorps est dirigé contre un épitope inclus dans le premier domaine répété de l'Annexine A3 canine, ledit épitope ayant une séquence en acides aminés sélectionnée parmi les séquences suivantes :
- TNAQHQLIVREYQAAYG (SEQ ID NO: 44),
- LIVREYQAAYG (SEQ ID NO: 45),
- IVREYQAAYGKE (SEQ ID NO: 46),
- REYQAAYG (SEQ ID NO: 47),
- DLSGHFKQL (SEQ ID NO: 48),
- LSGHFKQ (SEQ ID NO: 49), et
- REYQAAYGKELKDDLKG (SEQ ID NO: 50), à la condition que la séquence en acides aminés SEQ ID NO: 50 soit fusionnée du côté N-terminal à une séquence d'au moins 30 acides aminés.

Concernant le deuxième anticorps susvisé, ce dernier est sélectionné, de préférence, parmi les anticorps dirigés contre un épitope dont la séquence en acides aminés comprend au moins 7 acides aminés consécutifs et pas plus de 50 acides aminés consécutifs du quatrième domaine répété de l'Annexine A3 dudit mammifère. Si le mammifère en question est l'homme, le deuxième anticorps est préférablement sélectionné parmi les anticorps dirigés contre un épitope dont la séquence en acides aminés comprend au moins 7 acides aminés consécutifs et pas plus de 7 acides aminés consécutifs de SEQ ID NO: 2.

Avantageusement, le deuxième anticorps est sélectionné parmi les anticorps dirigés contre un épitope dont la séquence en acides aminés correspond à la séquence en acides aminés commençant au résidu 3 et se terminant au résidu 49 du quatrième domaine répété de l'Annexine A3 du mammifère d'intérêt. Là encore, si le mammifère est l'homme, le deuxième anticorps est sélectionné parmi les anticorps dirigés contre un épitope dont la séquence en acides aminés correspond à la séquence en acides aminés commençant au résidu 3 et se terminant au résidu 49 de SEQ ID NO : 2.

Selon un mode de réalisation préféré de la présente invention, le deuxième anticorps reconnaît un épitope comprenant un résidu lysine en position 6 du quatrième domaine répété de l'Annexine A3 d'un mammifère et/ou un résidu acide aspartique en position 49 dudit quatrième domaine répété de l'Annexine A3 dudit mammifère. Préférablement, ledit épitope comprend un résidu lysine en position 6 du quatrième domaine répété de l'Annexine A3 d'un mammifère et un résidu acide aspartique en position 49 dudit quatrième domaine répété de l'Annexine A3 dudit mammifère.

Dans le cadre d'un procédé de détection et/ou de dosage de l'Annexine A3 humaine, le deuxième anticorps reconnaît un épitope comprenant un résidu lysine en position 6 de SEQ ID NO: 2 et/ou un résidu acide aspartique en position 49 de SEQ ID NO: 2. Avantageusement, l'épitope reconnu par ce deuxième anticorps comprend à la fois un résidu lysine en position 6 de SEQ ID NO : 2 et un résidu acide aspartique en position 49 de SEQ ID NO : 2. En effet, la lysine en position 6 de SEQ ID NO: 2 et l'acide aspartique en position 49 de SEQ ID NO: 2 se sont révélés être - lors des processus de « mapping » - les résidus les plus importants dans l'interaction anticorps-antigène entre le deuxième anticorps et le quatrième domaine répété de l'Annexine A3 humaine de séquence SEQ ID NO: 2 (cf. Exemple 7, section « Localisation précise de l'épitope des anticorps 13A12G4H2 et 1F10A6 »).

Selon un mode de réalisation particulièrement préféré, le deuxième anticorps reconnaît et se lie à un épitope qui comprend, en sus des résidus lysine en position 6 et/ou acide aspartique en position 49 de D4, un résidu arginine ou glutamine en position 3 du quatrième domaine répété de l'Annexine A3 dudit mammifère et un résidu isoleucine ou alanine en position 8 dudit quatrième domaine répété de l'Annexine A3 dudit mammifère. Selon ce mode de réalisation préféré, lorsque l'on considère la détection et/ou le dosage de l'Annexine A3 humaine, le deuxième anticorps est dirigé contre un épitope qui comprend, en sus des résidus lysine en position 6 et/ou acide aspartique en position 49 de SEQ ID NO 2, les résidus arginine en position 3 et isoleucine en position 8 de SEQ ID NO: 2. En effet, la présence de ces acides aminés spécifiques aux positions 3, 6, 8 et 49 de SEQ ID NO: 2 (correspondant respectivement aux positions 260, 263, 265 et 306 de l'Annexine A3 humaine de séquence SEQ ID NO: 5) apparaît être primordiale par rapport à la capacité de fixation des anticorps reconnaissant le quatrième domaine répété de l'Annexine A3 humaine (SEQ ID NO: 2), à savoir les anticorps 13A12G4H2 et 1F10A6, tels qu'indiqués au sein de l'Exemple 7 infra.

De manière préférée, l'épitope reconnu par le deuxième anticorps comprend un résidu glycine en position 7 du quatrième domaine répété de l'Annexine A3 dudit mammifère, un résidu isoleucine ou alanine en position 8 du quatrième domaine répété de l'Annexine A3 dudit mammifère et un résidu glycine en position 9 dudit quatrième domaine répété de l'Annexine A3 dudit mammifère. Si l'on considère l'Annexine A3 humaine, l'épitope reconnu par le deuxième anticorps comprend, de préférence, un résidu glycine en position 7 de SEQ ID NO: 2, un résidu isoleucine en position 8 de SEQ ID NO: 2 et un résidu glycine en position 9 de SEQ ID NO: 2.

Selon un mode de réalisation particulièrement préféré, et dans le cadre de la détection et/ou du dosage de l'Annexine A3 humaine, le deuxième anticorps reconnaît un épitope qui comprend un résidu arginine en position 3 de SEQ ID NO: 2, un résidu lysine en position 6 de SEQ ID NO: 2, un résidu glycine en position 7 de SEQ ID NO: 2, un résidu isoleucine en position 8 de SEQ ID NO: 2, un résidu glycine en position 9 de SEQ ID NO: 2, ainsi qu'un résidu acide aspartique en position 49 de SEQ ID NO: 2.

Selon un mode de réalisation particulièrement préféré, le mammifère est l'homme et le premier anticorps est dirigé contre le premier domaine répété de ladite Annexine A3 humaine dont la séquence est identifiée en SEQ ID NO: 1 et le deuxième anticorps est dirigé contre le quatrième domaine répété de l'Annexine A3 humaine dont la séquence est identifiée en SEQ ID NO: 2.

Les anticorps spécifiques du premier domaine répété de l'Annexine A3 humaine (SEQ ID NO: 1) et ceux spécifiques du quatrième domaine répété de l'Annexine A3 humaine (SEQ ID NO: 2), sont des anticorps qui présentent une affinité élevée avec une constante d'affinité d'au moins 10⁻⁹, de préférence d'au moins 10⁻¹⁰ et qui de plus présentent une constante de dissociation faible inférieure à 2 10⁻³ s⁻¹, de préférence encore inférieure à 5 10⁻⁴ s⁻¹.

De préférence, ledit premier anticorps et/ou ledit deuxième anticorps est/sont des anticorps monoclonaux. Avantageusement, à la fois le premier anticorps et le deuxième anticorps sont des anticorps monoclonaux. Concernant la détection et/ou le dosage d'Annexine A3 humaine, les anticorps préférés sont les anticorps suivants : TGC42, TGC43, TGC44 utilisés en tant que premier anticorps (anticorps de capture selon un mode de réalisation préféré) et 13A12G4H2 et 1F10A6 utilisés comme deuxième anticorps (anticorps de détection selon ledit mode de réalisation préféré). Le couple préférentiel est constitué par le premier anticorps (anticorps de capture selon un mode de réalisation préféré) TGC43 ou TGC44 et le deuxième anticorps (anticorps de détection selon ledit mode de réalisation préféré) 13A12G4H2.

L'invention concerne également un procédé selon la présente invention pour le diagnostic *in vitro* et/ou le suivi *in vitro* de l'évolution d'une pathologie comprise dans le groupe constitué par :
- les cancers, tels que les cancers urogénitaux et notamment le cancer de la prostate, le cancer colorectal, le cancer du poumon, le cancer du pancréas, le cancer de la thyroïde papillaire, le cancer du sein,
- les désordres inflammatoires, tels que le syndrome de réponse inflammatoire systémique, le sepsis, la polyarthrite rhumatoïde, la maladie de Crohn, le diabète.

L'invention a également pour objet un coffret d'immunodétection et/ou d'immunodosage d'un échantillon biologique d'un mammifère, ledit échantillon biologique étant choisi parmi le sang ou au moins un de ses dérivés tels que le plasma et le sérum, ledit coffret comprenant un premier anticorps et un deuxième anticorps, le premier anticorps étant dirigé contre le premier domaine répété de l'Annexine A3 dudit mammifère et le deuxième anticorps étant dirigé contre le quatrième domaine de l'Annexine A3 dudit mammifère. Ledit coffret permet de mettre en oeuvre le procédé de détection et/ou de dosage de l'Annexine A3 d'un mammifère selon la présente invention. Lesdits premier et deuxième anticorps sont tels que définis précédemment. De préférence, ledit coffret d'immunodétection et/ou d'immunodosage sanguin, plasmatique ou sérique permet de détecter et/ou doser l'Annexine A3 humaine. Dans ce cas, le premier anticorps est dirigé contre le premier domaine répété de l'Annexine A3 humaine, de séquence SEQ ID NO: 1, et le deuxième anticorps est dirigé contre le quatrième domaine de l'Annexine A3 humaine dont la séquence est identifiée en SEQ ID NO: 2.

Selon un mode de réalisation préféré, l'invention a trait à un coffret d'immunodétection et/ou d'immunodosage d'un échantillon biologique d'un mammifère, ledit échantillon biologique étant choisi parmi le sang ou au moins un de ses dérivés tels que le plasma et le sérum, ledit coffret comprenant :
- un premier anticorps dirigé contre le premier domaine répété de l'Annexine A3 dudit mammifère, tel que défini dans la présente invention;
- un deuxième anticorps dirigé contre le quatrième domaine répété de l'Annexine A3 dudit mammifère, tel que défini dans la présente invention,
ledit coffret permettant de mettre en oeuvre le procédé selon la présente invention.

Les premier et deuxième anticorps - respectivement anticorps de capture et anticorps de détection selon un mode de réalisation préféré - répondent aux mêmes définitions et présentent les mêmes caractéristiques que celles présentées ci-dessus par rapport au procédé de détection et/ou de dosage selon la présente invention.

Avantageusement, ledit coffret comprend un moyen de calibration approprié.

Par « moyen de calibration approprié », l'on entend, au sens de la présente invention, un moyen de calibration adapté à la détection et/ou au dosage de l'Annexine A3 dans le sang ou au moins un de ses dérivés tels que le plasma et le sérum.

Selon un mode de réalisation préféré, le moyen de calibration comprend un échantillon de sang, de plasma ou de sérum de même origine (même mammifère). La concentration en Annexine A3 au sein de cet échantillon est connue. Avantageusement, ce sang, plasma ou sérum de même origine est dilué dans un diluant approprié de manière à établir une gamme étalon. Préférablement, le diluant approprié est un tampon approprié. A titre d'exemple, le susdit moyen de calibration comprend :
- de l'Annexine A3 d'origine endogène (purifiée ou non) diluée dans un tampon approprié, ou
- de l'Annexine A3 d'origine exogène (par exemple de l'Annexine A3 recombinante) diluée dans un tampon approprié.

A titre de tampon approprié, l'on peut notamment citer :
- Tris 0,1 M, NACl 10-200 mM, BSA 1-20%,
- Tris 0,1 M, NACl 10-200 mM, sérum de veau 1-20 %,
- Tris 0,1 M, NACl 10-200 mM, sérum de cheval 1-20%,
- Phosphate de potassium 20-100 mM, NACl 10-200 mM, BSA 1-20%,
- Glycine 5-200 mM, HEPES 5-200 mM , BSA 1-20%.

Bien évidemment, l'homme du métier déterminera aisément, à partir de ses connaissances générales, les tampons appropriés permettant d'obtenir un moyen de calibration approprié selon la présente invention.

En particulier, le tampon peut comprendre ou non un ou des détergents.

De plus, afin d'améliorer les performances du coffret, des composés additionnels peuvent être ajoutés, tels que :
- sucres : fructose, lactose, saccharose, maltose, tréhalose, etc. ;
- anti oxydants : acide ascorbique, méthionine, etc.;
- anti réducteurs ;
- anti chélatants : EDTA, etc. ;
- anti protéases : aprotinine, etc. ;
- polymères pour piéger l'eau : polyvinylpyrolidone, dextran, etc. ;
- stabilisants : acide citrique, etc. ;
- antibiotiques, antimycotiques, bactériostatiques : sodium azide, etc.

Tel que mentionné précédemment, il est essentiel que le moyen de calibration soit adapté à la détection et/ou au dosage dans l'échantillon biologique choisi, à savoir le sang ou au moins un de ses dérivés tels que le plasma et le sérum. Ainsi, un moyen de calibration utilisé dans le cadre de la détection et/ou du dosage de l'Annexine A3 humaine dans de l'urine exprimée ne convient pas au coffret d'immunodétection et/ou d'immunodosage sanguin, plasmatique ou sérique de l'invention.

Un autre objet de l'invention concerne l'utilisation d'un coffret d'immunodétection et/ou d'immunodosage selon la présente invention pour la mise en oeuvre du procédé de détection et/ou de dosage susvisé.

A la fois le procédé de détection et/ou de dosage de l'Annexine A3 d'un mammifère (de préférence l'homme) et le coffret d'immunodétection et/ou d'immunodosage selon la présente invention peuvent être employés, de manière efficace, pour effectuer le diagnostic *in vitro* et/ou le suivi *in vitro* de l'évolution de diverses pathologies et affections. Parmi les pathologies pouvant ainsi être diagnostiquées et/ou suivies, l'on peut citer : les cancers et en particulier les cancers urogénitaux (notamment les cancers de la prostate), le cancer colorectal, le cancer du poumon, le cancer du pancréas, le cancer de la thyroïde papillaire, le cancer du sein, etc. Peuvent également être diagnostiquées/suivies les pathologies inflammatoires, et en particulier les pathologies inflammatoires. Au titre de pathologies inflammatoires, l'on peut notamment citer le syndrome de réponse inflammatoire systémique (SIRS en langue anglaise), la polyarthrite rhumatoïde, la maladie de Crohn, le diabète, etc. Cette liste n'est pas exhaustive.

Par anticorps, on entend un anticorps polyclonal, un anticorps monoclonal, un anticorps humanisé, un anticorps humain ou un fragment desdits anticorps, en particulier les fragments Fab, Fab', F(ab')2, ScFv, Fv, Fd. La condition requise est que lesdits anticorps doivent être spécifiques de l'Annexine A3, c'est à dire qu'ils ne présentent pas de réactions croisées avec d'autres Annexines et qu'ils sont spécifiques du premier domaine répété de l'Annexine A3 ou spécifiques du quatrième domaine répété de l'Annexine A3 ; les anticorps présentant les affinités les plus élevées et les constantes de dissociation les plus faibles étant les anticorps préférés.

Les anticorps polyclonaux peuvent être obtenus par immunisation d'un animal avec l'immunogène approprié, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps.

Les anticorps monoclonaux peuvent être obtenus par la technique des hybridomes dont le principe général est rappelé ci-dessous.

Dans un premier temps, on immunise un animal, généralement une souris avec l'immunogène approprié, dont les lymphocytes B sont alors capables de produire des anticorps contre cet antigène. Ces lymphocytes producteurs d'anticorps sont ensuite fusionnés avec des cellules myélomateuses "immortelles" (murines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis de la protéine pourront être testées par exemple en ELISA, par immunotransfert (Western blot) en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps monoclonaux peuvent être également des anticorps recombinants obtenus par génie génétique, par des techniques bien connues de l'homme du métier.

L'anticorps de capture est de préférence fixé, directement ou indirectement, sur un support solide, par exemple un cône, un puits d'une plaque de microtitration, etc.

L'anticorps de détection est marqué à l'aide d'un réactif marqueur capable de générer directement ou indirectement un signal détectable. Une liste non limitative de ces réactifs marqueurs consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules fluorescentes telles que les Alexa ou les phycocyanines,
- les molécules radioactives comme le ³²P, le ³⁵S ou le ¹²⁵I.

Des systèmes indirects de détection peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine. Dans ce cas, c'est le ligand qui porte le partenaire de liaison. L'anti-ligand peut être détectable directement par les réactifs marqueurs décrits au paragraphe précédent ou être lui-même détectable par un ligand/anti-ligand.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR98/10084 ou WO-A-95/08000 de la Demanderesse.

Selon le type de marquage utilisé, l'homme du métier ajoutera des réactifs permettant la visualisation du marquage.

Selon un mode de réalisation préféré, le procédé de l'invention est un immunodosage de type « sandwich » effectué sur un prélèvement de sang ou dans les fluides biologiques qui en dérivent comme le sérum ou le plasma, avec ou sans traitement d'échantillon.

L'invention sera mieux comprise à l'aide des exemples suivants donnés à titre illustratif et non limitatif, ainsi qu'à l'aide des figures annexées.

### FIGURES

Les Figures 1a et 1b représentent respectivement les alignements des séquences protéiques des premier et quatrième domaines répétés de l'Annexine A3 chez huit mammifères de référence (dont l'être humain). Dans la ligne consensus en bas de chaque alignement, l'astérisque (*) indique une position qui correspond à un résidu totalement conservé ; les deux-points (:) indiquent une position très conservée, avec des résidus qui possèdent une similitude forte (= score > 0,5 dans la matrice PAM 250 de Gonnet) ; le point (.) indique une position conservée de manière modérée, avec des résidus qui possèdent une similitude modérée (= score < 0,5 dans la matrice PAM 250 de Gonnet). Ladite matrice de Gonnet et al. à laquelle il est précédemment fait référence est décrite dans la publication suivante : Gonnet G.H., Cohen M.A., Benner S.A.; "Exhaustive matching of the entire protein sequence database."; Science 256:1443-1445(1992) [25].

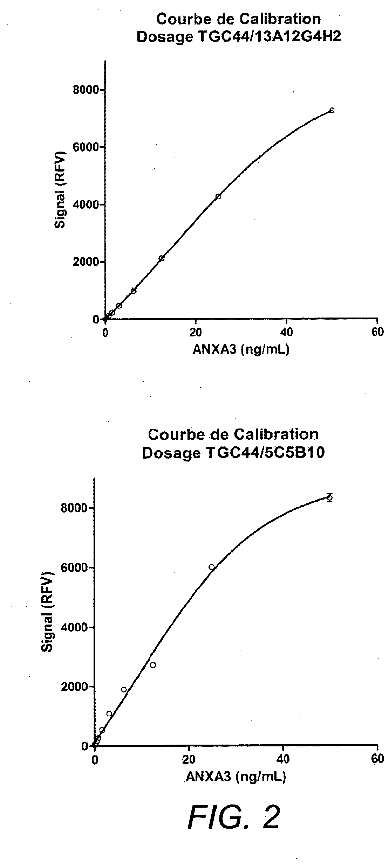

La Figure 2 représente les courbes de calibration des dosages ELISA TGC44/13A12G4H2 (groupe 1) et TGC44/5C5B10 (groupe 2). Pour chaque dosage, la courbe de calibration a été établie en dosant une gamme de concentration de la protéine Annexine A3 humaine native purifiée. La courbe de calibration a été tracée en reportant en abscisse la concentration d'Annexine A3 humaine et en ordonnée le signal mesuré par le VIDAS® en RFV.

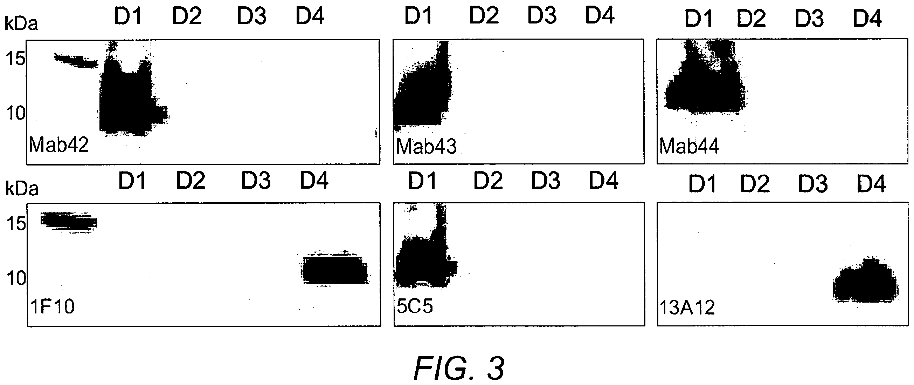

La Figure 3 représente la réactivité des 4 domaines répétés D1 à D4 de l'Annexine A3 humaine, exprimés sous une forme recombinante, avec les 6 anticorps monoclonaux anti-Annexine A3 indiqués et analysés par la technique du Western blot. Pour les gels TGC42 et 1F10A6, le premier puits correspond au marqueur de poids moléculaire.

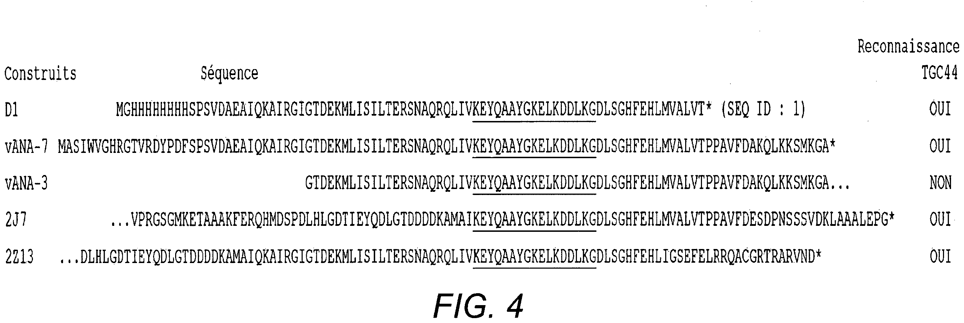

La Figure 4 est un alignement de séquences des différentes protéines recombinantes exprimant des domaines d'Annexine A3 humaine et récapitule l'immunoréactivité de chacun de ces recombinants avec l'anticorps monoclonal TGC44, analysée par la technique du Western blot. Les trois points de suspension à l'extrémité N-terminale ou C-terminale d'une séquence indiquent qu'elle continue dans la construction, même si elle n'est pas représentée en totalité sur la figure. Le codon stop est représenté par une étoile (*).

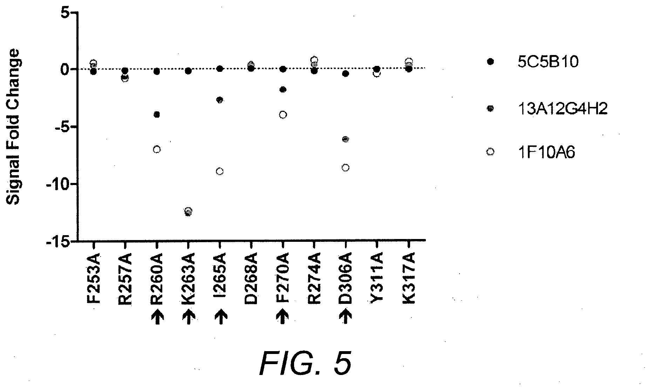

La Figure 5 illustre l'impact des mutations alanine du domaine D4 sur la reconnaissance de l'Annexine 3 humaine par les anticorps 13A12G4H2 et 1F10A6. L'analyse a été faite par la technique ELISA et l'anticorps de capture est le TGC44. Les anticorps 13A12G4H2, 1F10A6 testés ont été utilisés en détection. La position des mutations alanine sur la séquence protéique de l'Annexine A3 humaine est représentée en abscisse. L'ordonnée est le « signal fold change » qui correspond au logarithme en base 2 (signal protéine mutée / signal protéine non mutée). L'anticorps 5C5B10 qui n'est pas dirigé contre le domaine D4 n'est pas impacté, il sert de contrôle. Les flèches indiquent les mutations pour lesquelles il y a perturbation du signal de reconnaissance pour 13A12G4H2 et 1F10A6, qui permettent de définir les résidus impliqués dans la fixation de ces anticorps.

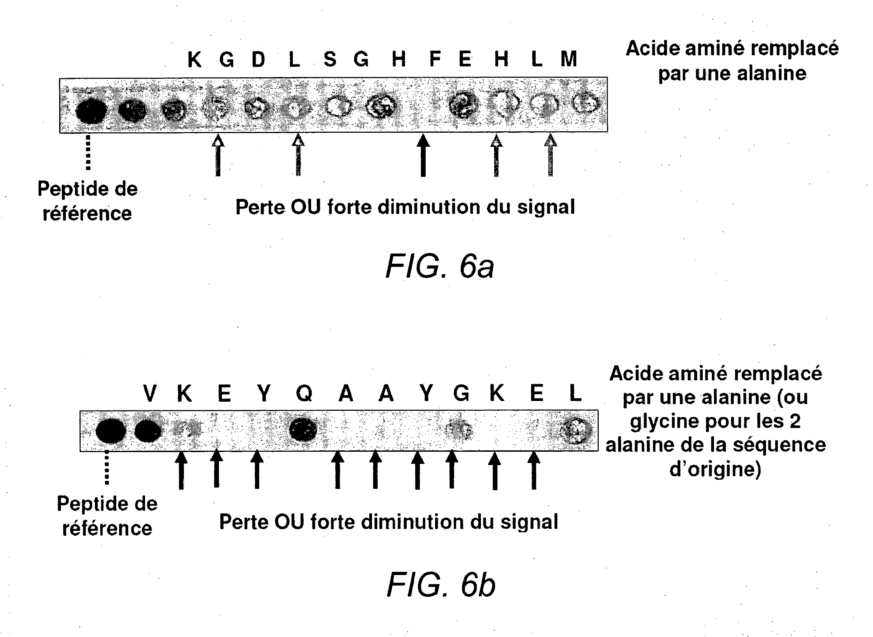

La Figure 6a correspond à l'immunorévélation de la membrane ALAscan du peptide KGDLSGHFEHLM (résidus 73 à 84) de l'Annexine A3 comprenant l'épitope reconnu par l'anticorps 5C5B10 avec cet anticorps. La flèche noire indique une perte totale du signal ; les flèches grises indiquent une diminution importante du signal.
La Figure 6b correspond à l'immunorévélation de la membrane ALAscan du peptide VKEYQAAYGKEL (résidus 57-68) de l'Annexine A3 comprenant l'épitope reconnu par l'anticorps TGC43 avec cet anticorps. La flèche noire indique une perte totale ou une diminution très importante du signal.

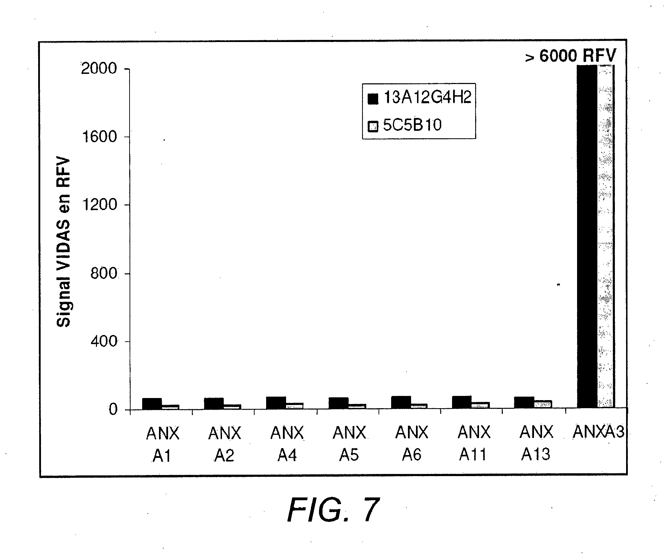

La Figure 7 illustre l'absence de réactivité croisée des formats de dosage ELISA décrits avec 7 autres protéines de la famille des Annexines. Le graphique représente le signal ELISA obtenu en « relative fluorescence units » (RFV) sur le VIDAS® pour chacun des formats de test TGC44/5C5B10 et TGC44/13A12G4H2 et pour chaque Annexine indiquée en abscisse. A titre de comparaison, dans les conditions expérimentales utilisées, l'Annexine A3 permettait d'obtenir un signal supérieur à 6000 RFV avec chacun des formats de test.

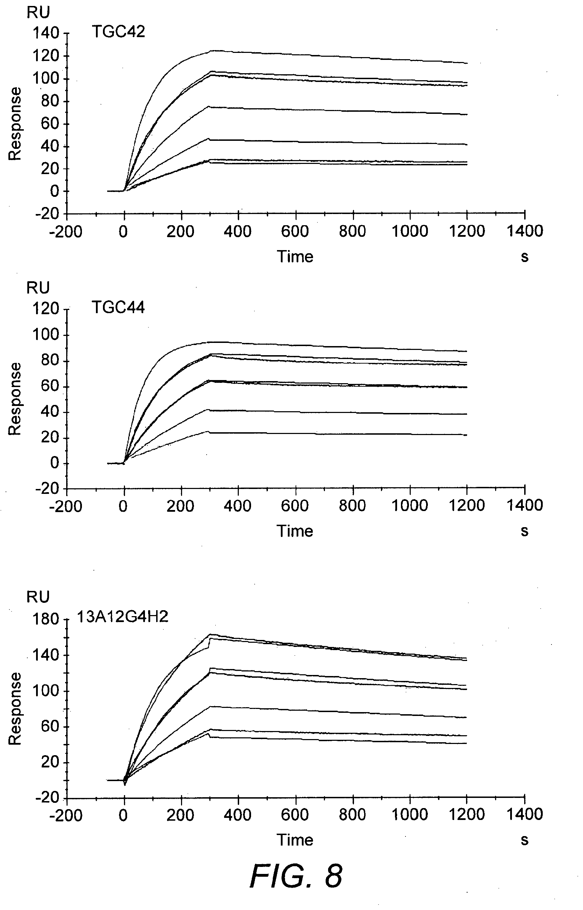

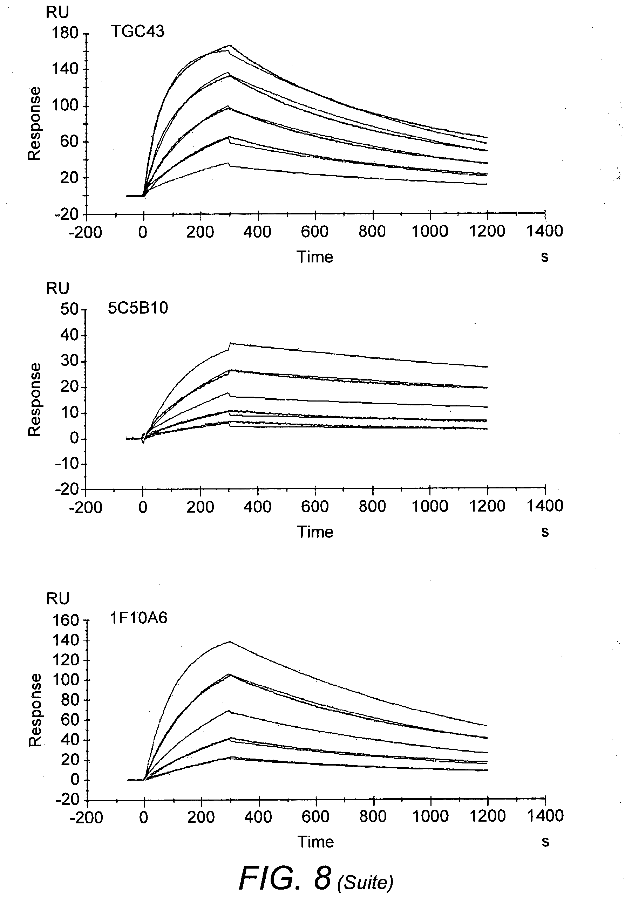

La Figure 8 représente les sensorgrammes obtenus pour chacun des 6 anticorps monoclonaux caractérisés au Biacore T100. Les graphiques représentent le signal de résonance mesuré en « resonance units » (RU) en fonction du temps. Chaque courbe d'un graphique représente l'ensemble des mesures effectuées pour une concentration d'Annexine A3 donnée. Pour chaque anticorps 9 dilutions comprises entre 0 et 64 nM d'Annexine A3 ont été analysées et sont représentées.

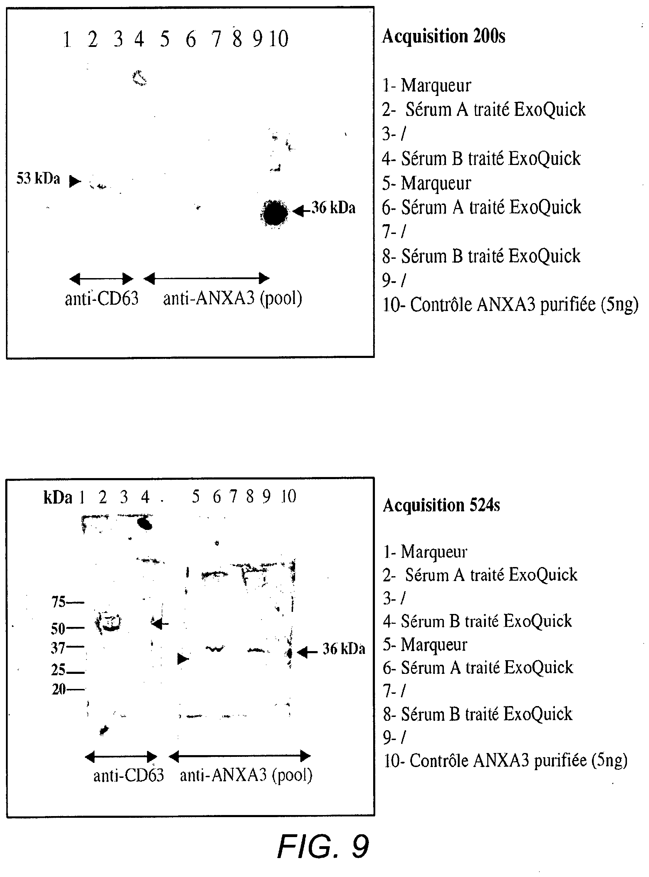

La Figure 9 représente l'analyse en Western blot des exosomes purifiés (traitement ExoQuick) à partir des sérums de 2 patients ayant le cancer de la prostate. Les membranes ont été incubeées soit avec un anticorps anti-CD63, soit avec un anticorps anti-ANXA3.

### EXEMPLES

### Exemple 1 : Détection de l'Annexine A3 humaine par ELISA dans le sérum de patients ayant un cancer de la prostate

### Obtention des anticorps monoclonaux

Les expériences d'immunisation ont été réalisées chez des souris BALB/c (H-2^{d}) femelles âgées de six à huit semaines au moment de la première immunisation. La protéine Annexine A3 native humaine a été achetée auprès de la société Arodia Arotech Diagnostic™ (Cat No. 25592), elle est purifiée à partir de neutrophiles humaines. Cette protéine a été mélangée volume pour volume avec l'adjuvant de Freund (Sigma Aldrich™), préparé sous forme d'émulsion eau-dans-huile et dont il est connu qu'il présente un bon pouvoir immunogène. Les souris ont reçu trois doses successives de 10 µg d'immunogène à zéro, deux et quatre semaines. Toutes les injections sont réalisées par voie sous cutanée. La première injection est faite en mélange avec l'adjuvant de Freund complet, les deux suivantes se font en mélange avec l'adjuvant de Freund incomplet. Entre J50 et J70 après la première injection, les réponses humorales ont été restimulées avec une injection intraveineuse de 100 µg de la protéine native.

Afin de suivre l'apparition des anticorps, on effectue régulièrement sur les souris des prélèvements de sang. La présence des anticorps anti-ANXA3 est testée en utilisant un ELISA. La protéine d'intérêt est utilisée en capture (1 µg/puit), après saturation on fait réagir avec l'antigène différentes dilutions de sérums à tester (incubation à 37°C, pendant 1h). Les anticorps spécifiques présents dans le sérum sont révélés par un anticorps de chèvre anti-IgG de souris AffiniPure™ conjugué à la phosphatase alcaline (H+L, Jacskon Immunoresearch, Cat no. 115-055-146), qui se lie aux anticorps recherchés (0,1 µg/puit).

Trois jours après la dernière injection, une des souris immunisées a été sacrifiée ; le sang et la rate ont été prélevés. Les splénocytes obtenues à partir de la rate ont été mises en culture avec les cellules de myélome Sp2/0-Ag14 pour qu'elles fusionnent et s'immortalisent, selon le protocole décrit par [22, 23]. Après une période d'incubation de 12-14 jours, les surnageants d'hybridomes obtenus ont été criblés pour déterminer la présence d'anticorps anti-ANXA3 en utilisant le test ELISA décrit dans le paragraphe précédent. L'immunogène (ANXA3 native), l'Annexine A3 recombinante produite en *E. coli,* et différentes cellules humaines exprimant l'ANXA3 ont été utilisés successivement pour cribler les surnageants d'hybridomes. Les colonies d'hybridomes positives ont été sous-clonées deux fois selon la technique de la dilution limite, bien connue par l'homme du métier.

Les anticorps monoclonaux anti-Annexine A3 suivants ont ainsi été obtenus : 5C5B10, 13A12G4H2, 9C6B4, 6D9D10, 1F10A6.

### Sélection des anticorps monoclonaux anti-ANXA3 pour dosage par immunoessai de l'ANXA3

La complémentarité des différents anticorps anti-ANXA3 obtenus comme décrit ci-dessus et des anticorps TGC42, TGC43 et TGC44 décrits dans la demande de brevet WO 2010/034825 a été analysée en utilisant comme antigène l'ANXA3 humaine native (immunogène) diluée en tampon PBS, en réalisant un immunoessai de type sandwich. Ce type de dosage peut être réalisé en microplaque, de façon automatisée ou manuelle, ou encore en utilisant des automates d'immunoanalyse comme le VIDAS® (bioMérieux).
Les réactifs du kit VIDAS® HBs Ag Ultra (bioMérieux, Cat no 30315) ont été utilisés, tels que décrits dans la notice correspondante (réf. 11728 D-FR-2005/5), et modifiés ainsi :
- Des cônes ont été sensibilisés avec un des anticorps de capture à tester, TGC42, TGC43, TGC44 ou 9C6B4 à une concentration de 10 µg /mL.
- Le contenu du deuxième puits de la cartouche HBs Ag Ultra a été remplacé par 300 µL d'anticorps de révélation à tester (5C5B10, 13A12G4H2, 9C6B4, 6D9D10, 1F10A6, TGC42, TGC43, TGC44), couplés à la biotine, dilués à 1µg/mL dans le tampon du deuxième puits du kit VIDAS® HBs Ag Ultra (puits X1) contenant du sérum de chèvre et de l'azodure de sodium à 1g/L.
- La protéine ANXA3 humaine native est testée diluée à 100, 25 et 3 ng/mL dans du tampon PBS. L'échantillon est déposé (150 µL) dans le premier puits (puits X0) de la cartouche HBs Ag Ultra.
- La réaction ELISA a été réalisée à l'aide de l'automate VIDAS® et du protocole décrit pour le test HBs Ag Ultra.
- Les résultats ont été obtenus sous forme de valeurs brutes après soustraction du bruit de fond. Le signal est en RFV (« relative fluorescente value »).

Le Tableau 2 ci-dessous résume les résultats obtenus (signal RFV), avec les différentes combinaisons d'anticorps utilisés en capture ou en détection, sur trois dilutions de l'Annexine A3 humaine native purifiée des neutrophiles (100, 25 et 3 ng/mL). La dilution 0 ng/mL correspond au témoin tampon PBS, sans Annexine A3.

**Tableau 2**

| **Anticorps de révélation biotinylés** | | **Anticorps de capture** | | | |
|---|---|---|---|---|---|
| **Clone** | **Dilution ANXA3 ng/ml** | **TGC42** | **TGC43** | **TGC44** | **9C6B4** |
| **TGC42** | 0 | - | 22 | 11 | 17 |
| | 3 | - | 44 | 20 | 78 |
| | 25 | - | 238 | 89 | 500 |
| | 100 | - | 859 | 297 | 1837 |
| **TGC43** | 0 | 106 | - | 39 | 63 |
| | 3 | 111 | - | 36 | 82 |
| | 25 | 144 | - | 56 | 257 |
| | 100 | 232 | - | 86 | 914 |
| **TGC44** | 0 | 21 | 9 | - | 12 |
| | 3 | 32 | 19 | - | 19 |
| | 25 | 98 | 89 | - | 74 |
| | 100 | 303 | 309 | - | 261 |
| **9C6B4** | 0 | 51 | 42 | 33 | - |
| | 3 | 65 | 122 | 39 | - |
| | 25 | 201 | 744 | 117 | - |
| | 100 | 590 | 3525 | 357 | - |
| **5C5B10** | 0 | 20 | 12 | 93 | 9 |
| | 3 | 5244 | 3669 | 5870 | 39 |
| | 25 | 10480 | 9874 | 10429 | 249 |
| | 100 | 11310 | 11095 | 11168 | 1139 |
| **13A12G4H2** | 0 | 26 | 21 | 18 | 19 |
| | 3 | 2298 | 676 | 3733 | 23 |
| | 25 | 9655 | 8468 | 10208 | 21 |
| | 100 | 11380 | 10995 | 11332 | 21 |
| **6D9D10** | 0 | 12 | 23 | 16 | 9 |
| | 3 | 11 | 23 | 14 | 9 |
| | 25 | 12 | 23 | 18 | 19 |
| | 100 | 18 | 41 | 43 | 27 |
| **1F10A6** | 0 | 45 | 9 | 14 | 14 |
| | 3 | 159 | 207 | 194 | 17 |
| | 25 | 4014 | 6728 | 5680 | 13 |
| | 100 | 10489 | 10931 | 10688 | 15 |

Le Tableau 3 ci-dessous résume les résultats obtenus décrits dans le Tableau 2 mais avec une grille de lecture différente, pour faciliter l'analyse et l'interprétation :
Si RFV à 3 ng/mL < 1000 alors « - »
Si RFV à 3 ng/mL > 1000 alors « + »
Si RFV à 25 ng/mL > 3000 alors « ++ »
Si (RFV à 100 ng/mL > 9000) et (3000 < RFV à 25 ng/mL < 9000) alors « +++ »
Si RFV à 100 ng/mL et 25 ng/mL > 9000 alors « ++++ »

**Tableau 3**

| | ***mAb de détection biotinylé** | | | | | | | |
|---|---|---|---|---|---|---|---|---|
| ***mAb de capture** | **TGC42** | **TGC43** | **TGC44** | **9C6B4** | **5C5B10** | **13A12G4H2** | **1F10A6** | **6D9D10** |
| **TGC42** | - | - | - | - | ++++ | ++++ | +++ | - |
| **TGC43** | - | - | - | + | ++++ | +++ | +++ | - |
| **TGC44** | - | - | - | - | ++++ | ++++ | +++ | - |
| **9C6B4** | + | - | - | - | + | - | - | - |

| | | | | | | | | |
|---|---|---|---|---|---|---|---|---|
| mAb = anticorps monoclonal | | | | | | | | |

Comme cela ressort du tableau ci-dessus, il existe 9 combinaisons d'anticorps monoclonaux complémentaires qui permettent de réaliser un dosage ELISA de type sandwich de l'ANXA3, avec une dynamique de signal très satisfaisante (couples « +++ » et « ++++ »). D'autres combinaisons d'anticorps monoclonaux complémentaires sont possibles, à savoir 9C6B4 / TGC42, 9C6B4 / TGC43 et 9C6B4 / 5C5B10, mais ces solutions ne sont pas suffisamment robustes d'un point de vue analytique et n'ont pas la sensibilité analytique suffisante pour être utilisées dans les fluides biologiques complexes comme le sérum ou l'urine par exemple.

Pour ces expériences de sélection réalisées en diluant l'Annexine A3 humaine purifiée en tampon PBS, les anticorps monoclonaux TGC42, TGC43 et TGC44 ont, en capture, des performances équivalentes vis-à-vis de l'Annexine A3 humaine.

En détection, les anticorps monoclonaux 5C5B10 et 13A12G4H2 possèdent également des performances équivalentes vis-à-vis de l'Annexine A3 humaine, alors que l'anticorps monoclonal 1F10A6 donne des signaux satisfaisants mais plus faibles qu'avec les anticorps monoclonaux 5C5B10 et 13A12G4H2.

Les expériences présentées en exemple 7 ont permis de montrer que les anticorps monoclonaux TGC42, TGC43 et TGC44 reconnaissent tous les trois la même région de l'Annexine A3. De la même manière, les anticorps monoclonaux 13A12G4H2 et 1F10A6 reconnaissent aussi le même épitope mais qui est distinct du premier. L'anticorps 5C5B10 reconnaît, quant à lui, un troisième épitope distinct des deux premiers. Ainsi, les différents types de dosages ELISA qui permettent de détecter l'ANXA3 sont divisés en deux groupes. Le groupe 1 correspond aux combinaisons des anticorps de capture TGC42, TGC43 ou TGC44 avec les clones 13A12G4H2 ou 1F10A6 utilisés en détection. Le groupe 2 correspond aux combinaisons des anticorps de capture TGC42, TGC43 ou TGC44 avec le monoclonal 5C5B10 utilisé en détection.

### Détection de l'Annexine A3 humaine dans le sérum de sujets ayant un cancer de la prostate

Le sang total a été prélevé chez 56 sujets en utilisant le système de prélèvement S-Monovette (Sarstedt, Cat. No. 02.1388) et traité selon les instructions du fabricant pour obtenir du sérum. Ces 56 sujets ont tous un cancer de la prostate (CaPro) avéré, dont le diagnostic a été confirmé par analyse histologique sur biopsie ultérieurement. L'ANXA3 contenue dans ces 56 échantillons de sérum a été quantifiée avec un dosage ELISA du groupe 1 (capture TGC44, détection 13A12G4H2) et un dosage ELISA du groupe 2 (capture TGC44, détection 5C5B10). A titre indicatif, les anticorps de détection biotinylés ont été dilués à 0,3 µg/mL pour le monoclonal 5C5B10 et à 2,5 ng/mL pour le monoclonal 13A12G4H2. Pour chaque dosage ELISA, une courbe de calibration a été établie en dosant une gamme de concentration de la protéine Annexine A3 humaine native purifiée (Arodia™) diluée dans un tampon approprié. Les sérums d'origine animale ou humaine éventuellement inclus dans ce tampon ont été déplétés au préalable de l'Annexine A3 endogène qu'ils contenaient pour ne pas fausser le résultat du dosage. Alternativement, des lots de sérum d'origine animale qui ne contenaient que de très faibles quantités d'Annexine A3 endogène ont été utilisés. La courbe de calibration a été tracée en reportant en abscisse la concentration et en ordonnée le signal mesuré par le VIDAS® en RFV (Figure 2). La concentration d'ANXA3 présente dans l'échantillon sérique a été calculée , lorsque c'était possible, en interpolant la concentration correspondante au signal RFV lu par le VIDASs®, à l'aide de modèles mathématiques de régression non linéaire comme un polynôme du troisième ordre ou un modèle 4-PL, bien connus de l'homme du métier. Les résultats sont présentés dans le Tableau 4 ci-dessous.

**Tableau 4**

| | Dosage TGC44/5C5B10 | | Dosage TGC44/13A12G4H2 | |
|---|---|---|---|---|
| Code échantillon | Signal (RFV) | Dose (ng/mL) | Signal (RFV) | Dose (ng/mL) |
| CaPro01 | 153 | <0,5 | 914 | 5,8 |
| CaPro02 | 38 | <0,5 | 380 | 2,7 |
| CaPro03 | 47 | <0,5 | 779 | 5,0 |
| CaPro04 | 38 | <0,5 | 572 | 3,8 |
| CaPro05 | 64 | <0,5 | 2431 | 14,1 |
| CaPro06 | 41 | <0,5 | 452 | 3,1 |
| CaPro07 | 71 | <0,5 | 2095 | 12,2 |
| CaPro08 | 44 | <0,5 | 619 | 4,1 |
| CaPro09 | 66 | <0,5 | 1493 | 9,0 |
| CaPro10 | 39 | <0,5 | 401 | 2,8 |
| CaPro11 | 42 | <0,5 | 736 | 4,8 |
| CaPro12 | 60 | <0,5 | 2000 | 11,7 |
| CaPro13 | 65 | <0,5 | 2419 | 14,0 |
| CaPro14 | 51 | <0,5 | 1121 | 6,9 |
| CaPro15 | 46 | <0,5 | 566 | 3,8 |
| CaPro16 | 58 | <0,5 | 852 | 5,5 |
| CaPro17 | 44 | <0,5 | 401 | 2,8 |
| CaPro18 | 62 | <0,5 | 1039 | 6,5 |
| CaPro19 | 56 | <0,5 | 983 | 6,2 |
| CaPro20 | 70 | <0,5 | 601 | 4,0 |
| CaPro21 | 55 | <0,5 | 954 | 6,0 |
| CaPro22 | 43 | <0,5 | 595 | 4,0 |
| CaPro23 | 43 | <0,5 | 680 | 4,5 |
| CaPro24 | 55 | <0,5 | 1324 | 8,1 |
| CaPro25 | 40 | <0,5 | 564 | 3,8 |
| CaPro26 | 45 | <0,5 | 758 | 4,9 |
| CaPro27 | 78 | <0,5 | 3106 | 17,9 |
| CaPro28 | 60 | <0,5 | 205 | 1,5 |
| CaPro29 | 57 | <0,5 | 1583 | 9,5 |
| CaPro30 | 63 | <0,5 | 1583 | 9,5 |
| CaPro31 | 85 | <0,5 | 583 | 3,9 |
| CaPro32 | 47 | <0,5 | 703 | 4,6 |
| CaPro33 | 50 | <0,5 | 331 | 2,3 |
| CaPro34 | 43 | <0,5 | 361 | 2,5 |
| CaPro35 | 46 | <0,5 | 1316 | 8,0 |
| CaPro36 | 42 | <0,5 | 513 | 3,5 |
| CaPro37 | 52 | <0,5 | 872 | 5,6 |
| CaPro38 | 36 | <0,5 | 100 | 0,6 |
| CaPro39 | 44 | <0,5 | 598 | 4,0 |
| CaPro40 | 33 | <0,5 | 643 | 4,3 |
| CaPro41 | 93 | <0,5 | 2891 | 16,7 |
| CaPro42 | 54 | <0,5 | 141 | 1,0 |
| CaPro43 | 54 | <0,5 | 829 | 5,3 |
| CaPro44 | 41 | <0,5 | 761 | 4,9 |
| CaPro45 | 56 | <0,5 | 742 | 4,8 |
| CaPro46 | 53 | <0,5 | 1148 | 7,1 |
| CaPro47 | 44 | <0,5 | 382 | 2,7 |
| CaPro48 | 48 | <0,5 | 490 | 3,3 |
| CaPro49 | 72 | <0,5 | 1422 | 8,6 |
| CaPro50 | 47 | <0,5 | 535 | 3,6 |
| CaPro51 | 88 | <0,5 | 1414 | 8,5 |
| CaPro52 | 72 | <0,5 | 2866 | 16,5 |
| CaPro53 | 43 | <0,5 | 315 | 2,2 |
| CaPro54 | 83 | <0,5 | 1777 | 10,5 |
| CaPro55 | 53 | <0,5 | 1315 | 8,0 |
| CaPro56 | 48 | <0,5 | 588 | 3,9 |

Il est possible de mesurer des concentrations d'ANXA3 sériques de l'ordre du ng/mL et de la dizaine de ng/mL en utilisant le dosage ELISA TGC44/13A12G4H2 (groupe 1). De façon très surprenante, les signaux obtenus dans les mêmes échantillons sériques avec le dosage TGC44/5C5B10 (groupe 2) sont très faibles et quasiment indistinguables du bruit de fond. D'ailleurs, une courbe de calibration utilisant un modèle 4-PL n'a pas permis de transformer ces signaux en dose, c'est pour cette raison que la dose est reportée comme « <0,5 ng/mL » dans le Tableau 4, qui est la limite de quantification du dosage dans les conditions d'utilisation précisées ici. La courbe de calibration réalisée avec l'ANXA3 humaine native purifiée indique que le dosage TGC44/5C5B10 possède la capacité de détecter la molécule lorsque sa concentration est de l'ordre du ng/mL. Mais malgré une sensibilité analytique (limite de quantification environ 0,5 ng/mL) et une spécificité analytique (voir exemple 3) satisfaisantes, le dosage TGC44/5C5B10 ne permet pas de doser l'ANXA3 présente dans le milieu biologique complexe qu'est le sérum.

Ces résultats sont extrêmement surprenants et mettent en évidence une complexité de l'ANXA3 sérique jusque-là insoupçonnée. La différence entre les deux tests ELISA vient de l'anticorps de détection. En effet, l'anticorps monoclonal 5C5B10 permet de détecter l'ANXA3 purifiée capturée par TGC44, mais pas l'ANXA3 capturée directement à partir du sérum par TGC44. L'anticorps 13A12GH2 (et aussi l'anticorps 1F10A6) permet, quant à lui, de détecter l'ANXA3 capturée, qu'il s'agisse de la protéine purifiée ou la protéine présente dans le sérum.

### Exemple 2 : Détection de l'Annexine A3 humaine par ELISA dans le sérum de patients ayant un cancer colorectal

Le sang total a été prélevé chez 39 sujets en utilisant les tubes BD Vacutainer® (BD, Cat. No. 367614) et traité selon les instructions du fabricant pour obtenir du sérum. Ces 39 sujets ont tous un cancer colorectal (CCR) avéré, dont le diagnostic a été confirmé par examen histologique sur pièces opératoires. L'ANXA3 contenue dans ces 39 échantillons de sérum a été quantifiée avec un dosage ELISA du groupe 1 (capture TGC44, détection 13A12G4H2) et un dosage ELISA du groupe 2 (capture TGC44, détection 5C5B10). A titre indicatif, les anticorps de détection biotinylés ont été dilués à 0,15 µg/mL pour le monoclonal 5C5B10 et à 2 ng/mL pour le monoclonal 13A12G4H2. Pour chaque dosage ELISA, une courbe de calibration a été établie en dosant une gamme de concentration de la protéine Annexine A3 humaine native purifiée (Arodia). La courbe de calibration a été tracée en reportant en abscisse la concentration et en ordonnée le signal mesuré par le VIDAS® en RFV. La concentration d'ANXA3 présente dans l'échantillon sérique a été calculée, lorsque c'était possible, en interpolant la concentration correspondante au signal RFV lu par le VIDAS®, à l'aide de modèles mathématiques de régression non linéaire comme un polynôme du troisième ordre ou un modèle 4-PL, bien connus de l'homme du métier. Les résultats sont présentés dans le Tableau 5 ci-dessous.

**Tableau 5**

| | Dosage TGC44/5C5B10 | | Dosage TGC44/13A12G4H2 | |
|---|---|---|---|---|
| Code échantillon | Signal (RFV) | Dose (ng/mL) | Signal (RFV) | Dose (ng/mL) |
| CCR01 | 59 | <0,5 | 5358 | 20,5 |
| CCR02 | 20 | <0,5 | 889 | 3,9 |
| CCR03 | 79 | <0,5 | 3648 | 13,1 |
| CCR04 | 289 | 0,9 | 2120 | 7,9 |
| CCR05 | 68 | <0,5 | 1526 | 6,0 |
| CCR06 | 49 | <0,5 | 1127 | 4,7 |
| CCR07 | 196 | 0,5 | 809 | 3,6 |
| CCR08 | 24 | <0,5 | 346 | 1,8 |
| CCR09 | 86 | <0,5 | 3692 | 13,3 |
| CCR10 | 58 | <0,5 | 2806 | 10,2 |
| CCR11 | 77 | <0,5 | 750 | 3,4 |
| CCR12 | 46 | <0,5 | 1533 | 6,0 |
| CCR13 | 102 | <0,5 | 210 | 1,1 |
| CCR14 | 42 | <0,5 | 2338 | 8,6 |
| CCR15 | 149 | <0,5 | 5744 | 22,5 |
| CCR16 | 32 | <0,5 | 141 | 0,7 |
| CCR17 | 98 | <0,5 | 1327 | 5,4 |
| CCR18 | 114 | <0,5 | 4228 | 15,4 |
| CCR19 | 69 | <0,5 | 4568 | 16,8 |
| CCR20 | 161 | <0,5 | 3071 | 11,1 |
| CCR21 | 65 | <0,5 | 1469 | 5,8 |
| CCR22 | 40 | <0,5 | 764 | 3,5 |
| CCR23 | 106 | <0,5 | 1983 | 7,5 |
| CCR24 | 53 | <0,5 | 5427 | 20,8 |
| CCR25 | 48 | <0,5 | 2743 | 10,0 |
| CCR26 | 136 | <0,5 | 2445 | 9,0 |
| CCR27 | 238 | 0,7 | 1382 | 5,5 |
| CCR28 | 38 | <0,5 | 1221 | 5,0 |
| CCR29 | 33 | <0,5 | 1604 | 6,3 |
| CCR30 | 85 | <0,5 | 3063 | 11,0 |
| CCR31 | 46 | <0,5 | 2176 | 8,1 |
| CCR32 | 42 | <0,5 | 1004 | 4,3 |
| CCR33 | 92 | <0,5 | 2383 | 8,8 |
| CCR34 | 35 | <0,5 | 1567 | 6,1 |
| CCR35 | 88 | <0,5 | 620 | 2,9 |
| CCR36 | 166 | <0,5 | 6897 | 30,3 |

Dans le sérum de patients ayant un cancer colorectal, il est possible de détecter et de quantifier l'Annexine A3 en utilisant le dosage ELISA TGC44/13A12G4H2 (groupe 1). Le dosage ELISA TGC44/5C5B10 (groupe 2) rend des signaux très faibles, inférieurs ou à la limite de ce qui est quantifiable. Il n'est donc pas possible d'utiliser le format TGC44/5C5B10 pour quantifier l'Annexine A3 présente dans le sérum de patients ayant un cancer colorectal.

### Exemple 3 : Détection de l'Annexine A3 humaine par ELISA dans le sérum de patients ayant un cancer des poumons

Les dosages d'Annexine A3 ont été réalisés sur des prélèvements de sérum effectués chez 33 patients ayant un cancer bronchopulmonaire (CaPou) confirmé selon les modalités précisées dans l'exemple 2. Les résultats sont présentés dans le Tableau 6 ci-dessous.

**Tableau 6**

| | Dosage TGC44/5C5B10 | | Dosage TGC44/13A12G4H2 | |
|---|---|---|---|---|
| Code échantillon | Signal (RFV) | Dose (ng/mL) | Signal (RFV) | Dose (ng/mL) |
| CaPou01 | 235 | 0,7 | 2347 | 8,7 |
| CaPou02 | 619 | 2,0 | 928 | 4,0 |
| CaPou03 | 1219 | 4,1 | 1453 | 5,8 |
| CaPou04 | 58 | <0,5 | 729 | 3,3 |
| CaPou05 | 253 | 0,7 | 5505 | 21,2 |
| CaPou06 | 127 | <0,5 | 514 | 2,5 |
| CaPou07 | 127 | <0,5 | 4433 | 16,2 |
| CaPou08 | 320 | 1,0 | 2953 | 10,7 |
| CaPou09 | 223 | 0,6 | 6373 | 26,4 |
| CaPou10 | 329 | 1,0 | 9056 | 61,0 |
| CaPou11 | 864 | 2,9 | 7703 | 37,9 |
| CaPou12 | 125 | <0,5 | 3115 | 11,2 |
| CaPou13 | 354 | 1,1 | 8069 | 42,4 |
| CaPou14 | 385 | 1,2 | 8268 | 45,3 |
| CaPou15 | 428 | 1,4 | 8060 | 42,3 |
| CaPou16 | 333 | 1,0 | 4396 | 16,1 |
| CaPou17 | 185 | <0,5 | 1770 | 6,8 |
| CaPou18 | 670 | 2,2 | 1278 | 5,2 |
| CaPou19 | 206 | 0,6 | 5219 | 19,8 |
| CaPou20 | 40 | <0,5 | 615 | 2,9 |
| CaPou21 | 330 | 1,0 | 6404 | 26,6 |
| CaPou22 | 110 | <0,5 | 860 | 3,8 |
| CaPou23 | 561 | 1,8 | 932 | 4,0 |
| CaPou24 | 50 | <0,5 | 573 | 2,7 |
| CaPou25 | 166 | <0,5 | 1087 | 4,6 |
| CaPou26 | 49 | <0,5 | 844 | 3,7 |
| CaPou27 | 82 | <0,5 | 534 | 2,6 |
| CaPou28 | 97 | <0,5 | 1288 | 5,2 |
| CaPou29 | 121 | <0,5 | 652 | 3,0 |
| CaPou30 | 131 | <0,5 | 2969 | 10,7 |
| CaPou31 | 99 | <0,5 | 503 | 2,5 |
| CaPou32 | 207 | 0,6 | 1668 | 6,5 |
| CaPou33 | 210 | 0,6 | 3770 | 13,6 |

Dans le sérum de patients ayant un cancer bronchopulmonaire, il est possible de détecter et de quantifier l'Annexine A3 en utilisant le dosage ELISA TGC44/13A12G4H2 (groupe 1). Les doses obtenues sur cette série de patients vont de 2,5 à 61 ng/mL. Le dosage ELISA TGC44/5C5B10 (groupe 2) rend des signaux bien plus faibles, avec 15 échantillons sur 33 pour lesquels la dose est inquantifiable car trop basse. Dans cette série de patients, le format TGC44/5C5B10 rend des doses aux alentours de 1-2 ng/mL, beaucoup trop basses par rapport aux doses obtenues avec le format TGC44/13A12G4H2. Il n'est donc pas possible d'utiliser le format TGC44/5C5B10 pour quantifier l'Annexine A3 présente dans le sérum de patients ayant un cancer des poumons.

### Exemple 4 : Détection de l'Annexine A3 humaine par ELISA dans le sérum de patients ayant une pathologie inflammatoire chronique

Les dosages d'Annexine A3 ont été réalisés sur des prélèvements de sérum effectués chez 10 patients ayant une polyarthrite rhumatoïde (PR, maladie dégénérative inflammatoire chronique des articulations) et chez 21 patients ayant la maladie de Crohn (MC, maladie inflammatoire chronique intestinale) selon les modalités précisées dans l'exemple 2. Les résultats sont présentés dans le Tableau 7 ci-dessous.

**Tableau 7**

| | Dosage TGC44/5C5B10 | | Dosage TGC44/13A12G4H2 | |
|---|---|---|---|---|
| Code échantillon | Signal (RFV) | Dose (ng/mL) | Signal (RFV) | Dose (ng/mL) |
| PR01 | 182 | <0,5 | 3060 | 11,0 |
| PR02 | 596 | 2,0 | 6323 | 26,1 |
| PR03 | 577 | 1,9 | 4358 | 15,9 |
| PR04 | 497 | 1,6 | 2482 | 9,1 |
| PR05 | 540 | 1,8 | 4862 | 18,1 |
| PR06 | 336 | 1,1 | 3226 | 11,6 |
| PR07 | 749 | 2,5 | 6067 | 24,4 |
| PR08 | 248 | 0,7 | 5669 | 22,1 |
| PR09 | 688 | 2,3 | 8630 | 51,4 |
| PR10 | 285 | 0,9 | 6369 | 26,4 |
| MC01 | 77 | <0,5 | 2279 | 8,4 |
| MC02 | 101 | <0,5 | 4034 | 14,6 |
| MC03 | 47 | <0,5 | 7029 | 31,4 |
| MC04 | 165 | <0,5 | 4575 | 16,8 |
| MC05 | 85 | <0,5 | 1947 | 7,4 |
| MC06 | 64 | <0,5 | 3924 | 14,2 |
| MC07 | 96 | <0,5 | 4267 | 15,5 |
| MC08 | 97 | <0,5 | 1797 | 6,9 |
| MC09 | 197 | 0,5 | 5052 | 19,0 |
| MC10 | 32 | <0,5 | 1004 | 4,3 |
| MC11 | 246 | 0,7 | 3095 | 11,2 |
| MC12 | 99 | <0,5 | 2972 | 10,7 |
| MC13 | 52 | <0,5 | 3938 | 14,2 |
| MC14 | 158 | <0,5 | 7637 | 37,2 |
| MC15 | 297 | 0,9 | 1640 | 6,4 |
| MC16 | 104 | <0,5 | 7952 | 40,9 |
| MC17 | 254 | 0,7 | 2510 | 9,2 |
| MC18 | 58 | <0,5 | 2570 | 9,4 |
| MC19 | 46 | <0,5 | 3027 | 10,9 |
| MC20 | 41 | <0,5 | 1947 | 7,4 |
| MC21 | 129 | <0,5 | 3320 | 11,9 |

Dans le sérum de patients ayant une polyarthrite rhumatoïde ou la maladie de Crohn, il est possible de détecter et de quantifier l'Annexine A3 en utilisant le dosage ELISA TGC44/13A12G4H2 (groupe 1). Les doses obtenues sur cette série de patients vont de 4,3 à 51,4 ng/mL. Le dosage ELISA TGC44/5C5B10 (groupe 2) rend des signaux bien plus faibles, avec 18 échatillons sur 31 pour lesquels la dose est inquantifiable car trop basse. Dans cette série de patients, les doses rendues par le format TGC44/5C5B10 ne dépassent pas 2,5 ng/mL, beaucoup trop basses par rapport aux doses obtenues avec le format TGC44/13A12G4H2. Il n'est donc pas possible d'utiliser le format TGC44/5C5B10 pour quantifier l'Annexine A3 présente dans le sérum de patients ayant une maladie inflammatoire chronique.

### Exemple 5 : Détection de l'Annexine A3 humaine par ELISA dans le sérum de patients à risque de sepsis

Les dosages d'Annexine A3 ont été réalisés sur des prélèvements de sérum effectués chez 24 patients présentant un risque de sepsis selon les modalités précisées dans l'exemple 2. La procalcitonine (PCT) est un marqueur spécifique et précoce d'infection bactérienne. Lors d'infections localisées, le taux de PCT peut atteindre 0,1 ng/mL et il dépasse les 1-2 ng/mL lors d'infections généralisées. Les sérums inclus dans la série présentée dans le Tableau 8 ont des taux de PCT compris entre 0,3 et 10,3 ng/mL (taux déterminé avec le kit PCT KRYPTOR™ de BRAHMS™).

**Tableau 8**

| | Dosage TGC44/5C5B10 | | Dosage TGC44/13A12G4H2 | |
|---|---|---|---|---|
| Code échantillon | Signal (RFV) | Dose (ng/mL) | Signal (RFV) | Dose (ng/mL) |
| PCT01 | 176 | <0,5 | 370 | 1,9 |
| PCT02 | 352 | 1,1 | 3786 | 13,6 |
| PCT03 | 225 | 0,6 | 902 | 3,9 |
| PCT04 | 2630 | 9,1 | 7797 | 39,0 |
| PCT05 | 719 | 2,4 | 5204 | 19,7 |
| PCT06 | 1244 | 4,2 | 5574 | 21,6 |
| PCT07 | 534 | 1,8 | 5773 | 22,7 |
| PCT08 | 137 | <0,5 | 1681 | 6,5 |
| PCT09 | 1365 | 4,6 | 9101 | 62,2 |
| PCT10 | 815 | 2,7 | 8800 | 54,9 |
| PCT11 | 563 | 1,9 | 6926 | 30,5 |
| PCT12 | 331 | 1,0 | 6579 | 27,8 |
| PCT13 | 405 | 1,3 | 2054 | 7,7 |
| PCT14 | 458 | 1,5 | 3276 | 11,8 |
| PCT15 | 2003 | 6,8 | 5609 | 21,8 |
| PCT16 | 303 | 0,9 | 3976 | 14,4 |
| PCT17 | 218 | 0,6 | 3298 | 11,9 |
| PCT18 | 738 | 2,5 | 7627 | 37,1 |
| PCT19 | 465 | 1,5 | 7374 | 34,5 |
| PCT20 | 527 | 1,7 | 798 | 3,6 |
| PCT21 | 236 | 0,7 | 1568 | 6,1 |
| PCT22 | 200 | 0,5 | 1876 | 7,1 |
| PCT23 | 601 | 2,0 | 5755 | 22,6 |
| PCT24 | 3405 | 12,3 | 9227 | 65,8 |

Dans le sérum de patients ayant une infection bactérienne, il est possible de détecter et de quantifier l'Annexine A3 en utilisant le dosage ELISA TGC44/13A12G4H2 (groupe 1). Les doses obtenues sur cette série de patients vont de 1,9 à 65,8 ng/mL. Le dosage ELISA TGC44/5C5B10 (groupe 2) rend des signaux bien plus faibles et les doses restent basses par rapport aux doses obtenues avec le format TGC44/13A12G4H2. Il ne semble pas utile d'utiliser le format TGC44/5C5B10 pour quantifier l'Annexine A3 présente dans le sérum de patients étant à risque de sepsis.

### Exemple 6 : Détection de l'Annexine A3 humaine par ELISA dans le sérum de patients ayant du diabète

Les dosages d'Annexine A3 ont été réalisés sur des prélèvements de sérum effectués chez 6 patients ayant du diabète, selon les modalités précisées dans l'exemple 2. Les résultats sont présentés dans le Tableau 9 ci-dessous.

**Tableau 9**

| | Dosage TGC44/5C5B10 | | Dosage TGC44/13A12G4H2 | |
|---|---|---|---|---|
| Code échantillon | Signal (RFV) | Dose (ng/mL) | Signal (RFV) | Dose (ng/mL) |
| Dia01 | 51 | <0,5 | 444 | 2,2 |
| Dia02 | 231 | 0,7 | 1664 | 6,5 |
| Dia03 | 44 | <0,5 | 585 | 2,8 |
| Dia04 | 171 | <0,5 | 3263 | 11,7 |
| Dia05 | 213 | 0,6 | 3495 | 12,6 |
| Dia06 | 289 | 0,9 | 2564 | 9,4 |

Dans le sérum de patients ayant du diabète, il est possible de détecter et de quantifier l'Annexine A3 en utilisant le dosage ELISA TGC44/13A12G4H2 (groupe 1). Le dosage ELISA TGC44/5C5B10 (groupe 2) rend des signaux très faibles, inférieurs ou à la limite de ce qui est quantifiable. Il n'est donc pas possible d'utiliser le format TGC44/5C5B10 pour quantifier l'Annexine A3 présente dans le sérum de patients ayant du diabète.

### Exemple 7 : Détermination des épitopes reconnus par les anticorps monoclonaux anti-ANXA3

### Expression des 4 domaines répétés (« annexin repeat » en langue anglaise) de l'Annexine A3 humaine sous forme recombinante et détermination des domaines répétés reconnus par les anticorps monoclonaux.

Comme tous les membres de la famille des Annexines, l'Annexine A3 contient dans sa séquence protéique des domaines répétés appelés « annexin repeat ». Ces domaines répétés sont au nombre de 4 et caractérisent la famille. Afin de déterminer le domaine répété reconnu par chacun des anticorps monoclonaux, ces domaines ont été exprimés sous une forme recombinante. Une séquence de 8 histidines a été rajoutée à la partie N-terminale de chaque domaine afin de permettre la purification par chromatographie d'affinité métal-chélate. Le Tableau 10 récapitule les séquences protéiques des constructions de recombinants permettant d'exprimer chacun des domaines répétés de façon isolée.

**Tableau 10**

| **Domaine** | **Réel ^{a}** | **Exprimé ^{b}** | **Séquence protéique** |
|---|---|---|---|
| D1 | 27-87 | 19-89 | |
| D2 | 99-159 | 92-160 | |
| D3 | 183-243 | 171-245 | |
| D4 | 258-318 | 252-323 | |

| | | | |
|---|---|---|---|
| ^{a} Réel: Etendue du domaine répété, du premier au dernier acide aminé, selon la base de données UniProtKB (http://www.uniprot.org). ^{b} Exprimé : Etendue de la construction contenant le domaine répété, numérotation des acides aminés selon UniProtKB. Les constructions contiennent quelques acides aminés supplémentaires des côtés N et C terminales du domaine, afin de ne pas interrompre les hélices alpha et leur permettre de se former. | | | |

Les séquences d'acides nucléiques correspondant aux séquences protéiques des domaines D1, D2, D3, D4 ont été obtenues par synthèse chimique par la société Geneart. Ces séquences nucléiques ont été optimisées pour favoriser l'expression des protéines en *Escherichia coli.* Les fragments d'ADN ont été clonés entre les sites Nco I et Xba I du vecteur d'expression procaryote pMRCH79 (dérivé du pMR78, bioMérieux). Les plasmides ainsi obtenus ont été transformés dans les bactéries BL21 (DE3) (Stratagene). Les cultures pour produire les différents domaines sont réalisées à 37°C sous agitation dans du milieu 2-YT (Invitrogen). L'induction est effectuée avec 0,5 mM d'IPTG (isopropyl beta-1-thiogalactosidase). Les culots bactériens sont directement repris dans le tampon d'échantillons des gels NuPAGE Novex (Invitrogen) en suivant le mode opératoire fourni avec les gels, en conditions réduites. La séparation des protéines est réalisée en gel NuPAGE Novex Bis-Tris 4-12%. L'analyse en Western blot de la réactivité des anticorps monoclonaux pour les différents domaines de l'Annexine A3 est réalisée en utilisant un substrat chemiluminescent, selon le procédé décrit dans la demande de brevet WO 2009/019365 par exemple, bien connu de l'homme du métier. Les anticorps à tester ont été utilisés à une dilution de 10 µg/mL. Le temps d'exposition a été de 100 secondes, sauf indication contraire.

Chaque anticorps anti-ANXA3 a été testé avec les recombinants exprimant les 4 domaines répétés de l'ANXA3 humaine ; les résultats de cette analyse Western blot sont présentés dans la Figure 3. Les anticorps monoclonaux TGC42, TGC43, TGC44 et 5C5B10 sont spécifiques du domaine D1. Les anticorps monoclonaux 13A12G4H2 et 1F10A6 sont dirigés contre le domaine D4. Quant aux anticorps 9C6D4 et 6D9D10, ils ne reconnaissent aucun des 4 domaines répétés, ce qui indique très probablement que leurs épitopes sont situés en dehors des domaines répétés de l'ANXA3.

### Analyse fine des épitopes reconnus par les anticorps anti-ANXA3

La détermination des épitopes a été effectuée avec la technique du Spotscan d'après Frank et Döring [24], qui est décrite en détail dans la demande de brevet WO 2009/019365. Dans ce but, la totalité de la séquence protéique de l'Annexine A3 a été reproduite sur une membrane de nitrocellulose sous forme de peptides de 12 acides aminés chevauchants, décalés de 2 acides aminés. Puis dans une seconde synthèse, la séquence d'ANXA3 a été reproduite sous forme de peptides de 15 acides aminés chevauchants, décalés d'un acide aminé. L'immunoréactivité de ces membranes de peptides chevauchants a été testée avec les anticorps anti-ANXA3.

Ainsi, il a été possible de délimiter de façon plus fine les épitopes de 5 anticorps monoclonaux anti-ANXA3 parmi les 8 étudiés. Les épitopes déduits de la comparaison des séquences des peptides chevauchants reconnus sont récapitulés dans le Tableau 11. L'épitope minimal est la séquence minimale nécessaire pour avoir une reconnaissance de l'anticorps, avec un signal plus ou moins intense. L'épitope optimal est la séquence idéale permettant la meilleure reconnaissance possible de l'anticorps, incluant ou identique à l'épitope minimal. Les résultats obtenus confirment que TGC42 et TGC43 sont bien dirigés contre un épitope unique, celui qui est décrit initialement dans la demande WO 2010/034825. De façon surprenante, l'anticorps 5C5B10 définit un nouvel épitope qui n'était pas décrit dans l'art antérieur. Les anticorps 6D9D10 et 9C6D4 sont spécifiques de l'extrémité N-terminale de la protéine, comme le monoclonal TGC7 de la demande WO 2007/141043. Les anticorps monoclonaux TGC44, 13A12G4H2 et 1F10A6 ne présentent aucune réactivité en Spotscan, même sur une membrane portant des peptides de 20 acides aminés de long. Ils possèdent probablement des épitopes conformationnels ou au moins semi-conformationnels dont les structures ne sont pas assez bien reproduites par des peptides de synthèse.

**Tableau 11**

| **Anticorps** | **Domaine** | **Epitope optimal ^{a}** | **Epitope minimal ^{b}** |
|---|---|---|---|
| TGC42 | D1 | SNAQRQLIVKEYQAAYG | LIVKEYQAAYG |
| | | (49-65) (SEQ ID NO: 10) | (55-65) (SEQ ID NO: 11) |
| TGC43 | D1 | IVKEYQAAYGKE | KEYQAAYG |
| | | (56-67) (SEQ ID NO: 12) | (58-65) (SEQ ID NO: 13) |
| 5C5B10 | D1 | DLSGHFEHL | LSGHFEH |
| | | (75-83) (SEQ ID NO: 14) | (76-82) (SEQ ID NO: 15) |
| 6D9D10 | N-term | ASIWVGHRGTVRDYPDFSPS | SIWVGHRGTVRDYPDFSP |
| | | (2-21) (SEQ ID NO: 16) | (3-20) (SEQ ID NO: 17) |
| 9C6D4 | N-term | YPDF | YPDF |
| | | (15-18) (SEQ ID NO: 18) | (15-18) SEQ ID NO: 18) |

| | | | |
|---|---|---|---|
| ^{a} Epitope optimal : Séquence idéale permettant la meilleure reconnaissance possible de l'anticorps (incluant ou identique à l'épitope minimal). ^{b} Epitope minimal : Séquence minimale nécessaire pour avoir une reconnaissance de l'anticorps (signal plus ou moins intense). | | | |

### Localisation précise de l'épitope de l'anticorps TGC44 par la technique Novatope

Le système Novatope (Merck, Cat No. 69279) est une technologie permettant l'analyse d'une protéine dans le but de sélectionner des domaines contenant des épitopes. La méthode est fondée sur la création d'une banque de clones bactériens, chacun exprimant une fragment de la protéine, coupée aléatoirement. Ces clones sont analysés par immunorévélation avec l'anticorps que l'on cherche à caractériser. Le séquençage de l'ADN des clones positifs permet de déduire la séquence protéique d'un fragment contenant l'épitope. La technique a été appliquée en suivant le mode opératoire fourni avec le kit.

Ainsi, deux clones réagissant avec l'anticorps TGC44 ont pu être isolés et séquencés. Le clone 2J7 exprime la séquence KEYQAAYGKELKDDLKGDLSGHFEHLMVALVTPPAVFD (SEQ ID NO: 19) qui correspond aux résidus 58-95 de l'ANXA3. Le clone 2Z13 exprime la séquence QKAIRGIGTDEKMLISILTERSNAQRQLIVKEYQAAYGKELKDDLKGDLSGHFEHL (SEQ ID NO: 20) qui correspond aux résidus 28-83 de l'ANXA3. La partie commune entre les séquences de ces deux clones sont les résidus 58-83 de l'Annexine A3, soit la séquence KEYQAAYGKELKDDLKGDLSGHFEHL (SEQ ID NO: 21). De plus, les anticorps TGC44 et 5C5B10 étant complémentaires (voir exemple 1), leurs deux épitopes ne peuvent être chevauchants, on peut donc retirer les acides aminés correspondants à l'épitope de l'anticorps 5C5B10, soit DLSGHFEHL (75-83) (SEQ ID : 14). Donc, l'épitope de l'anticorps TGC44 est compris dans la séquence KEYQAAYGKELKDDLKG (58-74) (SEQ ID NO: 22). Il s'agit de la même région que celle reconnue par TGC42 et TGC43. Par contre, le fait que l'anticorps TGC44 ne présente pas de réactivité en Spotscan indique une contrainte conformationnelle pour la reconnaissance. TGC44 est capable de se fixer sur son épitope uniquement lorsque la séquence KEYQAAYGKELKDDLKG (58-74) (SEQ ID NO: 22) est fusionnée du côté N-terminal à une séquence longue d'au moins 30 résidus. Ainsi, le domaine répété recombinant D1, dont la séquence est identifiée en SEQ ID NO: 6, les clones 2J7 et 2Z13 fusionnés à une protéine porteuse ou encore le fragment recombinant vANA-7 décrit demande WO 2010/034825 sont tous reconnus par le clone TGC44. A l'inverse, le fragment recombinant vANA-3, à qui il manque les 34 premiers acides aminés N-terminaux, n'est pas reconnu (Figure 4).

### Localisation précise de l'épitope des anticorps 13A12G4H2 et 1F10A6

L'expérience présentée dans la Figure 3 montre que les épitopes des anticorps 13A12G4H2 et 1F10A6 sont contenus dans le domaine D4 de l'Annexine A3. Nous avons construit par mutagenèse dirigée 12 protéines recombinantes afin d'améliorer le mapping des anticorps dirigés contre le domaine D4. Il s'agit de la séquence complète de l'ANXA3 native mature (aa 2-323), fusionnée du côté N-terminal à un tag histidine (séquence non mutée). La mutagenèse par PCR a été utilisée afin d'introduire des mutations alanine en positions 253, 257, 260, 263, 265, 268, 270, 274, 306, 311 et 317 de la séquence protéique (GeneArt Mutagenesis Service, Invitrogen). Tous ces fragments d'ADN ont été clonés dans le vecteur pMRCH79 (qui dérive du vecteur pMR78), puis transformés dans les bactéries BL21 (DE3). La production et la purification des protéines ont été réalisées selon les techniques bien connues de l'homme du métier et qui ont été citées au début de cet exemple.

Ces 12 protéines (11 mutants et 1 témoin non muté) ont ensuite été utilisées pour évaluer la capacité de fixation des anticorps 13A12G4H2 et 1F10A6 dans un ELISA au format sandwich, utilisant l'anticorps TGC44 en capture. L'anticorps de détection 5C5B10 dont l'épitope est dans le domaine D1 et de ce fait, ne devrait pas être affecté par les mutations du domaine D4 est utilisé comme témoin. Les résultats sont présentés en Figure 5. Le graphe représente le « signal fold change » (en ordonnée) de chaque mutation (en abscisse). Le « signal fold change » correspond au log₂(signal protéine mutée / signal protéine non mutée). Plus la valeur absolue du signal fold change est élevée, plus les mutations pour lesquelles cette variation est observée affectent la fixation de l'anticorps. Ainsi, pour 5C5B10, le « signal fold change » est toujours autour de 0, indiquant qu'aucune des mutations testées ne perturbe la fixation de ce monoclonal. Par contre, pour 13A12G4H2 et 1F10A6, il a été possible d'identifier des acides aminés dont la mutation empêche ou perturbe de façon très significative la fixation. Il s'agit des positions 260, 263, 265 et 306. La mutation de la position 270 a un impact mesurable mais moins important que pour les positions précédentes. Nous pouvons en conclure que les acides aminés 260, 263, 265 et 306 de l'ANXA3 appartiennent à l'épitope reconnu par les anticorps monoclonaux 13A12G4H2 et 1F10A6, les deux résidus les plus importants dans l'interaction antigène-anticorps étant la lysine en position 263 et l'acide aspartique en position 306.

### Détermination des acides aminés essentiels sur le premier domaine répété de l'Annexine A3 humaine pour la reconnaissance par les anticorps 5C5B10 et TGC43

Aux fins de la détermination des acides aminés essentiels des épitopes inclus dans D1 pour la reconnaissance par les anticorps 5C5B10 et TGC43, la technique dite d'« ALAscan » a été présentement utilisée. Cette technique d'« ALAscan » consiste, lors d'une synthèse peptidique, à remplacer successivement les acides aminés contenus dans un épitope par un résidu alanine. Cette technique permet ainsi de déterminer les acides aminés essentiels pour la reconnaissance de l'épitope par un anticorps donné. Lorsque l'acide aminé est strictement nécessaire pour l'interaction avec l'anticorps, son remplacement par une alanine provoque une disparition de l'immunoréactivité du peptide. Lorsque l'acide aminé est moins important en vue de ladite interaction avec l'anticorps, l'immunoréactivité baisse légèrement ou demeure inchangée. Cette technique a été décrite par Geysen et al. en 1987 [26] et peut être élargie en remplaçant chaque acide aminé par les 19 autres.

Ainsi, afin de déterminer les acides aminés essentiels pour la reconnaissance de l'anticorps 5C5B10 susvisé, la séquence optimale consensus de l'épitope, telle que représentée dans le Tableau 11 supra (SEQ ID NO: 14), « rallongée » d'un ou deux acides aminés de part et d'autre de cette séquence, a été synthétisée sur membrane en remplaçant successivement chaque acide aminé par une alanine. Le peptide de référence utilisé est KGDLSGHFEHLM (résidus 73 à 84 de l'Annexine A3 humaine). Le résultat obtenu suite à l'immunorévélation de cette membrane par l'anticorps 5C5B10 est présenté en Figure 6a.

Cette analyse montre que le résidu primordial pour la reconnaissance de l'épitope DLSGHFEHL par l'anticorps 5C5B10 est la phénylalanine (F) en position 54 de SEQ ID NO: 1. Il y a en effet une perte totale de la reconnaissance lorsqu'il est remplacé par une alanine. Les autres résidus qui contribuent de manière importante à la reconnaissance sont l'acide aspartique (D), la sérine (S), l'histidine (H) et la leucine (L), respectivement en positions 49, 51, 56 et 57 de SEQ ID NO: 1. Il est important de rappeler que d'autres mutations, à savoir le remplacement d'un acide aminé par un acide aminé analogue, n'auront que peu ou pas d'impact sur la réactivité antigénique. Ainsi, le remplacement de l'acide glutamique par l'acide aspartique ne devrait en principe pas influencer la fixation de l'anticorps 5C5B10 sur son épitope inclus dans le premier domaine répété (D1) de l'Annexine A3.

Tel qu'indiqué supra, l'expression « acide aminé analogue » se réfère à un acide aminé qui, lorsqu'il remplace l'acide aminé présent dans l'épitope d'intérêt, n'entraîne aucune destruction de la réactivité antigénique dudit épitope vis à vis de l'anticorps d'intérêt.

Les analogues particulièrement préférés incluent les substitutions conservatrices en nature, c'est-à-dire les substitutions qui prennent place dans une famille d'acides aminés. Il existe plusieurs classifications d'acides aminés en famille, comme bien connu de l'homme du métier. Ainsi, selon un exemple de classification, les acides aminés peuvent être divisés en quatre familles, à savoir (1) les acides aminés acides tels que l'acide aspartique ou l'acide glutamique, (2) les acides aminés basiques tels que la lysine, l'arginine et l'histidine, (3) les acides aminés non polaires tels que la leucine, l'isoleucine et la méthionine et (4) les acides aminés non chargés polaires tels que la glycine, l'asparagine, la glutamine, la sérine, la thréonine et la tyrosine. La phénylalanine, le tryptophane et la tyrosine sont parfois classés en acides aminés aromatiques. Par exemple, l'on peut prédire, de façon raisonnable, qu'un remplacement isolé d'un résidu leucine par un résidu isoleucine ou valine, d'un résidu aspartate par un résidu glutamate, d'un résidu thréonine par un résidu sérine, ou un remplacement conservateur similaire d'un acide aminé par un autre acide aminé ayant un rapport structurel, n'aura pas d'effet majeur sur l'activité biologique. Un autre exemple de méthode permettant de prédire l'effet sur l'activité biologique d'une substitution d'acide aminé a été décrite par Ng et Henikoff, 2001 [27].

De la même manière, les acides aminés essentiels pour la reconnaissance de l'anticorps TGC43 ont été déterminés en utilisant le peptide de référence VKEYQAAYGKEL (résidus 57-68 de l'Annexine A3). Les deux alanines contenues dans cette séquence ont été remplacées par des glycines lors de la synthèse ALAScan. Le résultat obtenu suite à l'immunorévélation de la membrane d'ALAScan par l'anticorps TGC43 est présenté en Figure 6b.

Cette analyse montre que 9 résidus sur 10 sont indispensables pour la reconnaissance de l'épitope KEYQAAYGKE. En effet, il y a une perte totale ou très importante de la reconnaissance lorsque l'on remplace chaque résidu de la séquence par une alanine (ou une glycine le cas échéant) chaque résidu de la séquence, à l'exception de la glutamine (Q) en position 35 de SEQ ID NO : 1. Comme mentionné précédemment; il est important de rappeler que d'autres mutations, à savoir le remplacement d'un acide aminé par un acide aminé analogue n'auront que peu ou pas d'impact sur la réactivité antigénique. Ainsi le remplacement des résidus lysine par des résidus arginine, ou encore de l'acide glutamique par l'acide aspartique ne devrait pas influencer la fixation de l'anticorps TGC43.

### Exemple 8 : Spécificité analytique des formats de dosage ELISA anti-ANXA3

Les Annexines sont une famille de protéines qui partagent des homologies de fonction et de séquence. Une interrogation BLAST effectuée sur la base de données UniProtKB, réduite aux séquences d'origine humaine, a permis d'identifier les protéines ayant la plus grande homologie de séquence avec l'Annexine A3. Il s'agit, dans l'ordre décroissant d'homologie, de l'Annexine A4, l'Annexine A11, l'Annexine A6 et l'Annexine A5.

Par conséquent, il était important de démontrer la spécificité des dosages ELISA vis-à-vis de l'Annexine A3 et l'absence de réaction croisée avec les autres membres de la famille. Comme les 3 anticorps TGC42, TGC43 et TGC44 sont dirigés contre le même épitope, le dosage TGC44/13A12G4H2 (appelé uniquement 13A12G4H2 par la suite) a été choisi comme dosage prototype du groupe 1 défini dans l'exemple 1. De la même façon, le dosage TGC44/5C5B10 (appelé uniquement 5C5B10 par la suite) a été choisi comme dosage prototype représentant le groupe 2. L'absence de réactivité croisée a été testée en utilisant des antigènes disponibles commercialement obtenus chez Abnova™ : Annexine A1 (Cat. No. H00000301-P01), Annexine A2 (Cat. No. H00000302-P01), Annexine A4 (Cat. No. H00000307-P01), Annexine A5 (Cat. No. H00000308-P01), Annexine A6 (Cat. No. H00000309-P01) et Annexine A11 (Cat. No. H00000311-P01). L'Annexine A13 a été exprimée sous forme recombinante au laboratoire par clonage dans le vecteur d'expression pMRCH79 ; fusionnée à un tag histidine, puis purifiée par chromatographie d'affinité métal-chélate. Les protéines ont été diluées dans le tampon du puits X1 du VIDAS® à une concentration de 12,5 µg/mL pour l'ELISA TGC44/5C5B10 et à une concentration de 16 µg/mL pour l'ELISA TGC44/13A12G4H2. Les résultats sont présentés en Figure 7. Les deux formats d'ELISA TGC44/13A12G4H2 et TGC44/5C5B10 sont tous les deux très spécifiques de l'Annexine A3 et ne présentent pas de réactivité croisée avec les autres Annexines testées.

### Exemple 9 : Détermination de l'affinité des anticorps anti-Annexine A3

La technologie de résonance plasmonique de surface permet de visualiser en temps réel les interactions entre diverses biomolécules non marquées. Un des réactifs est fixé de manière spécifique sur un biocapteur (sensor chip) tandis que l'autre espèce impliquée dans l'interaction est dans un flux continu de tampon. Les mesures de résonance plasmonique de surface ont été effectuées en utilisant un Biacore T100. Les réactifs incluant le sensor chip CM5, l'anti-IgG de souris, spécifique du fragment Fc, obtenu chez le lapin (RAM Fc), et le kit de couplage via les amines pour l'immobilisation des anticorps ont tous été obtenus chez GE-Healthcare Bioscience AB.

Afin d'étudier les caractéristiques cinétiques de fixation des anticorps anti-Annexine A3, ces derniers ont été immobilisés par capture sur le sensor chip, sur lequel, l'anticorps RAM Fc avait été couplé de façon covalente, au préalable. Les expériences de fixation ont été réalisées en tampon, HEPES, à 25°C, avec un débit de 30 µL/min. La modification du signal de résonnance en RU (Resonance Unit) permet de suivre en temps réel la fixation puis la dissociation des biomolécules à la surface du biocapteur. Dans un premier temps, l'anticorps monoclonal à étudier a été injecté dans le canal 2 pour obtenir un signal d'environ 250 RU. Ensuite, l'Annexine A3 (Arodia) a été injecté dans les canaux 1 et 2. Les durées d'association et de dissociation étaient respectivement de 5 et 15 minutes. Après mesure des réponses de résonance, la surface du biocapteur a été régénérée par un lavage avec du HCl 50 mM, à 10 µL /min pendant 120 secondes. Le même procédé de mesure a été répété pour chaque dilution de la protéine Annexine A3 ; au total 9 dilutions différentes de la protéine comprises entre 0 et 64 nM ont été analysées. Les sensorgrammes obtenus ont été tracés et analysés avec le logiciel dédié du Biacore T100, selon le modèle d'interaction 1:1. Les constantes cinétiques d'association (Kon) et de dissociation (Koff) ont été mesurées en utilisant les anticorps à une concentration de 3 µg/mL, sauf pour 13A12G4H2 et 1F10A6 qui ont été utilisés à 0,75 µg/mL afin de limiter l'impact du bruit de fond. L'affinité, représentée par la constante de dissociation (Kd), a été calculée (Kd=Koff/Kon).

Pour chaque anticorps anti-Annexine A3, les graphiques représentant le signal de résonance en fonction du temps sont présentés en Figure 8. Les valeurs mesurées des constantes d'association et de dissociation, ainsi que la valeur calculée des constantes d'affinité sont indiquées dans le Tableau 12.

L'ensemble des anticorps anti-ANXA3 étudiés présente des affinités élevées, avec des Kd allant de 10⁻⁹ à 5 x 10⁻¹⁰ M. Il est possible de classer les anticorps en deux groupes distincts en fonction de leur Kd. Le premier groupe correspond aux anticorps 13A12G4H2, TGC42 et TGC44 dont la Kd est supérieure à 10⁻¹⁰ M, c'est le groupe des anticorps de très haute affinité. Le second groupe correspond aux anticorps 5C5B10, 1F10A6 et TGC43 dont la Kd est comprise entre 10⁻⁹ et 3,5 x 10⁻⁹ M, c'est le groupe des anticorps de haute affinité.

Les trois anticorps utilisés en capture, TGC42, TGC43 et TGC44, ont des constantes cinétiques d'association équivalentes. L'anticorps 13A12G4H2 a également une constante cinétique d'association comparable. La constante cinétique d'association de l'anticorps 5C5B10 est d'environ 1 log plus bas que celle de TGC44.

En ce qui concerne les constantes cinétiques de dissociation, il est aussi possible de diviser les anticorps anti-ANXA3 en 2 groupes. Le premier groupe contient TGC44, TGC42, 13A12G4H2 et 5C5B10 ; il est caractérisé par une constante cinétique de dissociation très faible, comprise entre 9 x 10⁻⁵ et 3.5 x 10⁻⁴ s⁻¹. Une fois que la fixation antigène-anticorps a eu lieu, l'Annexine A3 est retenue par les anticorps de ce groupe et ne se dissocie pas. Le second groupe contient TGC43 et 1F10A6 ; il est caractérisé par une constante cinétique de dissociation plus élevée, de l'ordre du 10⁻³ s⁻¹. Les anticorps de ce groupe, même s'ils arrivent à fixer l'Annexine A3, s'en dissocient bien plus rapidement. Ainsi, bien qu'ayant des constantes cinétiques d'association comparables, TGC42 et TGC44 sont de meilleurs anticorps de capture à utiliser pour la mise au point d'un dosage ELISA que TGC43.

**Tableau 12**

| Anticorps fixé sur le biocapteur | Kon (M⁻¹.s⁻¹) | Koff (s⁻¹) | Kd (M) |
|---|---|---|---|
| 13A12G4H2 | 5,3E+05 | 2,7E-04 | 5,0E-10 |
| TGC42 | 7,3E+05 | 1,0E-04 | 1,8E-10 |
| TGC44 | 9,5E+05 | 9,7E-05 | 1,0E-10 |
| 5C5B10 | 9,8E+04 | 3,4E-04 | 3,4E-09 |
| 1F10A6 | 3,8E+05 | 1,1E-03 | 2,9E-09 |
| TGC43 | 8,4E+05 | 1,1E-03 | 1,3E-09 |

### Exemple 10 : Présence d'ANXA3 dans les exosomes sériques chez l'homme

Les exosomes sont des vésicules membranaires de 40-100 nm de diamètre sécrétés par différents types cellulaires *in vivo.* Ils sont retrouvés dans le sang, l'urine, les liquides d'ascites malins et contiennent des marqueurs nucléiques et protéiques de cellules tumorales, à partir desquelles ils sont sécrétés.

Afin de détecter la présence d'ANXA3 exosomale sérique, un enrichissement d'exosomes à partir de sérum a été réalisé pour 2 patients A et B atteints de tumeurs prostatique, en utilisant la solution de précipitation d'exosome ExoQuick (System Biosciences, Cat. No. EXOQ20A-1), selon les instructions du fabricant. Pour chaque échantillon sérique A et B, les protéines isolées par enrichissement exosomal ont été analysées par migration sur gel SDS-PAGE 10%. Après resuspension du culot exosomal en tampon RIPA (G-Biosciences, Cat. No. 786-489), une prise d'essai correspondant à 1/10^{e} de la solution est chauffée 4 min à 95°C en présence de tampon de charge Laemmli 5X puis déposée sur gel en conditions réductrices. Le marqueur de poids moléculaire Precision Plus Protein (Bio-Rad) est déposé simultanément sur gel comme contrôle de masse moléculaire. Afin d'analyser la présence spécifique d'ANXA3 exosomale, un western blot est réalisé selon un protocole standard. Le gel SDS-PAGE est transféré sur membrane PVDF (iblot Gel Transfer Stacks PVDF, Cat. No. IB4010-02), la membrane est colorée 1 min en solution amino-black puis rincée à l'eau distillée.

Pour l'étude, un anticorps monoclonal anti-CD63 (Santa Cruz, Cat. No. SC-5275) dirigé contre le marqueur protéique exosomal commun Tétraspanine CD63, et un pool de 3 anticorps monoclonaux anti-ANXA3 (TGC44, 13A12G4H2, 5C5B10) ont été utilisés en détection. La réaction immune est réalisée par incubation de la membrane 1 nuit à +4°C sous agitation lente en présence des anticorps primaires dilués en tampon TNT-lait 5% aux concentrations de 4 µg/mL pour l'anti-CD63 et 10 µg/mL pour le pool d'anticorps anti-ANXA3. Les complexes immuns sont révélés par un anticorps secondaire de chèvre anti-IgG de souris AffiniPure conjugué à la peroxydase (H+L, Jackson Immunoresearch Cat No. 115-035-062) dilué au 1/5000^{e} en TNT-lait 5% et incubé 1 heure à température ambiante. Les signaux révélés par « enhanced chemiluminescence » (ECL, Supersignal West substrate Pierce) sont visualisés sur la plateforme Molecular Imager ChemiDoc XRS+ (BioRad) avec le Logiciel Quantity One 4.6.9. Les résultats sont présentés en Figure 9. Ils montrent qu'il est possible de détecter de l'Annexine A3 dans les exosomes précipités par ExoQuick à partir du sérum de patients.

Il est également possible de doser l'Annexine A3 après purification des exosomes. Pour cela, une adaptation du dosage sandwich développé sur VIDAS® utilisant en capture l'anticorps TGC44 et en détection l'anticorps 13A12G4H2 biotinylé a été faite en format Luminex. La technique permet à partir de microsphères en suspension de détecter et de quantifier plusieurs biomolécules dans un même échantillon de faible volume avec une sensibilité élevée.

Des billes magnétiques de 5,6 µm de diamètre, présentant une adresse spectrale basée sur leur contenu rouge/infrarouge ont servi de support pour le dosage. Une quantité de 9 µg d'anticorps de capture TGC44 a été greffée à la surface des billes magnétiques (Bio-Rad, Bio-Plex Pro Magnetic COOH Beads Amine Coupling Kit) selon les instructions du fournisseur.

L'Annexine A3 a été dosée dans le sérum de 4 patients atteints de tumeur prostatique, avant et après enrichissement exosomal (solution de précipitation ExoQuick). Pour le dosage du sérum, les échantillons B, C, D, E sont dilués au 1/5^{e} en tampon TBST. Le dosage de l'Annexine A3 après traitement du sérum et purification des exosomes est effectué à partir de 1/3 du culot exosomal repris en eau distillée qsp 100 µL. Les échantillons sont incubés en présence de 5000 billes couplées à l'anticorps de capture en plaque 96 puits (Bio-Rad, Cat No. 171025001) pendant 2 heures à 37°C, 650 rpm, à l'abri de la lumière. Entre chaque étape, les puits sont lavés 3 fois en TBST 0,05%. La détection est réalisée avec 100 µL d'anticorps secondaire 13A12G4H2 biotinylé à la concentration de 0,005 µg/mL pendant 1 heure à 37°C sous agitation. La révélation du complexe immun a lieu par incubation de 100 µL de solution de streptavidine couplée à la phycoérythrine (RPE) à la concentration de 2 µg/mL (Dako) pendant 30 minutes à 37 °C sous agitation. L'étape finale consiste à resuspendre les complexes immuns dans 100 µL de TBS pour une analyse de fluorimétrie en flux effectuée par l'automate Bio-Plex 200 (Bio-Rad). Chaque bille va subir une double excitation par un laser rouge (633 nm) pour son identification et un laser vert (532 nm) pour la quantification de l'analyte par mesure du conjugué fluorescent.

Les résultats du dosage direct dans le sérum et après enrichissement en exosomes par le traitement ExoQuick sont présentés dans le Tableau 13 ci-dessous. Cette expérience montre bien qu'il est possible de doser directement l'Annexine A3 dans des exosomes purifiés à partir du sérum par le procédé de l'invention.

**Tableau 13**

| | Dose d'ANXA3 en ng/mL | |
|---|---|---|
| Sérum | Dosage direct du sérum | Dosage après traitement ExoQuick (enrichissement) |
| D | 4,38 | 27,98 |
| E | 4,78 | 23,25 |
| B | 5,62 | 53,58 |
| C | 17,98 | 148,28 |

### Exemple 11 : Réactivité antigénique attendue entre les séquences protéiques homologues des épitopes chez différents mammifères et certains anticorps anti-ANXA3 humaine

Comme montré dans le Tableau 1 et représenté dans les Figures la et 1b, les séquences des premier et quatrième domaines répétés (respectivement D1 et D4) de l'Annexine A3 sont bien conservées entre les principaux mammifères et présentent un fort pourcentage d'identité. En conséquence de quoi, les demanderesses ont cherché à prédire la réactivité antigénique entre les homologues des épitopes LIVKEYQAAYGKE et DLSGHFEHL chez différents mammifères et les anticorps TGC42, TGC43 et TGC44 d'une part et 5C5B10 d'autre part.

Comme cela est connu de l'homme du métier, certaines substitutions d'acides aminés peuvent influencer, c'est-à-dire diminuer ou augmenter la réactivité antigénique d'un épitope vis-à-vis d'un anticorps donné, alors que la substitution par un autre acide aminé de la même position n'aura pas d'influence. Le classement des acides aminés évoqué dans la description, ainsi que les matrices de substitution, dont celle de Gonnet par exemple [25], permettent de prédire avec des chances raisonnables de succès l'impact que peut avoir une mutation donnée à un endroit précis d'un épitope.

Les données expérimentales d'ALAScan présentées dans l'Exemple 7 ont permis d'identifier les acides aminés directement impliqués dans l'interaction épitope-anticorps pour chacun des épitopes LIVKEYQAAYGKE et DLSGHFEHL. De la même manière, les données de mutagenèse par PCR présentées dans le même exemple ont permis de déterminer les résidus strictement nécessaires à la reconnaissance de l'épitope par les anticorps monoclonaux 13A12G4H2 et 1F10A6, épitope qui s'avère également être le site de fixation primaire du Ca2+ du domaine D4 de l'Annexine A3. A partir de ces données expérimentales et en s'appuyant sur l'enseignement des matrices de substitution (telles que celle de Gonnet), il est possible de prédire la réactivité antigénique entre les homologues des épitopes LIVKEYQAAYGKE et DLSGHFEHL dans les Annexines A3 d'autres mammifères et les anticorps correspondants qui se fixent sur le premier domaine répété de l'Annexine A3 humaine. Ces analyses sont présentées ci-après, respectivement dans les Tableaux 14 et 15.

**Tableau 14 - Séquence de l'épitope LIVKEYQAAYGKE de l'Annexine A3 chez d'autres mammifères et reconnaissance attendue par les anticorps TGC42, TGC43 et TGC44**

| Identifiant | Séquence | Réactivité attendue | Commentaire |
|---|---|---|---|
| SP\|P12429\|ANXA3_HUMAN | LIVKEYQAAYGKE | + | |
| SP\|O35639\|ANXA3_MOUSE | LIVKQYQAAYEQE | - | |
| SP\|P14669\|ANXA3_RAT | LIVKQYQEAYEQA | - | |
| SP\|Q3SWX7\|ANXA3_BOVIN | LIAKEYQALCGKE | +/- | |
| TR\|E2R0N3\|E2R0N3_CANFA | LIVREYQAAYGKE | + | K et R sont analogues |
| TR\|G1TEM7\|G1TEM7_RABIT | LIVREYQAAYGKE | + | K et R sont analogues |
| TR\|F6Z8Y2\|F6Z8Y2_MACMU | LIVKEYQAAYGKE | + | |
| TR\|G3R3Y9\|G3R3Y9_GORGO | LIVKEYQAAYGKE | + | |

**Tableau 15 - Séquence de l'épitope DLSGHFEHL de l'Annexine A3 chez d'autres mammifères et reconnaissance attendue par l'anticorps 5C5B10**

| Identifiant | Séquence | Réactivité attendue | Commentaire |
|---|---|---|---|
| SP\|P12429\|ANXA3_HUMAN | DLSGHFEHL | + | |
| SP\|O35639\|ANXA3_MOUSE | DLSGHFEHV | + | L et V sont analogues |
| SP\|P14669\|ANXA3_RAT | DLSGHFEHV | + | L et V sont analogues |
| SP\|Q3SWX7\|ANXA3_BOVIN | DLSGHFKHL | + | K remplace le E qui n'est pas impliqué dans la fixation |
| TR\|E2R0N3\|E2R0N3_CANFA | DLSGHFKQL | +/- | |
| TR\|G1TEM7\|G1TEM7_RABIT | DLSGHFEHL | + | |
| TR\|F6Z8Y2\|F6Z8Y2_MACMU | DLSGHFEHL | + | |
| TR\|G3R3Y9\|G3R3Y9_GORGO | DLSGHFEHL | + | |

En ce qui concerne l'épitope qui correspond au site de fixation de l'ion Ca2+ dans le domaine D4 (épitope reconnu par les anticorps monoclonaux 13A12G4H2 et 1F10A6), la mutagenèse par PCR (cf. Exemple 7) a permis de montrer que la lysine (K) en position 263 était strictement nécessaire à l'interaction épitope-anticorps. Ce résidu est conservé dans toutes les séquences présentées en 1b, il se trouve en position 6 de la séquence en acides aminés du quatrième domaine répéte de l'Annexine A3 (domaine D4). Le deuxième résidu le plus important est l'acide aspartique (D) en position 306 de l'Annexine A3. Il est également conservé dans toutes les séquences présentées enFigure 1b et se trouve en position 49 de la séquence en acides aminés du quatrième domaine répéte de l'Annexine A3(domaine D4). La conservation de ces deux positions permet de prédire que les anticorps 13A12G4H2 et 1F10A6 pourront se fixer sur toutes les Annexines A3 de mammifère présentées dans cet alignement. En revanche, l'arginine (R) en position 260 et l'isoleucine (I) en position 265 ne sont pas toujours conservées. Les données de mutagenèse par PCR montrent que ces positions sont aussi impliquées dans la reconnaissance, mais à un moindre degré (cf. Exemple 7). Le signal de fixation de l'anticorps diminue lorsque ces positions sont remplacées par une alanine mais la fixation n'est pas totalement abolie. Il sera possible de compenser cette baisse du signal grâce à une augmentation de la concentration d'utilisation des anticorps de détection. Les anticorps divulgués possèdent une très grande affinité pour l'Annexine A3 humaine et, de ce fait, sont utilisés de façon assez diluée dans les dosages de l'invention. Par exemple, l'anticorps 13A12G4H2 peut être utilisé à une concentration dans l'intervalle 1-20 pg/mL (concentration dans le tampon conjugué X1 qui est aussi le tampon de dilution de l'échantillon, dilution = 300 µL tampon conjugué + 150 µL échantillon biologique). Pour pouvoir doser les Annexines A3 de mammifère portant des mutations en positions 260 et 265, l'homme du métier pourra aisément déterminer une nouvelle concentration optimale d'utilisation de l'anticorps qui peut-être 10, 100 voire 1000 fois plus élevée que lorsque la cible du dosage est l'Annexine A3 humaine tout en restant inférieure à une concentration d'environ 30 µg/mL dans le tampon conjugué X1.

### Exemple 12 : Reproductibilité des dosages TGC44/13A12G4H2 et TGC44/5C5B10

L'objectif de l'expérience présentée dans cet exemple est d'étudier une importante caractéristique analytique des dosages ELISA TGC44/13A12G4H2 et TGC44/5C5B10, à savoir la reproductibilité. Cette analyse a été réalisée en utilisant des échantillons biologiques, c'est-à-dire du sérum ou de l'urine collectée juste après le toucher rectal (urine post-TR, également dénommée « urine exprimée ») obtenus chez des patients atteints de pathologies prostatiques (cancer de la prostate ou hyperplasie bénigne de la prostate), afin de s'approcher le plus possible des conditions d'utilisation réelles des dosages. La reproductibilité du dosage TGC44/5C5B10 sur des échantillons de sérum n'a pas été étudiée car ce dosage ne permet pas de doser l'Annexine A3 humaine dans le sérum.

Pour chaque dosage et chaque type d'échantillon, 5 échantillons ont été dosés sur 1 instrument en 3 jours, à raison de 2 séries par jour et de 2 répétitions par série. Il y a donc n=12 répétitions de la mesure pour chaque échantillon. Le coefficient de variation (CV) correspondant à cette série de 12 mesures a été calculé et est présenté dans le Tableau 16 infra. Ce coefficient de variation est l'indicateur qui permet de juger de la reproductibilité de la mesure : plus le CV est petit, plus la mesure est reproductible.

Cette analyse nécessite de disposer de grands volumes d'échantillons afin de pouvoir réaliser des mesures répétées. L'obtention de larges volumes de sérum est moins facile comparée à de l'urine. Ainsi les expériences sur urine ont été réalisées sur urines individuelles alors que certains sérums ont été « poolés », afin d'obtenir le volume nécessaire.

**Tableau 16 - Reproductibilité des ELISA TGC44/13A12G4H2 et TGC44/5C5B10**

| TGC44/13A12G4H2 Sur urine Post-TR | | TGC44/13A12G4H2 Sur sérum | | TGC44/5C5B10 Sur urine Post-TR | |
|---|---|---|---|---|---|
| Echantillon | % CV | Echantillon | % CV | Echantillon | % CV |
| 50237 | 8 | L02+L13 | 4 | 50222 | 15 |
| 10204 | 11 | L04+L07 | 6 | 10204 | 9 |
| 30299 | 5 | L01 | 6 | 50237 | 13 |
| 1001450 | 7 | L41 | 4 | 1001450 | 14 |
| 1001425 | 7 | N03+N05+N11 | 4 | 1001425 | 11 |

L'ELISA TGC44/13A12G4H2 possède une bonne reproductibilité lorsqu'il est utilisé pour doser des urines post-TR. En effet, pour les 5 échantillons testés, les CV sont inférieurs à 15%, qui représente la limite supérieure admise. L'ELISA TGC44/13A12G4H2 possède aussi une très bonne reproductibilité pour doser des sérums, meilleure que dans l'urine. Parmi les échantillons de sérum testés, aucun ne dépasse un CV de 6%. Le dosage TGC44/5C5B10 possède également une reproductibilité correcte dans l'urine post-TR, quoique un peu moins bonne que l'ELISA TGC44/13A12G4H2.

### Références bibliographiques

1. Moss SE, Morgan RO. The annexins. Genome biology 2004; 5: 219.
2. Barton GJ, Newman RH, Freemont PS, Crumpton MJ. Amino acid sequence analysis of the annexin super-gene family of proteins. Eur J Biochem 1991; 198: 749-760.
3. Gerke V, Moss SE. Annexins: from structure to function. Physiological reviews 2002; 82: 331-371.
4. Gerke V, Creutz CE, Moss SE. Annexins: linking Ca2+ signalling to membrane dynamics. Nature reviews. Molecular cell biology 2005; 6: 449-461.
5. Hayes MJ, Rescher U, Gerke V, Moss SE. Annexin-actin interactions. Traffic 2004; 5: 571-576.
6. Rescher U, Gerke V. Annexins--unique membrane binding proteins with diverse functions. Journal of cell science 2004; 117: 2631-2639.
7. Harashima M, Niimi S, Koyanagi H et al. Change in annexin A3 expression by regulatory factors of hepatocyte growth in primary cultured rat hepatocytes. Biological & pharmaceutical bulletin 2006; 29: 1339-1343.
8. Le Cabec V, Russo-Marie F, Maridonneau-Parini I. Differential expression of two forms of annexin 3 in human neutrophils and monocytes and along their differentiation. Biochem Biophys Res Commun 1992; 189: 1471-1476.
9. Le Cabec V, Maridonneau-Parini I. Annexin 3 is associated with cytoplasmic granules in neutrophils and monocytes and translocates to the plasma membrane in activated cells. Biochem J 1994; 303 (Pt 2): 481-487.
10. Bianchi C, Bombelli S, Raimondo F et al. Primary cell cultures from human renal cortex and renal-cell carcinoma evidence a differential expression of two spliced isoforms of Annexin A3. Am J Pathol 176: 1660-1670.
11. Tait JF, Sakata M, McMullen BA et al. Placental anticoagulant proteins: isolation and comparative characterization four members of the lipocortin family. Biochemistry 1988; 27: 6268-6276.
12. Ernst JD, Hoye E, Blackwood RA, Jaye D. Purification and characterization of an abundant cytosolic protein from human neutrophils that promotes Ca2(+)-dependent aggregation of isolated specific granules. The Journal of clinical investigation 1990; 85: 1065-1071.
13. Chong KW, Chen MJ, Koay ES et al. Annexin A3 is associated with cell death in lactacystin-mediated neuronal injury. Neuroscience letters 2010; 485: 129-133.
14. Kollermann J, Schlomm T, Bang H et al. Expression and prognostic relevance of annexin A3 in prostate cancer. Eur Urol 2008; 54: 1314-1323.
15. Wozny W, Schroer K, Schwall GP et al. Differential radioactive quantification of protein abundance ratios between benign and malignant prostate tissues: cancer association of annexin A3. Proteomics 2007; 7: 313-322.
16. Madoz-Gurpide J, Lopez-Serra P, Martinez-Torrecuadrada JL et al. Proteomics-based validation of genomic data: applications in colorectal cancer diagnosis. Molecular & cellular proteomics : MCP 2006; 5: 1471-1483.
17. Marshall KW, Mohr S, Khettabi FE et al. A blood-based biomarker panel for stratifying current risk for colorectal cancer. International journal of cancer. Journal international du cancer 2010; 126: 1177-1186.
18. Liu YF, Xiao ZQ, Li MX et al. Quantitative proteome analysis reveals annexin A3 as a novel biomarker in lung adenocarcinoma. The Journal of pathology 2009; 217: 54-64.
19. Baine MJ, Chakraborty S, Smith LM et al. Transcriptional profiling of peripheral blood mononuclear cells in pancreatic cancer patients identifies novel genes with potential diagnostic utility. PLoS One 2011; 6: e17014.
20. Jung EJ, Moon HG, Park ST et al. Decreased annexin A3 expression correlates with tumor progression in papillary thyroid cancer. Proteomics. Clinical applications 2010; 4: 528-537.
21. Wang XM, Wu TX, Hamza M et al. Rofecoxib modulates multiple gene expression pathways in a clinical model of acute inflammatory pain. Pain 2007; 128: 136-147.
22. Kohler G, Milstein C. Continuous cultures of fused cells secreting antibody of predefined specificity. Nature 1975; 256: 495-497.
23. Kohler G, Howe SC, Milstein C. Fusion between immunoglobulin-secreting and nonsecreting myeloma cell lines. Eur J Immunol 1976; 6: 292-295.
24. Frank R, Döring R. Simultaneous multiple peptide synthesis under continuous flow conditions on cellulose papers discs as segmental solid synthesis. Tetrahedron 1988; 44: 6031-6040.
25. Gonnet G.H., Cohen M.A., Benner S.A.; "Exhaustive matching of the entire protein sequence database."; Science 256:1443-1445(1992).
26. Geysen MS, Rodda T, Mason TJ, Triubbick G, Schoofs P. Strategies of epitope analysis using synthetic peptide synthesis. J. Immunol. Meth. 1987. 102 : 259-265.
27. Ng et Henikoff, 2001, Genome Res 11: 863-874.

## Revendications

1. Procédé de détection et/ou de dosage *in vitro* de l'Annexine A3 dans un échantillon biologique d'un mammifère, ledit échantillon biologique étant choisi parmi le sang ou au moins un de ses dérivés tels que le plasma et le sérum, dans lequel l'échantillon biologique est mis en contact avec un premier anticorps et un deuxième anticorps, le premier anticorps étant dirigé contre le premier domaine répété de l'Annexine A3 dudit mammifère et le deuxième anticorps étant dirigé contre le quatrième domaine répété de l'Annexine A3 dudit mammifère.

2. Procédé selon la revendication 1, dans lequel le premier anticorps est un anticorps de capture et le deuxième anticorps est un anticorps de détection.

3. Procédé selon la revendication 1 ou 2, dans lequel le premier anticorps est sélectionné parmi les anticorps dirigés contre un épitope dont la séquence en acides aminés comprend au moins 7 acides aminés consécutifs et pas plus de 17 acides aminés consécutifs du premier domaine répété de l'Annexine A3 dudit mammifère.

4. Procédé selon l'une des revendications 1 à 3, dans lequel le premier anticorps est sélectionné parmi les anticorps dirigés contre un épitope inclus dans le premier domaine répété de l'Annexine A3 dudit mammifère, ledit épitope ayant une séquence en acides aminés sélectionnée parmi les séquences suivantes :
Xaa1-Xaa2-A-Q-Xaa3-Xaa4-L-I-Xaa5-Xaa6-Xaa7-Y-Q-Xaa8-Xaa9-Xaa10-Xaa11 (SEQ ID NO: 37),
L-1-Xaa5-Xaa6-Xaa7-Y-Q-Xaa8-Xaa9-Xaa10-Xaa11 (SEQ ID NO: 38),
I-Xaa5-Xaa6-Xaa7-Y-Q-Xaa8-Xaa9-Xaa10-Xaa11-Xaa12-Xaa13 (SEQ ID NO: 39),
Xaa6-Xaa7-Y-Q-Xaa8-Xaa9-Xaa10-Xaa11 (SEQ ID NO: 40),
Xaa6-Xaa7-Y-Q-Xaa8-Xaa9-Xaa10-Xaa11-Xaa12-Xaa13-L-K-Xaa14-D-L-K-G (SEQ ID NO: 41) à la condition que la séquence en acides aminés SEQ ID NO: 41 soit fusionnée du côté N-terminal à une séquence d'au moins 30 acides aminés,
DLSGHF-Xaa20-Xaa21-Xaa22 (SEQ ID NO: 42),
LSGHF-Xaa20-Xaa21 (SEQ ID NO: 43),
dans lequel :
- Xaa1 représente un résidu sérine, un résidu thréonine, ou un acide aminé analogue,
- Xaa2 représente un résidu asparagine, un résidu sérine ou un acide aminé analogue,
- Xaa3 représente un résidu arginine, un résidu histidine ou un acide aminé analogue,
- Xaa4 représente un résidu glutamine, un résidu leucine ou un acide aminé analogue,
- Xaa5 représente un résidu valine, un résidu alanine ou un acide aminé analogue,
- Xaa6 représente un résidu lysine, un résidu arginine ou un acide aminé analogue,
- Xaa7 représente un résidu acide glutamique, un résidu glutamine ou un acide aminé analogue,
- Xaa8 représente un résidu alanine, un résidu acide glutamique ou un acide aminé analogue,
- Xaa9 représente un résidu alanine, un résidu leucine ou un acide aminé analogue,
- Xaa10 représente un résidu tyrosine, un résidu cystéine ou un acide aminé analogue,
- Xaa1 représente un résidu glycine, un résidu acide glutamique ou un acide aminé analogue,
- Xaa12 représente un résidu lysine, un résidu glutamine ou un acide aminé analogue,
- Xaal3 représente un résidu acide glutamique, un résidu alanine ou un acide aminé analogue,
- Xaal4 représente un résidu acide aspartique, un résidu alanine ou un acide aminé analogue,
- Xaa20 représente un résidu acide glutamique, un résidu lysine ou un acide aminé analogue,
- Xaa21 représente un résidu histidine, un résidu glutamine ou un acide aminé analogue,
- Xaa22 représente un résidu leucine, un résidu valine ou un acide aminé analogue.

5. Procédé selon l'une des revendications 1 à 4, dans lequel le deuxième anticorps est sélectionné parmi les anticorps dirigés contre un épitope dont la séquence en acides aminés comprend au moins 7 acides aminés consécutifs et pas plus de 50 acides aminés consécutifs du quatrième domaine répété de l'Annexine A3 dudit mammifère.

6. Procédé selon l'une des revendications 1 à 5, dans lequel le deuxième anticorps est sélectionné parmi les anticorps dirigés contre un épitope dont la séquence en acides aminés correspond à la séquence en acides aminés commençant au résidu 3 et se terminant au résidu 49 du quatrième domaine répété de l'Annexine A3 dudit mammifère.

7. Procédé selon la revendication 5 ou 6, dans lequel l'épitope comprend un résidu lysine en position 6 du quatrième domaine répété de l'Annexine A3 dudit mammifère et/ou un résidu acide aspartique en position 49 dudit quatrième domaine répété de l'Annexine A3 dudit mammifère.

8. Procédé selon la revendication 7, dans lequel l'épitope comprend un résidu arginine ou glutamine en position 3 du quatrième domaine répété de l'Annexine A3 dudit mammifère et un résidu isoleucine ou alanine en position 8 dudit quatrième domaine répété de l'Annexine A3 dudit mammifère.

9. Procédé selon la revendication 7 ou 8, dans lequel l'épitope comprend un résidu glycine en position 7 du quatrième domaine répété de l'Annexine A3 dudit mammifère, un résidu isoleucine ou alanine en position 8 du quatrième domaine répété de l'Annexine A3 dudit mammifère et un résidu glycine en position 9 dudit quatrième domaine répété de l'Annexine A3 dudit mammifère.

10. Procédé selon l'une des revendications 1 à 9, dans lequel le mammifère est l'homme et dans lequel le premier anticorps est dirigé contre le premier domaine répété de ladite Annexine A3 humaine dont la séquence est identifiée en SEQ ID NO: 1 et le deuxième anticorps est dirigé contre le quatrième domaine répété de l'Annexine A3 humaine dont la séquence est identifiée en SEQ ID NO: 2.

11. Procédé selon l'une des revendications 1 à 10 pour le diagnostic *in vitro* et/ou le suivi *in vitro* de l'évolution d'une pathologie comprise dans le groupe constitué par les cancers, tels que les cancers urogénitaux et notamment les cancers de la prostate, le cancer colorectal, le cancer du poumon, le cancer du pancréas, le cancer de la thyroïde papillaire, le cancer du sein, les désordres inflammatoires , tels que le syndrome de réponse inflammatoire systémique, le sepsis, la polyarthrite rhumatoïde, la maladie de Crohn, le diabète.

12. Coffret d'immunodétection et/ou d'immunodosage d'un échantillon biologique d'un mammifère, ledit échantillon biologique étant choisi parmi le sang ou au moins un de ses dérivés tels que le plasma et le sérum, ledit coffret comprenant :
- un premier anticorps dirigé contre le premier domaine répété de l'Annexine A3 dudit mammifère, tel que défini dans l'une des revendications 1 à 10,
- un deuxième anticorps dirigé contre le quatrième domaine répété de l'Annexine A3 dudit mammifère, tel que défini dans l'une des revendications 1 à 10,
- un moyen de calibration approprié,
ledit coffret permettant de mettre en oeuvre le procédé selon l'une des revendications 1 à 11.

13. Utilisation du coffret selon la revendication 12 pour la mise en oeuvre du procédé selon l'une des revendications 1 à 11.

## Patentansprüche

1. Ein In-vitro-Verfahren zur Detektion und/oder Dosierung von Annexin A3 in einer biologischen Probe eines Säugetiers, wobei die biologische Probe ausgewählt ist aus Blut oder mindestens einem seiner Derivate wie etwa Plasma oder Serum, wobei die biologische Probe mit einem ersten Antikörper und einem zweiten Antikörper in Kontakt gebracht wird, wobei der erste Antikörper gegen die erste Wiederholungsdomäne des Annexin A3 des Säugetiers gerichtet ist und der zweite Antikörper gegen die vierte Wiederholungsdomäne des Annexin A3 des Säugetiers gerichtet ist.

2. Verfahren gemäß Anspruch 1, wobei der erste Antikörper ein Fängerantikörper ist und der zweite Antikörper ein Detektorantikörper ist.

3. Verfahren gemäß Anspruch 1 oder 2, wobei der erste Antikörper ausgewählt ist aus den Antikörpern, die gegen ein Epitop gerichtet sind, dessen Aminosäuresequenz mindestens 7 aufeinanderfolgende Aminosäuren und nicht mehr als 17 aufeinanderfolgende Aminosäuren der ersten Wiederholungsdomäne des Annexin A3 des Säugetiers umfasst.

4. Verfahren gemäß einem der Ansprüche 1 bis 3, wobei der erste Antikörper ausgewählt ist aus den Antikörpern, die gegen ein Epitop gerichtet werden, das in der ersten Wiederholungsdomäne des Annexin A3 des Säugetiers eingeschlossen ist, wobei das Epitop eine Aminosäuresequenz aufweist, die aus folgenden Sequenzen ausgewählt ist:
Xaa1-Xaa2-A-Q-Xaa3-Xaa4-L-I-Xaa5-Xaa6-Xaa7-Y-Q-Xaa8-Xaa9-Xaa10-Xaa11 (SEQ ID NO: 37),
L-I-Xaa5-Xaa6-Xaa7-Y-Q-Xaa8-Xaa9-Xaa10-Xaa11 (SEQ ID NO: 38),
I-Xaa5-Xaa6-Xaa7-Y-Q-Xaa8-Xaa9-Xaa10-Xaa11-Xaa12-Xaa13 (SEQ ID NO: 39),
Xaa6-Xaa7-Y-Q-Xaa8-Xaa9-Xaa10-Xaa11 (SEQ ID NO: 40),
Xaa6-Xaa7-Y-Q-Xaa8-Xaa9-Xaa10-Xaa11-Xaa12-Xaa13-L-K-Xaa14-D-L-K-G (SEQ ID NO: 41), vorausgesetzt, dass die Aminosäuresequenz SEQ ID NO: 41 auf der N-terminalen Seite mit einer Sequenz mit mindestens 30 Aminosäuren verschmolzen ist,
DLSGHF-Xaa20-Xaa21-Xaa22 (SEQ ID NO: 42),
LSGHF-Xaa20-Xaa21 (SEQ ID NO: 43),
wobei
- Xaa1 einen Serinrest, einen Threoninrest oder ein Aminosäure-Analogon darstellt,
- Xaa2 einen Asparaginrest, einen Serinrest oder ein Aminosäure-Analogon darstellt,
- Xaa3 einen Argininrest, einen Histidinrest oder ein Aminosäure-Analogon darstellt,
- Xaa4 einen Glutaminrest, einen Leucinrest oder ein Aminosäure-Analogon darstellt,
- Xaa5 einen Valinrest, einen Alaninrest oder ein Aminosäure-Analogon darstellt,
- Xaa6 einen Lysinrest, einen Argininrest oder ein Aminosäure-Analogon darstellt,
- Xaa7 einen Glutaminsäurerest, einen Glutaminrest oder ein Aminosäure-Analogon darstellt,
- Xaa8 einen Alaninrest, einen Glutaminsäurerest oder ein Aminosäure-Analogon darstellt,
- Xaa9 einen Alaninrest, einen Leucinrest oder ein Aminosäure-Analogon darstellt,
- Xaa10 einen Tyrosinrest, einen Cysteinrest oder ein Aminosäure-Analogon darstellt,
- Xaa11 einen Glycinrest, einen Glutaminsäurerest oder ein Aminosäure-Analogon darstellt,
- Xaa12 einen Lysinrest, einen Glutaminrest oder ein Aminosäure-Analogon darstellt,
- Xaa13 einen Glutaminsäurerest, einen Alaninrest oder ein Aminosäure-Analogon darstellt,
- Xaa14 einen Asparaginsäurerest, einen Alaninrest oder ein Aminosäure-Analogon darstellt,
- Xaa20 einen Glutaminsäurerest, einen Lysinrest oder ein Aminosäure-Analogon darstellt,
- Xaa21 einen Histidinrest, einen Glutaminrest oder ein Aminosäure-Analogon darstellt,
- Xaa22 einen Leucinrest, einen Valinrest oder ein Aminosäure-Analogon darstellt.

5. Verfahren gemäß einem der Ansprüche 1 bis 4, wobei der zweite Antikörper ausgewählt ist aus den Antikörpern, die gegen ein Epitop gerichtet werden, dessen Aminosäuresequenz mindestens 7 aufeinanderfolgende Aminosäuren und nicht mehr als 50 aufeinanderfolgende Aminosäuren der vierten Wiederholungsdomäne des Annexin A3 des Säugetiers umfasst.

6. Verfahren gemäß einem der Ansprüche 1 bis 5, wobei der zweite Antikörper ausgewählt ist aus den Antikörpern, die gegen ein Epitop gerichtet werden, dessen Aminosäuresequenz der Aminosäuresequenz entspricht, die mit Rest 3 der vierten Wiederholungsdomäne des Annexin A3 des Säugetiers beginnt und mit dessen Rest 49 endet.

7. Verfahren gemäß Anspruch 5 oder 6, wobei das Epitop einen Lysinrest in Position 6 der vierten Wiederholungsdomäne des Annexin A3 des Säugetiers und/oder einen Asparaginsäurerest in Position 49 der vierten Wiederholungsdomäne des Annexin A3 des Säugetiers umfasst.

8. Verfahren gemäß Anspruch 7, wobei das Epitop einen Arginin- oder Glutaminrest in Position 3 der vierten Wiederholungsdomäne des Annexin A3 des Säugetiers und einen Isoleucin- oder Alaninrest in Position 8 der vierten Wiederholungsdomäne des Annexin A3 des Säugetiers umfasst.

9. Verfahren gemäß Anspruch 7 oder 8, wobei das Epitop einen Glycinrest in Position 7 der vierten Wiederholungsdomäne des Annexin A3 des Säugetiers, einen Isoleucin- oder Alaninrest in Position 8 der vierten Wiederholungsdomäne des Annexin A3 des Säugetiers und einen Glycinrest in Position 9 der vierten Wiederholungsdomäne des Annexin A3 des Säugetiers umfasst.

10. Verfahren gemäß einem der Ansprüche 1 bis 9, wobei das Säugetier der Mensch ist und wobei der erste Antikörper gegen die erste Wiederholungsdomäne des menschlichen Annexin A3, dessen Sequenz als SEQ ID NO: 1 festgelegt ist, gerichtet ist und der zweite Antikörper gegen die vierte Wiederholungsdomäne des menschlichen Annexin A3, dessen Sequenz als SEQ ID NO: 2 identifiziert ist, gerichtet ist.

11. Verfahren gemäß einem der Ansprüche 1 bis 10 zur In-vitro-Diagnose und/oder In-vitro-Überwachung der Entwicklung einer pathologischen Erscheinung, enthalten in der Gruppe, die aus Folgendem besteht: Krebsen wie etwa Urogenitalkrebsen und insbesondere Prostatakrebsen, Darmkrebs, Lungenkrebs, Bauchspeicheldrüsenkrebs, papilläres Schilddrüsenkarzinom, Brustkrebs, entzündliche Störungen wie etwa systemisches inflammatorisches Response-Syndrom, Sepsis, rheumatische Polyarthritis, Morbus Crohn, Diabetes.

12. Ein Kit für die Immundetektion und/oder Immundosierung einer biologischen Probe eines Säugetiers, wobei die biologische Probe ausgewählt ist aus Blut oder mindestens einem seiner Derivate wie etwa Plasma oder Serum, wobei das Kit Folgendes umfasst:
- einen ersten Antikörper, der gegen die erste Wiederholungsdomäne des Annexin A3 des Säugetiers gerichtet ist, wie in einem der Ansprüche 1 bis 10 definiert,
- einen zweiten Antikörper, der gegen die vierte Wiederholungsdomäne des Annexin A3 des Säugetiers gerichtet ist, wie in einem der Ansprüche 1 bis 10 definiert,
- ein geeignetes Kalibriermittel,
wobei das Kit das Durchführen des Verfahrens gemäß einem der Ansprüche 1 bis 11 ermöglicht.

13. Eine Verwendung des Kits gemäß Anspruch 12 zum Durchführen des Verfahrens gemäß einem der Ansprüche 1 bis 11.

## Claims

1. A method of detecting and/or assaying *in vitro* Annexin A3 in a biological sample of a mammal, said biological sample being chosen from blood or at least one of its derivatives, such as plasma and serum, wherein the biological sample is brought into contact with a first antibody and a second antibody, the first antibody being directed against the first repeat domain of Annexin A3 from said mammal and the second antibody being directed against the fourth repeat domain of Annexin A3 from said mammal.

2. The method according to claim 1, wherein the first antibody is a capture antibody and the second antibody is a detection antibody.

3. The method according to claim 1 or 2, wherein the first antibody is selected from amongst the antibodies directed against an epitope the amino acid sequence of which comprises at least 7 consecutive amino acids and no more than 17 consecutive amino acids of the first repeat domain of Annexin A3 from said mammal.

4. The method according to one of claims 1 to 3, wherein the first antibody is selected from amongst the antibodies directed against an epitope included in the first repeat domain of Annexin A3 from said mammal, said epitope having an amino acid sequence selected from amongst the following sequences:
Xaa1-Xaa2-A-Q-Xaa3-Xaa4-L-I-Xaa5-Xaa6-Xaa7-Y-Q-Xaa8-Xaa9-Xaa10-Xaa11 (SEQ ID NO: 37),
L-I-Xaa5-Xaa6-Xaa7-Y-Q-Xaa8-Xaa9-Xaa10-Xaa11 (SEQ ID NO: 38),
I-Xaa5-Xaa6-Xaa7-Y-Q-Xaa8-Xaa9-Xaa10-Xaa11-Xaa12-Xaa13 (SEQ ID NO: 39),
Xaa6-Xaa7-Y-Q-Xaa8-Xaa9-Xaa10-Xaa11 (SEQ ID NO: 40),
Xaa6-Xaa7-Y-Q-Xaa8-Xaa9-Xaa10-Xaa11-Xaa12-Xaa13-L-K-Xaa14-D-L-K-G (SEQ ID NO: 41) provided that the amino acid sequence SEQ ID NO: 41 is fused on the N-terminus side to a sequence of at least 30 amino acids,
DLSGHF-Xaa20-Xaa21-Xaa22 (SEQ ID NO: 42),
LSGHF-Xaa20-Xaa21 (SEQ ID NO: 43),
wherein:
- Xaa1 represents a serine residue, a threonine residue or an analogous amino acid,
- Xaa2 represents an asparagine residue, a serine residue or an analogous amino acid,
- Xaa3 represents an arginine residue, a histidine residue or an analogous amino acid,
- Xaa4 represents a glutamine residue, a leucine residue or an analogous amino acid,
- Xaa5 represents a valine residue, an alanine residue or an analogous amino acid,
- Xaa6 represents a lysine residue, an arginine residue or an analogous amino acid,
- Xaa7 represents a glutamic acid residue, a glutamine residue or an analogous amino acid,
- Xaa8 represents an alanine residue, a glutamic acid residue or an analogous amino acid,
- Xaa9 represents an alanine residue, a leucine residue or an analogous amino acid,
- Xaa10 represents a tyrosine residue, a cysteine residue or an analogous amino acid,
- Xaa11 represents a glycine residue, a glutamic acid residue or an analogous amino acid,
- Xaa12 represents a lysine residue, a glutamine residue or an analogous amino acid,
- Xaa13 represents a glutamic acid residue, an alanine residue or an analogous amino acid,
- Xaa14 represents an aspartic acid residue, an alanine residue or an analogous amino acid,
- Xaa20 represents a glutamic acid residue, a lysine residue or an analogous amino acid,
- Xaa21 represents a histidine residue, a glutamine residue or an analogous amino acid,
- Xaa22 represents a leucine residue, a valine residue or an analogous amino acid,

5. The method according to one of claims 1 to 4, wherein the second antibody is selected from amongst the antibodies directed against an epitope the amino acid sequence of which comprises at least 7 consecutive amino acids and no more than 50 consecutive amino acids of the fourth repeat domain of Annexin A3 from said mammal.

6. The method according to one of claims 1 to 5, wherein the second antibody is selected from amongst the antibodies directed against an epitope the amino acid sequence of which corresponds to the amino acid sequence starting at residue 3 and ending at residue 49 of the fourth repeat domain of Annexin A3 from said mammal.

7. The method according to claim 5 or 6, wherein the epitope comprises a lysine residue at position 6 of the fourth repeat domain of Annexin A3 from said mammal and/or an aspartic acid residue at position 49 of said fourth repeat domain of Annexin A3 from said mammal.

8. The method according to claim 7, wherein the epitope comprises an arginine or glutamine residue at position 3 of the fourth repeat domain of Annexin A3 from said mammal and an isoleucine or alanine residue at position 8 of said fourth repeat domain of Annexin A3 from said mammal.

9. The method according to claim 7 or 8, wherein the epitope comprises a glycine residue at position 7 of the fourth repeat domain of Annexin A3 from said mammal, an isoleucine or alanine residue at position 8 of said fourth repeat domain of Annexin A3 from said mammal and a glycine residue at position 9 of said fourth repeat domain of Annexin A3 from said mammal.

10. The method according to one of claims 1 to 9, wherein the mammal is human and wherein the first antibody is directed against the first repeat domain of said human Annexin A3 the sequence of which is identified in SEQ ID NO: 1 and the second antibody is directed against the fourth repeat domain of the human Annexin A3 the sequence of which is identified in SEQ ID NO: 2.

11. The method according to one of claims 1 to 10 for the *in vitro* diagnosis and/or the *in vitro* monitoring of the evolution of a pathology comprised in the group constituted by cancers, such as urogenital cancers and particularly prostate cancers, colorectal cancer, lung cancer, pancreatic cancer, papillary thyroid cancer, breast cancer, inflammatory disorders such as systemic inflammatory response syndrome, sepsis, rheumatoid polyarthritis, Crohn's disease, diabetes.

12. A kit for the immunodetection and/or immunoassaying of a biological sample of a mammal, said biological sample being chosen from blood or at least one of its derivatives, such as plasma and serum, said kit comprising:
- a first antibody directed against the first repeat domain of Annexin A3 from said mammal, as defined in one of claims 1 to 10,
- a second antibody directed against the fourth repeat domain of Annexin A3 from said mammal, as defined in one of claims 1 to 10,
- an appropriate calibration means,
said kit making it possible to implement the method according to one of claims 1 to 11.

13. The use of the kit according to claim 12 for the implementation of the method according to one of claims 1 to 11.
